# EUROPEAN PATENT APPLICATION

(11) **EP 4 435 803 A1**
(43) Date of publication of application: **25.09.2024**
(21) Application number: 24161669.7
(22) Date of filing: 06.03.2024
(51) Int. Cl.: H01B 1/12, A61B 5/268, C08L 75/04

(54) **BIO-ELECTRODE COMPOSITION, BIO-ELECTRODE, AND METHOD FOR MANUFACTURING THE SAME**

(30) Priority: 23.03.2023 JP 2023046841
(71) Applicant: SHIN-ETSU CHEMICAL CO., LTD., Tokyo 1000005 (JP)
(72) Inventor: Hatakeyama, Jun, Niigata (JP); Ikeda, Joe, Tokyo (JP); Nonaka, Shiori, Niigata (JP); Iwabuchi, Motoaki, Niigata (JP); Hasegawa, Koji, Tokyo (JP)
(74) Representative: Sonnenhauser, Thomas Martin

(57) **Abstract**

The present invention is a bio-electrode composition includes, as the component (A), a resin which has a urethan bond in a main chain and at least one salt selected from the group consisting of salts of ammonium, sodium, potassium, and silver formed with sulfonamide in a side chain. This can provide: a bio-electrode composition capable of forming a living body contact layer for a bio-electrode that is excellent in electric conductivity and biocompatibility, light-weight, manufacturable at low cost, capable of preventing significant reduction in the electric conductivity even when wetted with water or dried, soft, and excellent in stretchability and strength; a bio-electrode including a living body contact layer formed of the bio-electrode composition; and a method for manufacturing the bio-electrode.

## Description

### TECHNICAL FIELD

The present invention relates to: a bio-electrode composition; a bio-electrode; and a method for manufacturing the same.

### BACKGROUND ART

A recent growing popularity of Internet of Things (IoT) has accelerated the development of wearable devices. The typical examples include watches and eyeglasses that can connect to the Internet. Even in the fields of medicine and sports, wearable devices for constantly monitoring the user's physical state are demanded, and such technological development is expected to be further encouraged.

In the field of medicine, a use of wearable devices has been studied for monitoring the state of human organs by sensing extremely weak current as an electrocardiogram detects an electric signal to measure the motion of the heart. The electrocardiogram measurement is performed by attaching electrodes coated with an electro-conductive paste of water-soluble gel to a body, but this is a single and short-time measurement. On the other hand, development of the above medical wearable device is aimed at a device for monitoring the health condition continuously for a few weeks. Accordingly, bio-electrodes used in a medical wearable device is required to make no changes in electric conductivity and cause no skin allergy even in long-time use. In addition to these, it is also required that a bio-electrodes are light-weight and can be manufactured at low cost.

Medical wearable devices are classified into two types: a type which is directly attached to the body and a type which is incorporated into clothes. In the type which is attached to the body, there has been proposed a bio-electrode using water-soluble gel containing water and electrolyte, which are materials of the electro-conductive paste (Patent Document 1). The water-soluble gel contains sodium, potassium, or calcium as the electrolyte in a water-soluble polymer for retaining water, and converts changes in ion concentration of the skin into electricity. On the other hand, in the type which is incorporated into clothes, there has been proposed a use of cloth, in which an electro-conductive polymer such as PEDOT-PSS (poly-3,4-ethylenedioxythiophene-polystyrenesulfonate) or silver paste is incorporated into the fibers, for electrodes (Patent Document 2).

However, the use of the water-soluble gel containing water and electrolyte brings about loss of electric conductivity due to water evaporation by dryness. Meanwhile, the use of a higher-ionization-tendency metal such as copper or the like can cause some users to suffer from skin allergy. The use of an electro-conductive polymer such as PEDOT-PSS can also cause skin allergy due to having strong acidity as the electro-conductive polymer, and further has a problem in that the conductive polymer peels off from the fibers during washing.

By taking advantage of excellent electric conductivity, the use of metal nanowire, carbon black, carbon nanotube, etc. for electrode materials has been studied (Patent Documents 3, 4, and 5). With high contact probability among metal nanowires, the metal nanowire can conduct electricity even when added in a small quantity. The metal nanowire, however, can cause skin allergies because they are thin materials with a sharp tip. As described above, even if these electrode materials themselves cause no allergic reaction, the biocompatibility may be degraded on account of the shape of the material and its skin irritancy, and therefore, it was difficult to achieve both electrical conductivity and biocompatibility.

In a wristwatch sold by Apple Inc., a biological signal of lead-I ECG can be obtained by touching the metal on the skin side of the watch, which is always in contact with the skin, and a metal button called Digital Crown, with the fingers of the hand not wearing the wristwatch (Patent Document 6).

Metals are thought to act as excellent bioelectrodes because they have high electric conductivity, this is not always the case. What are emitted from the skin when the heart beats are not only a weak electrical current but also sodium, potassium, and calcium ions. For this reason, it is necessary to convert changes in ion concentration into electric current, but precious metals that are difficult to ionize are inefficient at converting ions from the skin into electric current. Therefore, bioelectrodes made of precious metals have high impedance and high resistance when conducting electricity to the skin.

### CITATION LIST

### PATENT LITERATURE

Patent Document 1: WO 2013/039151 A1
Patent Document 2: JP 2015-100673 A
Patent Document 3: JP H05-095924 A
Patent Document 4: JP 2003-225217 A
Patent Document 5: JP 2015-019806 A
Patent Document 6: JP 2021-093127 A

### SUMMARY OF INVENTION

### TECHNICAL PROBLEM

The present invention has been made to solve the above problems, and has an object to provide: a bioelectrode composition capable of forming a living body contact layer for a bio-electrode that is excellent in electric conductivity and biocompatibility, light-weight, manufacturable at low cost, capable of preventing significant reduction in the electric conductivity even when wetted with water or dried, soft, and excellent in stretchability and strength; a bio-electrode including a living body contact layer formed of the bio-electrode composition; and a method for manufacturing the bioelectrode.

### SOLUTION TO PROBLEM

To solve the above problems, the present invention provides a bio-electrode composition comprising a resin as a component (A), wherein
the component (A) has a urethan bond in a main chain and at least one salt selected from the group consisting of salts of ammonium, sodium, potassium, and silver formed with sulfonamide in a side chain.

Such a bio-electrode composition can form a living body contact layer for a bio-electrode that is excellent in electric conductivity and biocompatibility, light-weight, manufacturable at low cost, capable of preventing significant reduction in the electric conductivity even when wetted with water or dried, soft, and excellent in stretchability and strength.

Further, in the present invention, the component (A) is preferably represented by the following general formula (1), 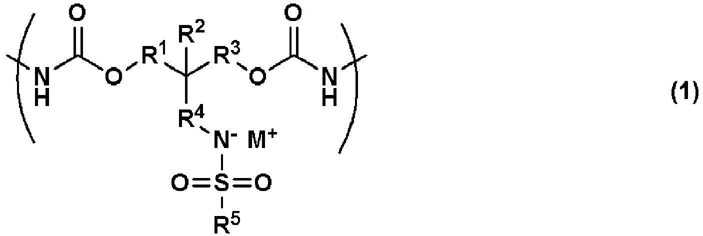 wherein, R¹ and R³ represent a single bond, or a linear or branched alkylene group having 1 to 6 carbon atoms, and optionally have an ether bond or a bromine atom; R² represents a hydrogen atom, a hydroxy group, or a linear or branched alkyl or alkoxy group having 1 to 6 carbon atoms; R⁴ represents a single bond or a linear, branched, or cyclic hydrocarbylene group having 1 to 20 carbon atoms, and optionally have a carbonyl group, an ether group, or an ester group, or optionally form a ring together with R¹; R⁵ represents a linear, branched, or cyclic alkyl group having 1 to 10 carbon atoms and optionally substituted with a fluorine atom, or R⁵ represents an alkenyl group having 2 to 6 carbon atoms, a alkynyl group having 2 to 6 carbon atoms, or an aryl group having 6 to 10 carbon atoms and optionally has a fluorine atom, a trifluoromethyl group, a cyano group, a nitro group, a hydroxy group, an alkoxy group having 1 to 6 carbon atoms, or an alkyl group having 1 to 6 carbon atoms; and M⁺ represents ammonium ion, sodium ion, potassium ion, or silver ion.

The bio-electrode composition including such a component (A) can form a living body contact layer for a bio-electrode that is more excellent in electric conductivity and biocompatibility.

Further, in the present invention, the component (A) preferably comprises an ammonium ion represented by the following general formula (2) as an ammonium ion for forming the ammonium salt, 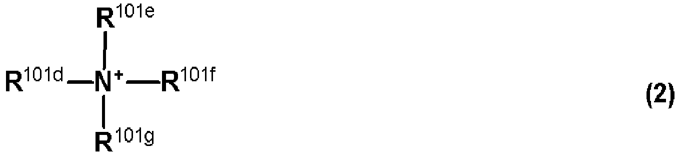 wherein R^{101d}, R^{101e}, R^{101f}, and R^{101g} each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, and optionally have one or more selected from the group consisting of an ether group, a carbonyl group, an ester group, a hydroxy group, an amino group, a nitro group, a sulfonyl group, a sulfinyl group, a halogen atom, and a sulfur atom; and R^{101d} and R^{101e}, or R^{101d}, R^{101e}, and R^{101f}, are optionally bonded to each other together with a nitrogen atom bonded therewith to form a ring in which R^{101d} and R^{101e}, or R^{101d}, R^{101e}, and R^{101f}, represent an alkylene group having 3 to 10 carbon atoms, or to form a heteroaromatic ring having the nitrogen atom in the general formula (2) within the ring.

The bio-electrode composition including the component (A) containing such an ammonium ion can form a living body contact layer for a bio-electrode that is more excellent in electric conductivity and biocompatibility.

Further, in the present invention, the bioelectrode composition preferably further includes a resin other than the component (A) as a component (B).

By including such a component (B), it is possible to prevent elution of salts by being compatible with the component (A) and enhance adhesiveness.

In this event, the component (B) is preferably at least one resin selected from the group consisting of a silicone resin, a (meth)acrylate resin, and a urethane resin.

Such a component (B) can further enhance adhesiveness.

Further, in the present invention, the bioelectrode composition preferably further includes a carbon material and/or a metal powder as a component (C).

With including such a component (C), electron conductivity can be enhanced.

In this event, the carbon material is preferably one or both of carbon black and carbon nanotube.

With including such a carbon material, higher electric conductivity can be achieved.

Further, in the present invention, the metal powder is preferably a metal powder selected from the group consisting of gold, silver, platinum, copper, tin, titanium, nickel, aluminum, tungsten, molybdenum, ruthenium, chromium, and indium.

Such metals can be used as a metal powder.

In this event, the metal powder is preferably a silver powder.

The silver powder is preferable comprehensively in terms of electric conductivity, price, and biocompatibility.

Further, in the present invention, the bioelectrode composition preferably further includes an organic solvent as a component (D).

With including such a component (D), high coatability can be achieved.

Further, the present invention provides a bioelectrode including an electro-conductive base material and a living body contact layer formed on the electro-conductive base material, wherein the living body contact layer is a cured product of the bio-electrode composition described above.

Such a bio-electrode can be excellent in electric conductivity and biocompatibility, light-weight, manufacturable at low cost, capable of preventing significant reduction in the electric conductivity even when wetted with water or dried, soft, and excellent in stretchability and strength.

In this event, the electro-conductive base material preferably includes one or more selected from the group consisting of gold, silver, silver chloride, platinum, aluminum, magnesium, tin, tungsten, iron, copper, nickel, stainless steel, chromium, titanium, carbon, and an electro-conductive polymer.

Such an electro-conductive base can be used as the electro-conductive base material.

Further, the present invention provides a method for manufacturing a bio-electrode having an electro-conductive base material and a living body contact layer formed on the electro-conductive base material, the method which includes applying the bio-electrode composition described above onto the electro-conductive base material and curing the bio-electrode composition to form the living body contact layer.

According to such a method for manufacturing a bio-electrode, it is possible to manufacture easily a bio-electrode that is excellent in electric conductivity and biocompatibility, light-weight, manufacturable at low cost, capable of preventing significant reduction in the electric conductivity even when wetted with water or dried, soft, and excellent in stretchability and strength.

In this event, the electro-conductive base material includes one or more selected from the group consisting of gold, silver, silver chloride, platinum, aluminum, magnesium, tin, tungsten, iron, copper, nickel, stainless steel, chromium, titanium, carbon, and an electro-conductive polymer.

Such an electro-conductive base can be used as the electro-conductive base material.

### ADVANTAGEOUS EFFECTS OF INVENTION

As described above, the bio-electrode composition, the bio-electrode, and the method for manufacturing the bio-electrode of the present invention can provide a bio-electrode composition capable of forming a living body contact layer for a bio-electrode that is excellent in electric conductivity and biocompatibility, light-weight, manufacturable at low cost, capable of preventing significant reduction in the electric conductivity even when wetted with water or dried, soft, and excellent in stretchability and strength; a bioelectrode having a living body contact layer formed of the bio-electrode composition; and a method for manufacturing the bio-electrode.

### BRIEF DESCRIPTION OF DRAWINGS

FIG. 1 is an example of a schematic crosssectional view of the inventive bio-electrode.
FIG. 2 is an example of a schematic crosssectional view of the inventive bio-electrode when attached to the skin.
FIG. 3 is a schematic view after printing of an electrode prepared in Examples of the present invention.
FIG. 4 is a schematic view of an electrode with an adhesive layer and wiring attached after one of bio electrodes prepared in Examples of the present invention is cutting off.
FIG. 5 shows locations where bio-electrodes and an earth were attached on the human body when measuring biological signals in Examples of the present invention.
FIG. 6 is an electrocardiogram waveform obtained using the bio-electrodes in Examples of the present invention.

### DESCRIPTION OF EMBODIMENTS

As described in the above, it is desire to develop the bio-electrode composition capable of forming a living body contact layer for a bio-electrode that is excellent in electric conductivity and biocompatibility, light-weight, manufacturable at low cost, soft, having stretchability and strength, capable of collecting biological signal stably even when attached on the skin for long time and when wetted with water in bathing or the like or dried; a bioelectrode having a living body contact layer formed of the bio-electrode composition; and a method for manufacturing the bio-electrode.

Ions of sodium, potassium, and calcium are released from the surface of the skin, in accordance with heartbeats. A bio-electrode has to convert the increase and decrease of ions released from the skin to electric signals. Accordingly, the bio-electrode requires a material that is excellent in ionic conductivity to transmit the increase and decrease of ions.

An electrode of a watch-typed biological monitor uses metal as described before. Metal is not only low in sensitivity for biological data but also capable of causing an allergy to a certain number of users. If a rubber band used for a watch works as a bio-electrode, a metal allergy issue is eliminated. In general, however, a rubber band has insulation properties and does not work as a bio-electrode.

A bio-electrode of the watch rubber band requires strong properties because the bio-electrode should not be peeled off or cracked due to friction with the skin. It is also required to have strong adhesiveness with metal wiring in a rubber band and high stretchability.

On the other hand, a bio-electrode attached to the body requires strong adhesiveness because the bioelectrode should keep attached to the body for a long time and even with body movements in doing sport.

Materials having high strong properties, high stretchability, and high adhesiveness can include polyurethane. Since polyurethane has insulation properties, it is conceivable to blend with additives for the purpose of imparting ionic conductivity and electron conductivity. For the purpose of improving ionic conductivity, it is conceivable to add a neutralizing salt.

When an acid that forms a neutralized salt is highly acidic, ions are strongly polarized, and ionic conductivity increases. This is why lithium salts of bis(trifluoromethanesulfonyl)imidic acid and tris(trifluoromethanesulfonyl)methide acid show high ionic conductivity in case of a lithium ion battery. On the other hand, the higher acidity the acid before neutralization as a salt has, there is a problem where the resultant salt has stronger irritation on the body. That is, the relation between ionic conductivity and irritation to the body is a trade-off relation. However, a salt applied to a bio-electrode must achieve both high ionic conductivity and low irritation to the body.

An ion compound having higher molecular weight has a property that the permeability is less and irritation to the skin is decreased. Accordingly, an ion compound is preferably a polymer type with higher molecular weight. Hence, the present inventors have solved the problem of irritation to the skin by incorporating such an ionic compound into a polyurethane.

That is, the inventive bio-electrode composition includes, as the component (A), a resin which has a urethan bond in a main chain and at least one salt selected from the group consisting of salts of ammonium, sodium, potassium, and silver formed with sulfonamide in a side chain.

The present invention is described below in detail, but not limited thereto.

### <Bio-Electrode Composition>

The inventive bio-electrode composition includes, as the component (A), a resin which has a urethan bond in a main chain and at least one salt selected from the group consisting of salts of ammonium, sodium, potassium, and silver formed with sulfonamide in a side chain. Each component will be explained in more detail below.

[(A) Resin which has a urethan bond in a main chain and at least one salt selected from the group consisting of salts of ammonium, sodium, potassium, and silver formed with sulfonamide in a side chain]

The inventive bio-electrode composition includes, as the component (A), a resin which has a urethan bond in a main chain and at least one salt selected from the group consisting of salts of ammonium, sodium, potassium, and silver formed with sulfonamide in a side chain.

The component (A) is preferably represented by the following general formula (1).

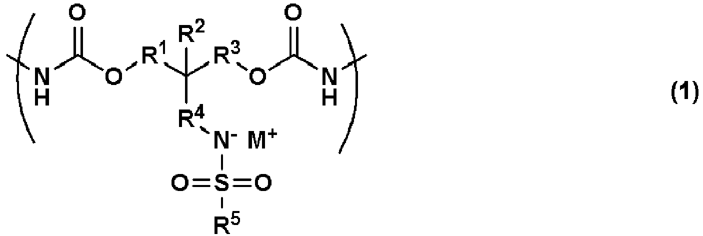

In the general formula (1), R¹ and R³ represent a single bond, or a linear or branched alkylene group having 1 to 6 carbon atoms, and optionally have an ether bond or a bromine atom. R² represents a hydrogen atom, a hydroxy group, or a linear or branched alkyl or alkoxy group having 1 to 6 carbon atoms. R⁴ represents a single bond or a linear, branched, or cyclic hydrocarbylene group having 1 to 20 carbon atoms, and optionally have a carbonyl group, an ether group, or an ester group, or optionally form a ring together with R¹. R⁵ represents a linear, branched, or cyclic alkyl group having 1 to 10 carbon atoms and optionally substituted with a fluorine atom, or R⁵ represents an alkenyl group having 2 to 6 carbon atoms, an alkynyl group having 2 to 6 carbon atoms, or an aryl group having 6 to 10 carbon atoms and optionally has a fluorine atom, a trifluoromethyl group, a cyano group, a nitro group, a hydroxy group, an alkoxy group having 1 to 6 carbon atoms, or an alkyl group having 1 to 6 carbon atoms. M⁺ represents ammonium ion, sodium ion, potassium ion, or silver ion.

A repeating unit represented by the general formula (1) can be obtained by a reaction between a monomer represented by the following general formula (1)-1 having a dihydroxy group and a compound having an isocyanate group.

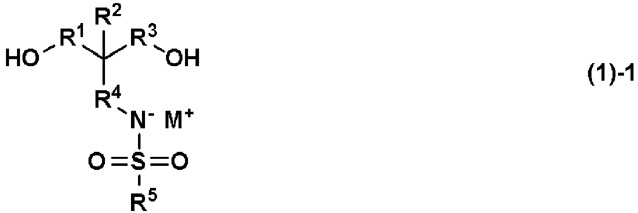

In the general formula (1)-1, R¹ to R⁵ and M⁺ represent as described before.

Specific examples of the monomer represented by the general formula (1)-1 having a dihydroxy group can be exemplified below.

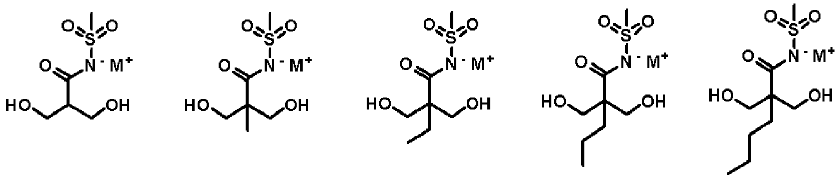

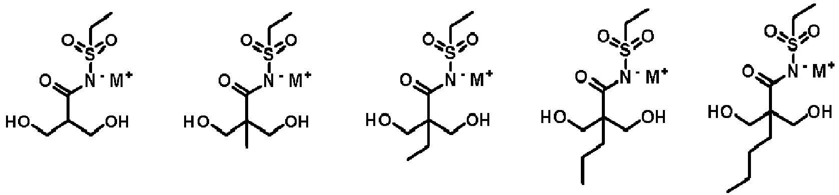

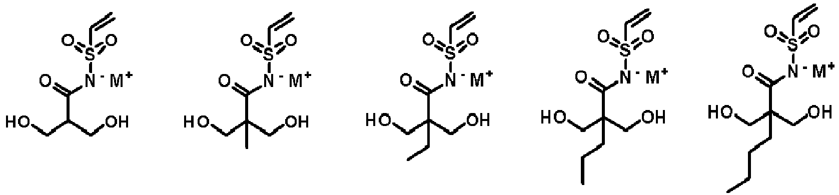

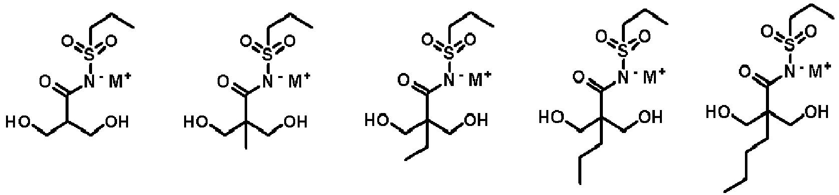

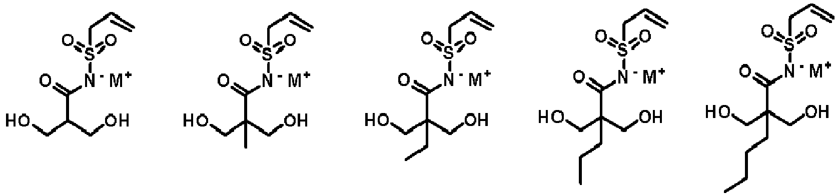

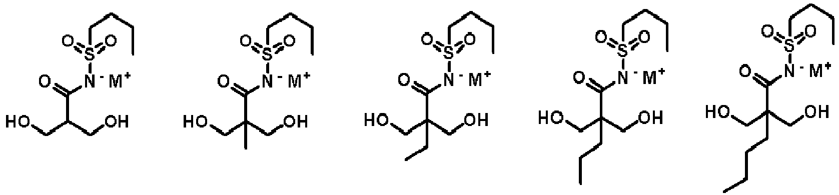

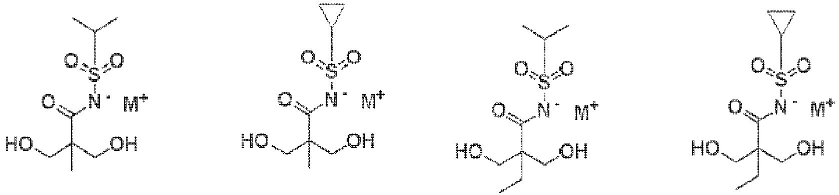

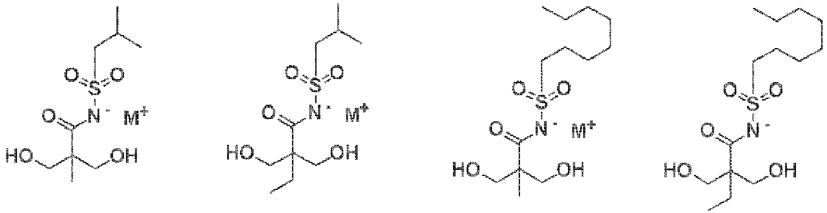

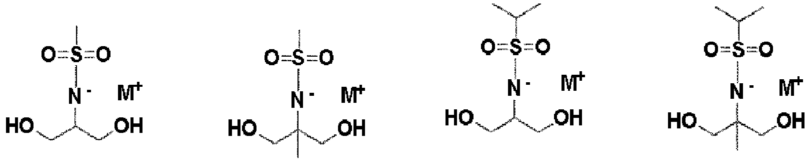

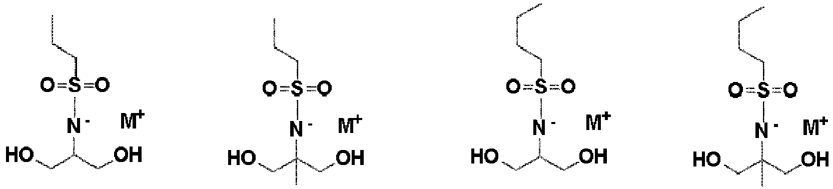

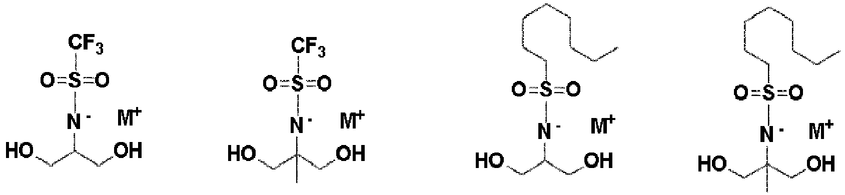

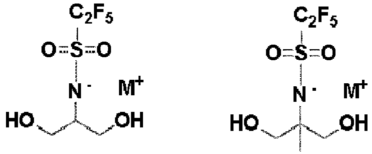

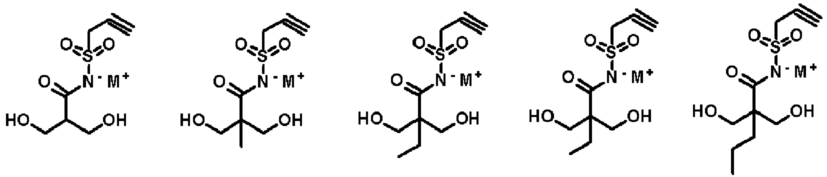

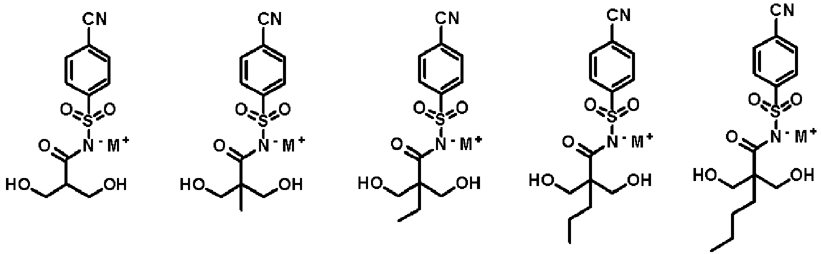

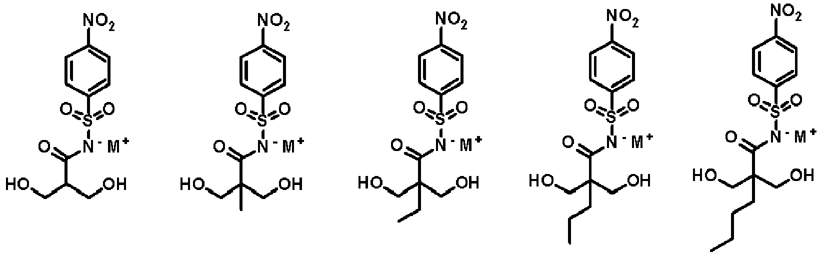

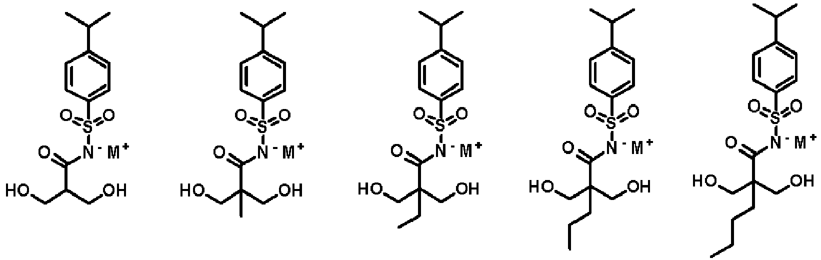

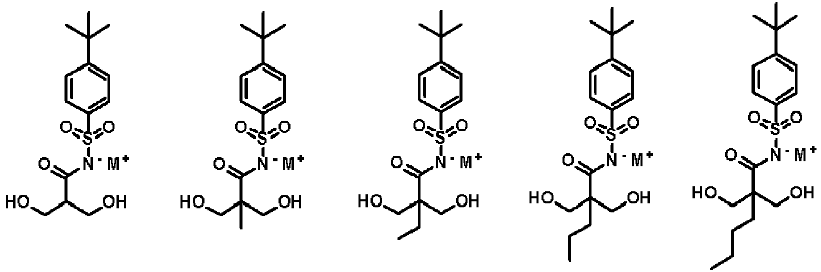

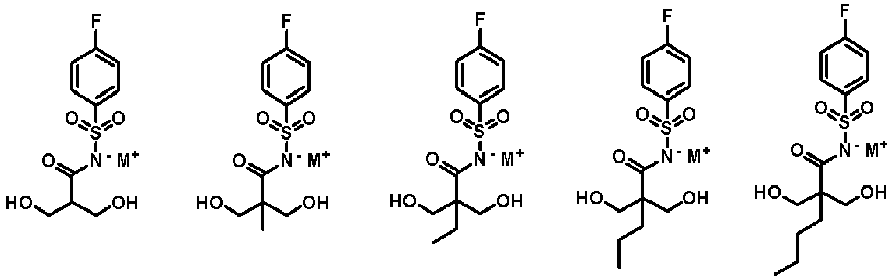

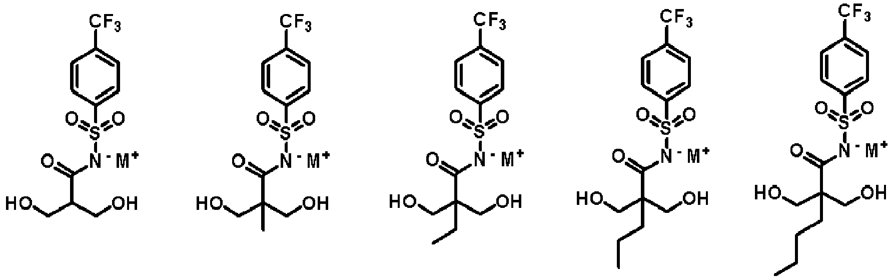

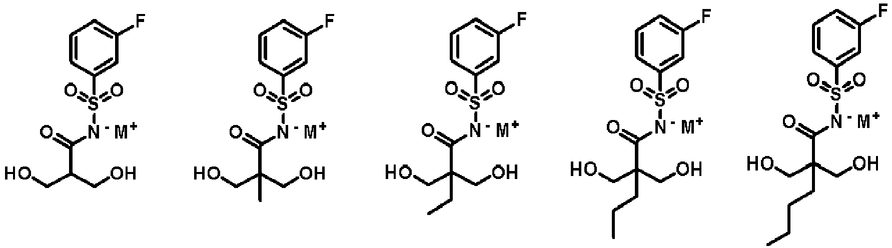

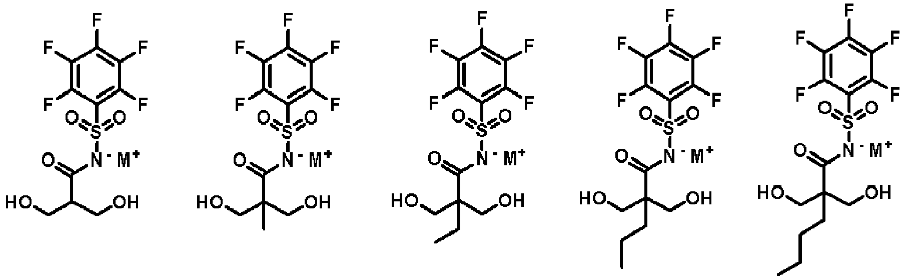

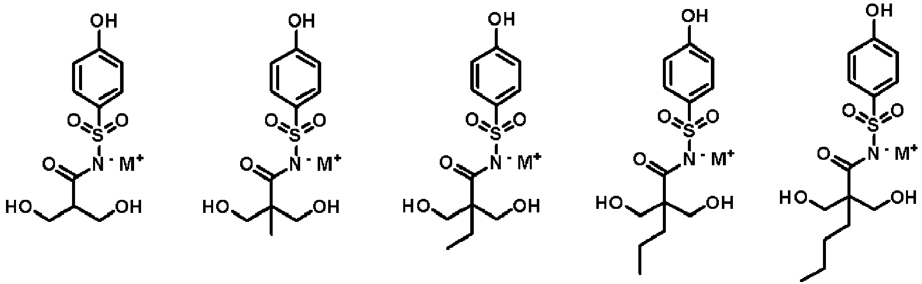

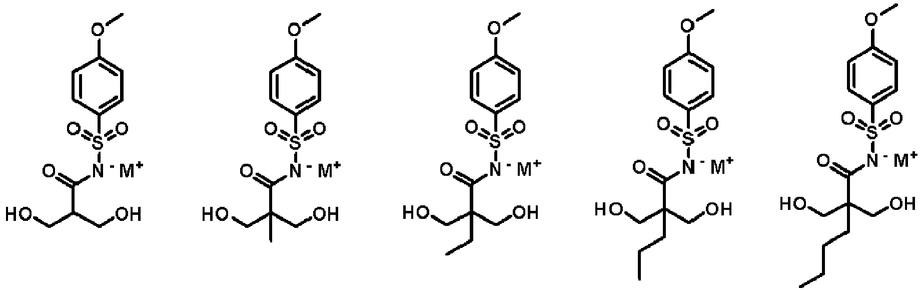

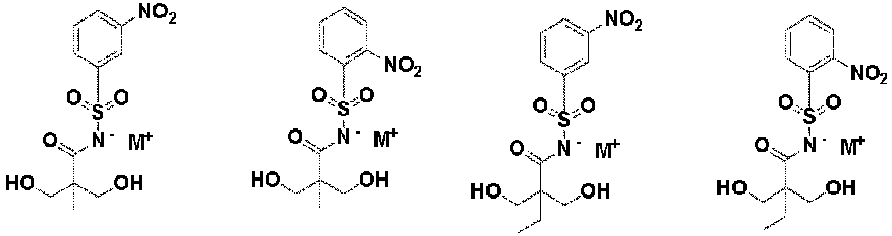

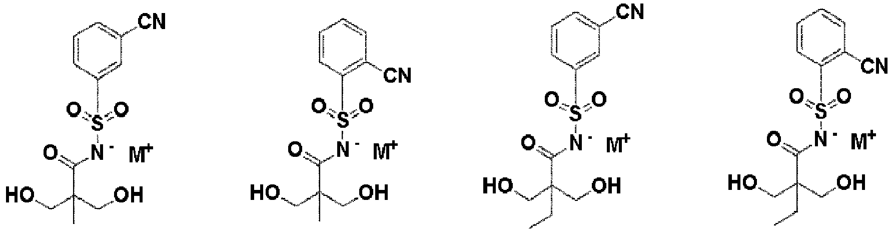

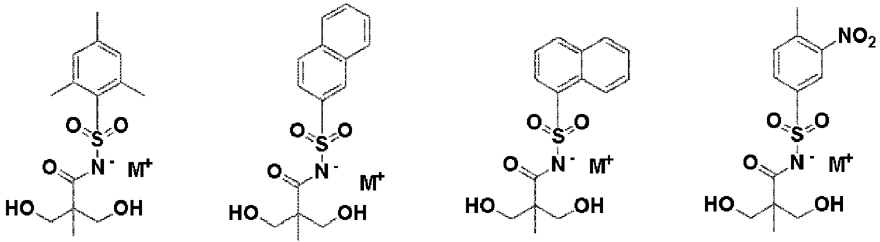

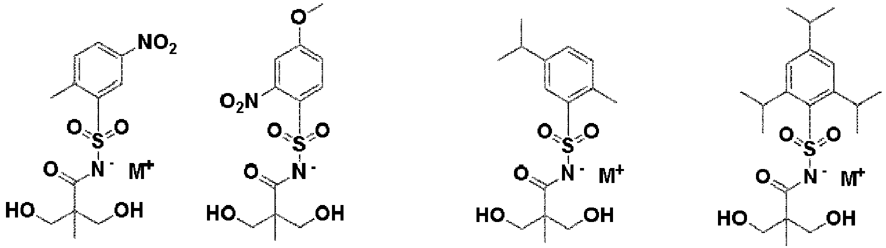

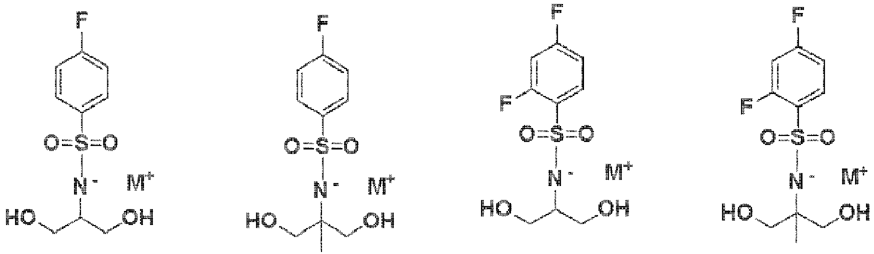

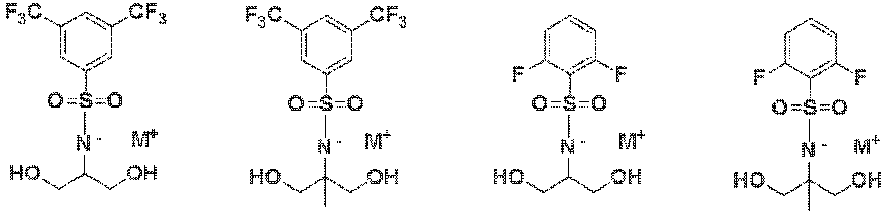

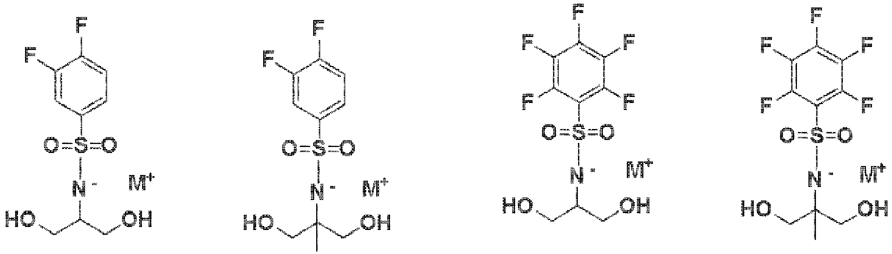

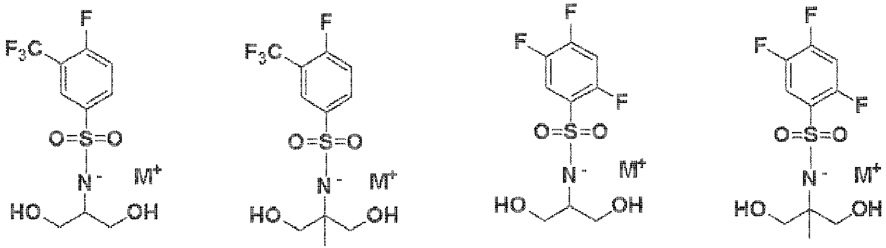

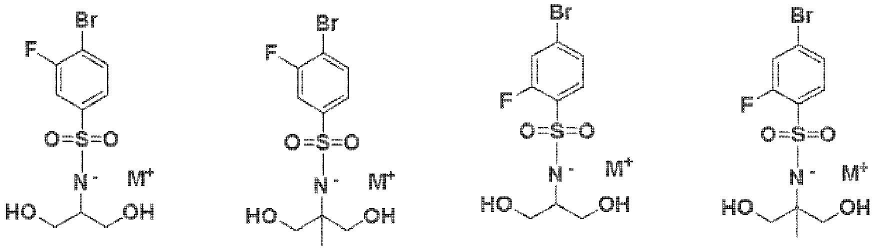

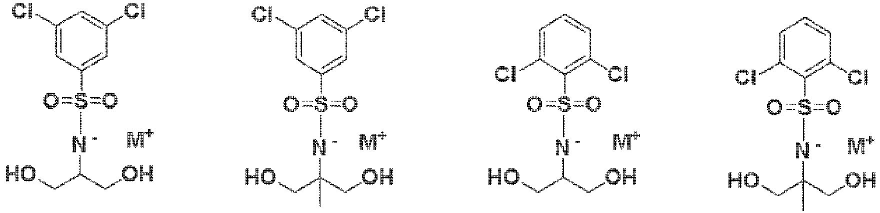

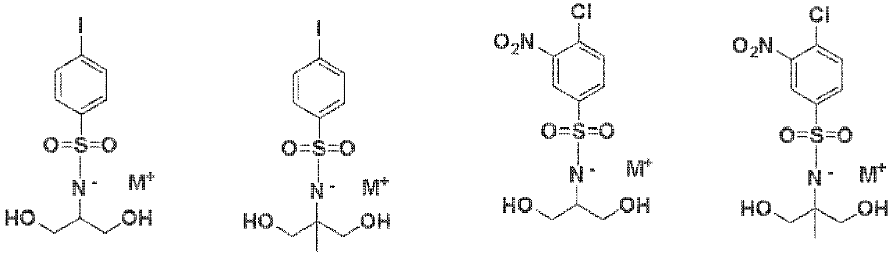

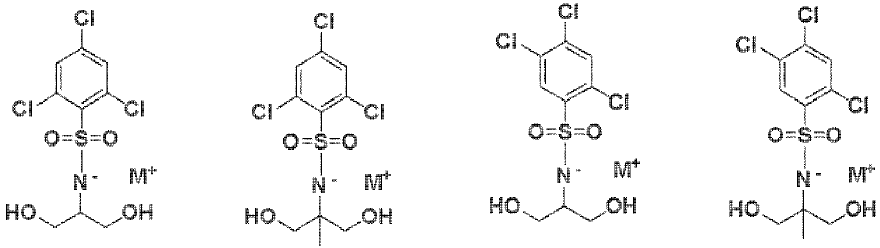

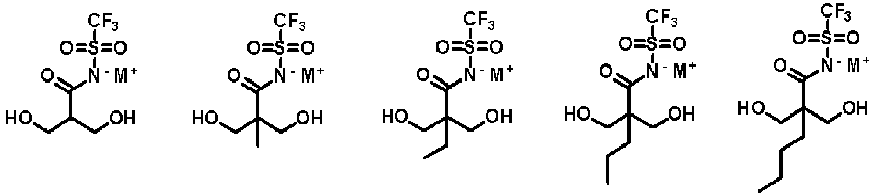

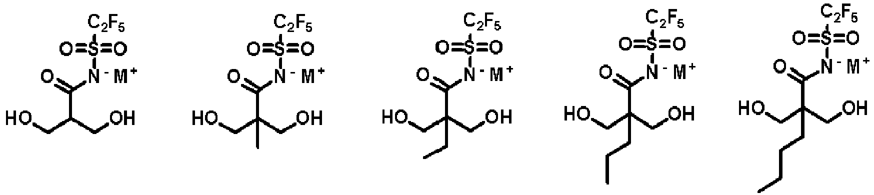

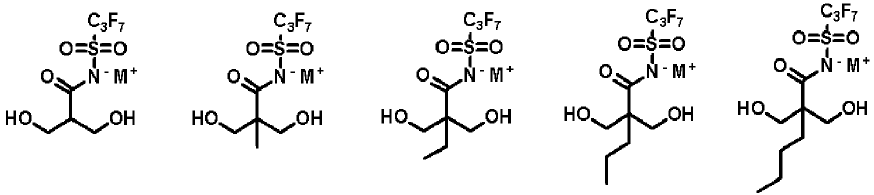

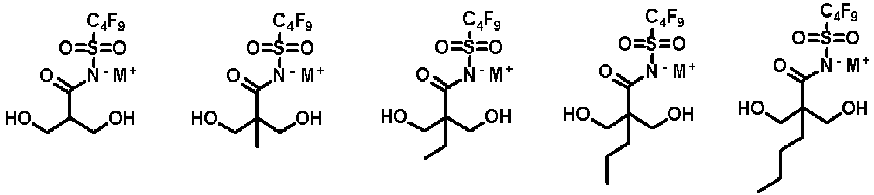

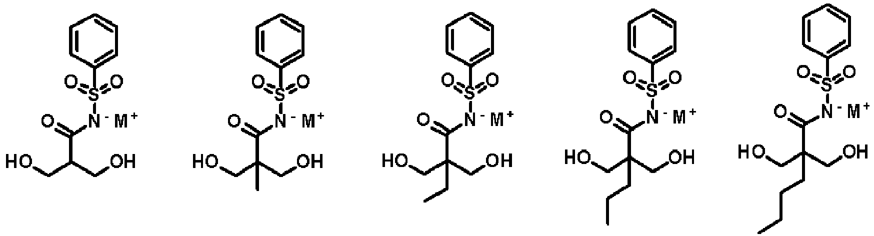

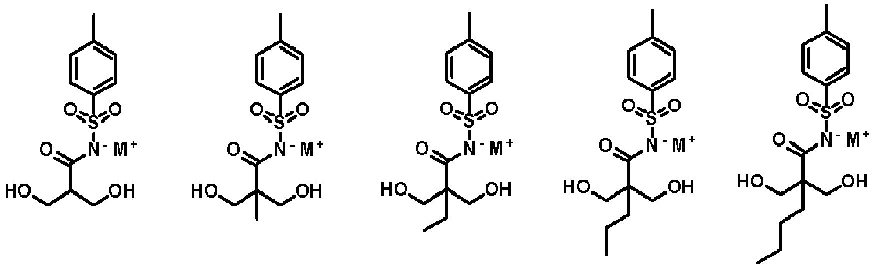

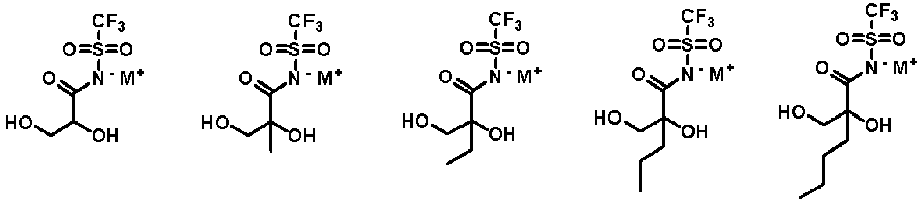

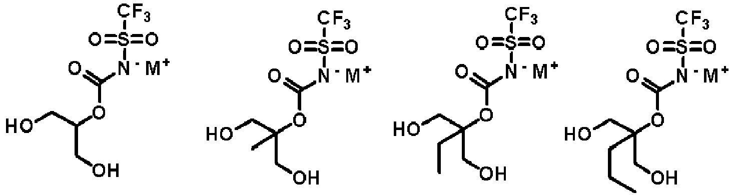

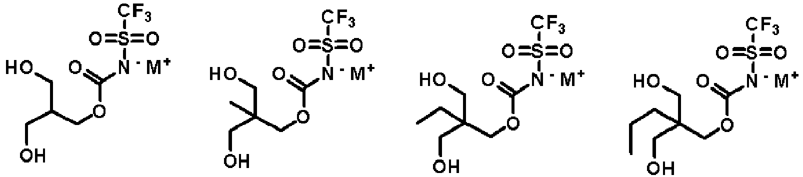

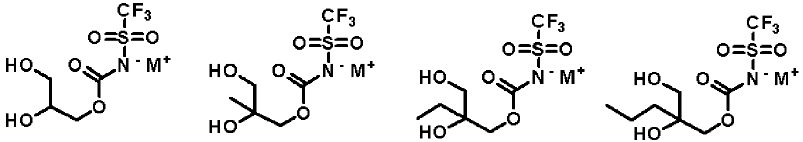

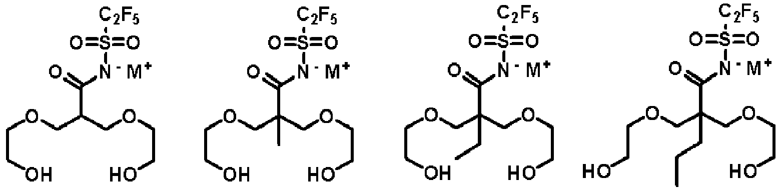

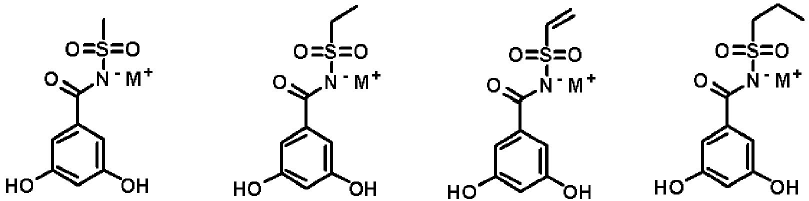

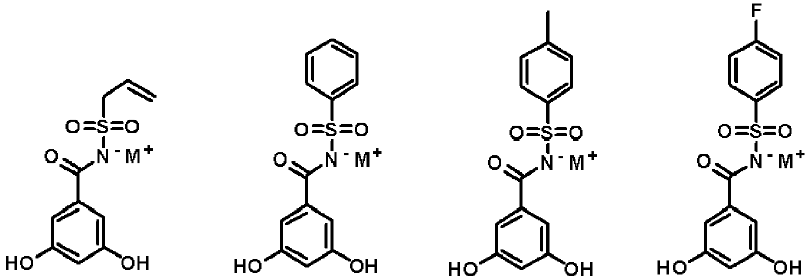

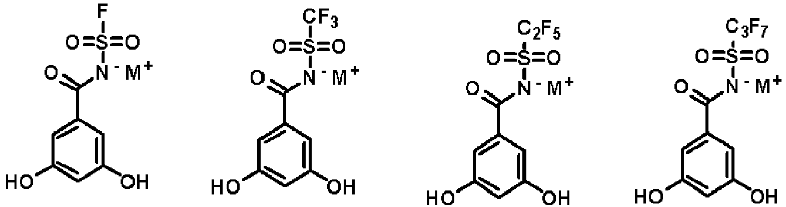

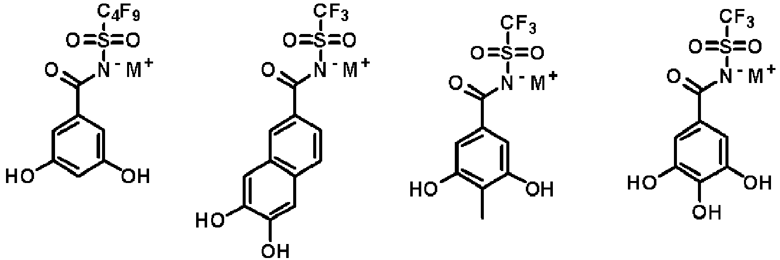

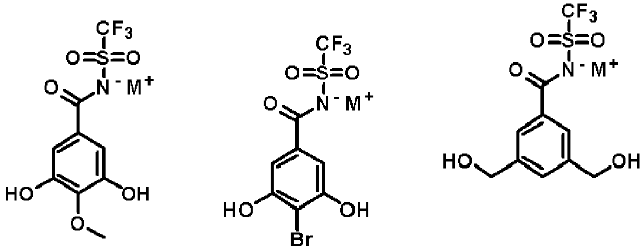

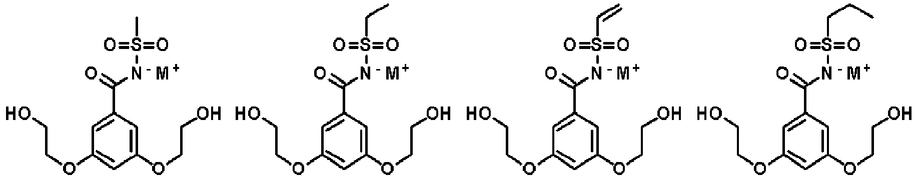

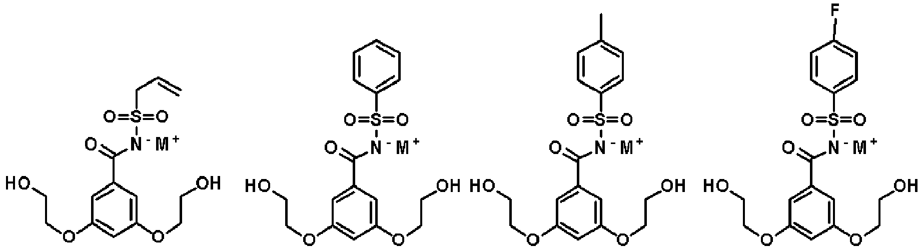

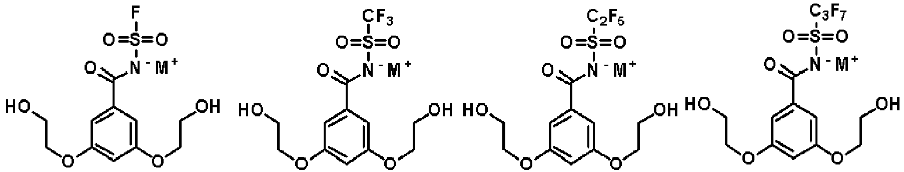

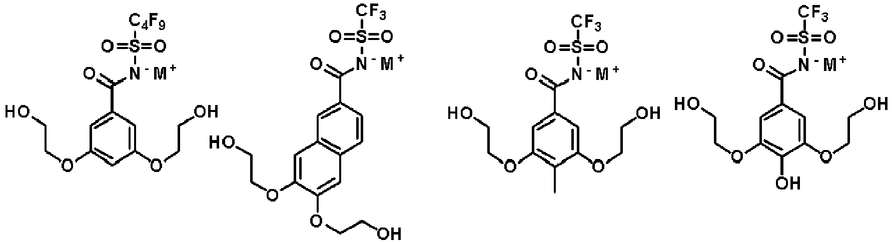

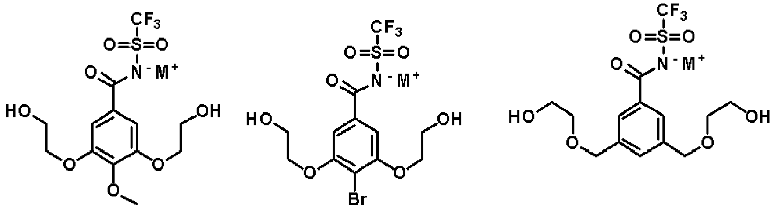

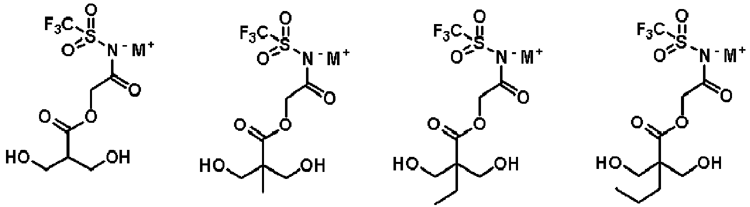

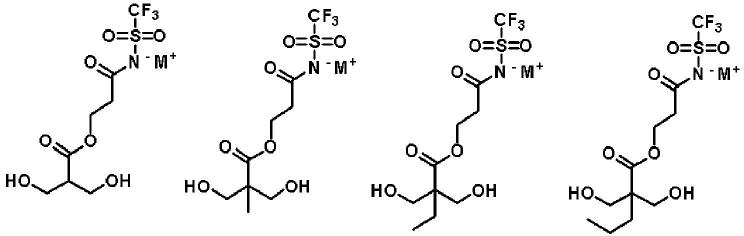

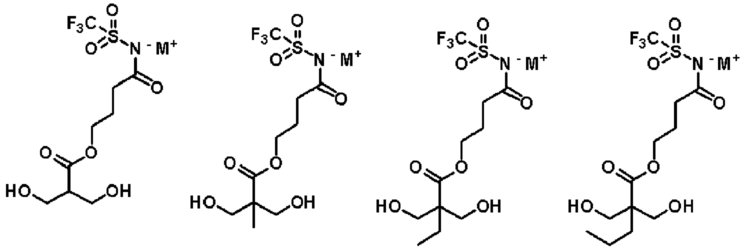

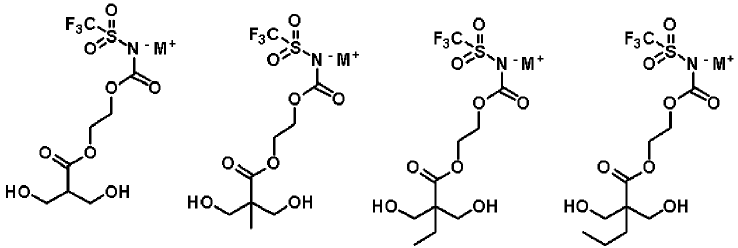

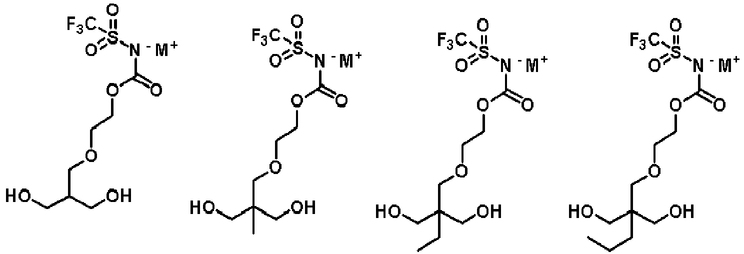

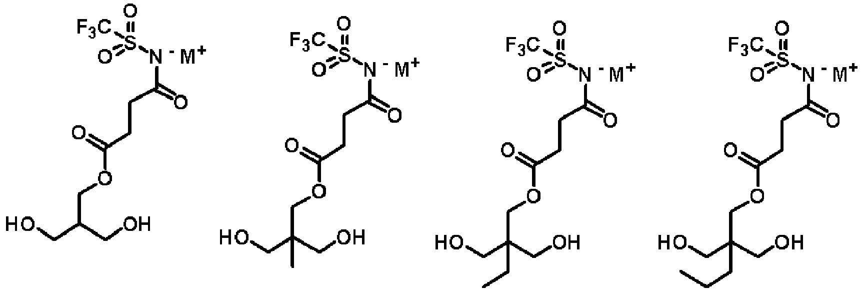

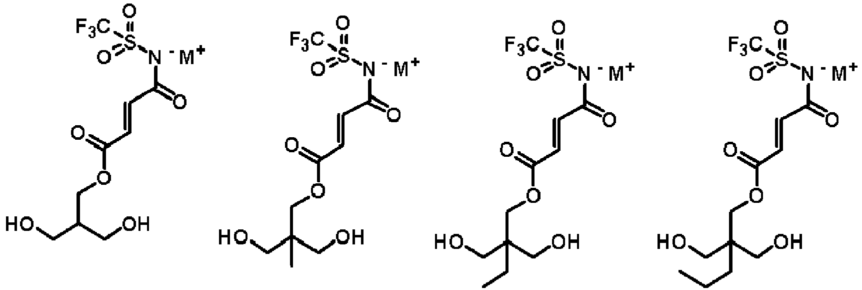

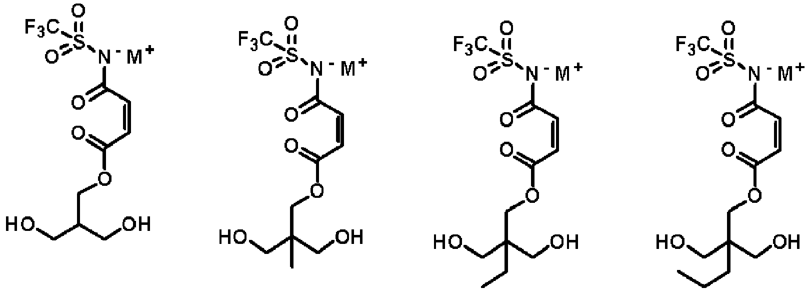

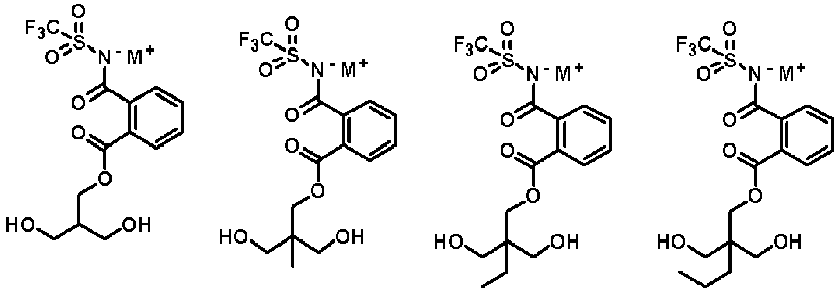

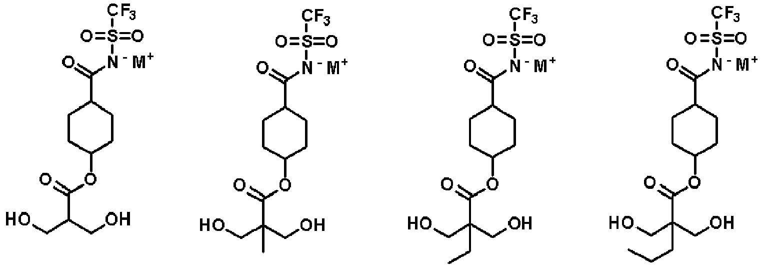

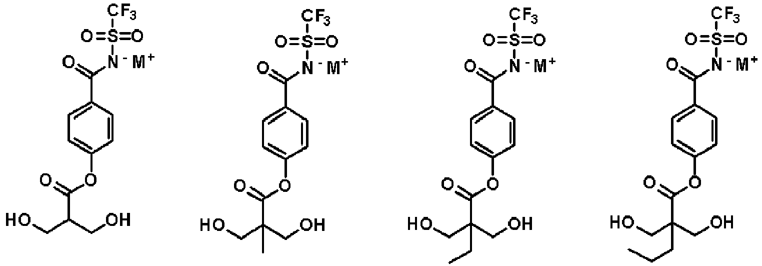

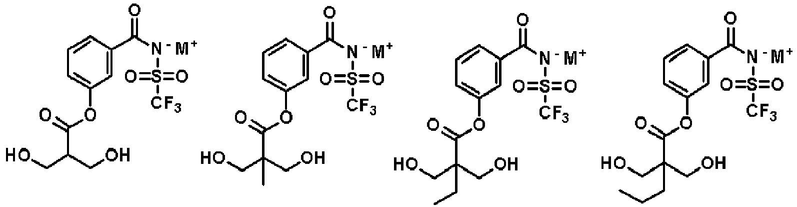

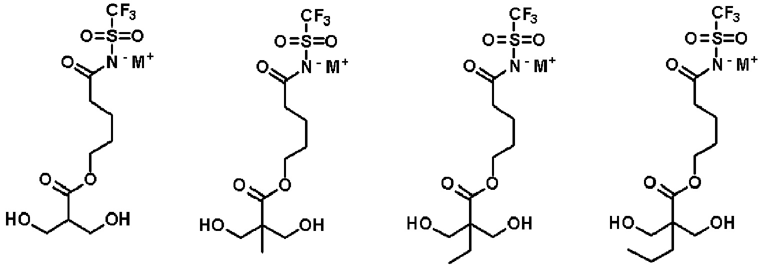

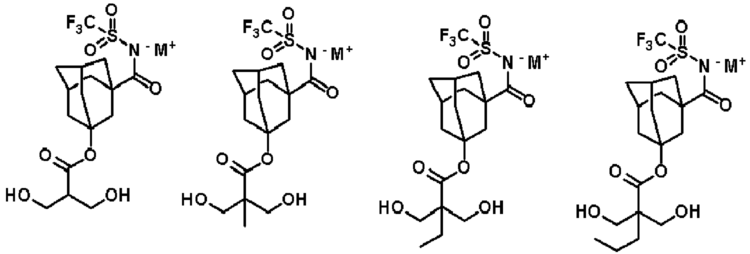

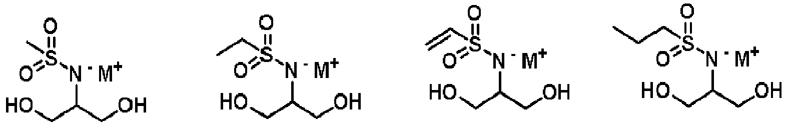

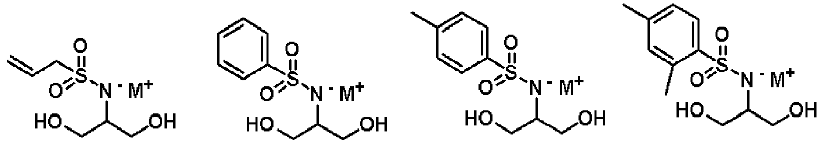

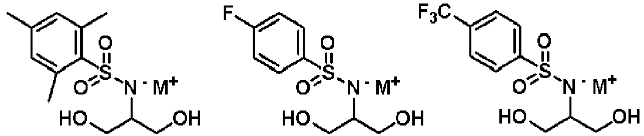

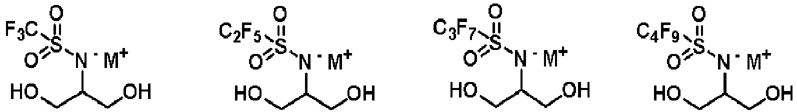

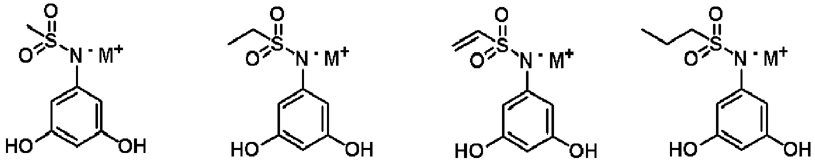

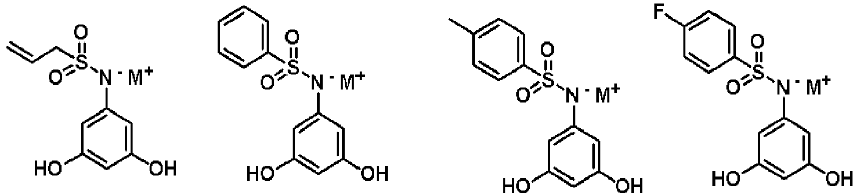

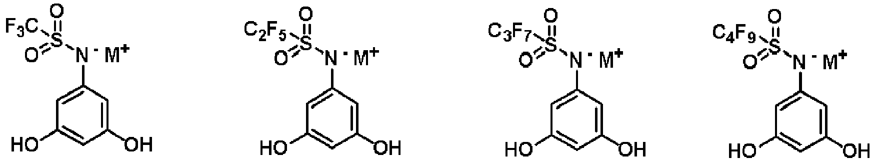

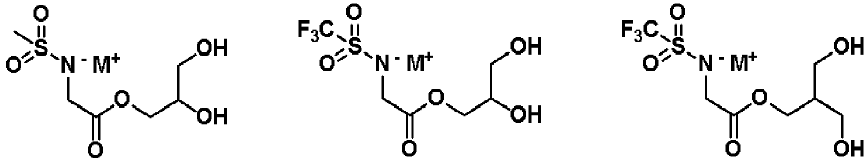

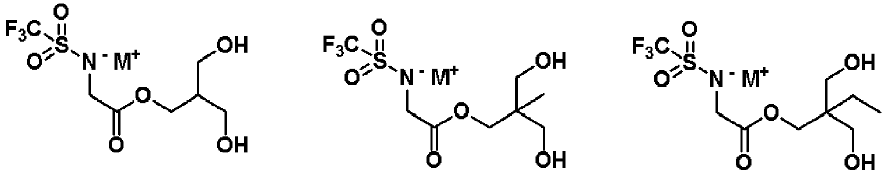

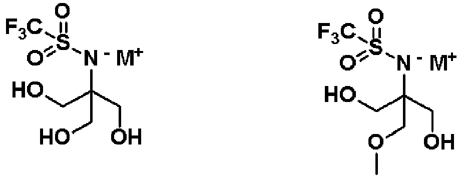

The dihydroxy group-containing N-carbonyl-sulfonamide salt described above can be obtained first by synthesizing an acid chloride from a carboxylic acid compound as disclosed in paragraph [0176] of JP 2022-078861 A and then by reacting the obtained acid chloride with sulfonamide as disclosed in paragraph [0132] of JP 2019-011454 A.

The dihydroxy group-containing sulfonamide salt described above can be obtained first by synthesizing a sulfonamide as disclosed in paragraph [0177] of JP 2022-078861 A and then by subjecting the obtain sulfonamide to neutralization or ion exchange with ammonium hydroxide, ammonium chloride, sodium hydroxide, sodium chloride, potassium hydroxide, potassium chloride, silver hydroxide, silver chloride, silver nitrate, etc.

The component (A) preferably includes an ammonium ion represented by the following general formula (2) as an ammonium ion for forming the ammonium salt.

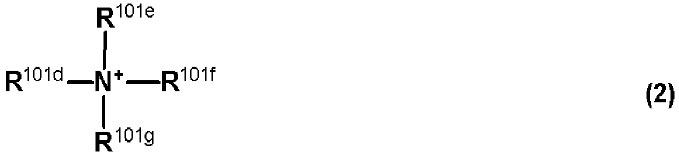

In the general formula (2), R^{101d}, R^{101e}, R^{101f}, and R^{101g} each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, and optionally have one or more selected from the group consisting of an ether group, a carbonyl group, an ester group, a hydroxy group, an amino group, a nitro group, a sulfonyl group, a sulfinyl group, a halogen atom, and a sulfur atom. R^{101d} and R^{101e}, or R^{101d}, R^{101e}, and R^{101f}, are optionally bonded to each other together with a nitrogen atom bonded therewith to form a ring in which R^{101d} and R^{101e}, or R^{101d}, R^{101e}, and R^{101f}, represent an alkylene group having 3 to 10 carbon atoms, or to form a heteroaromatic ring having the nitrogen atom in the general formula (2) within the ring.

Specific examples of the ammonium ion represented by the above general formula (2) can be exemplified below.

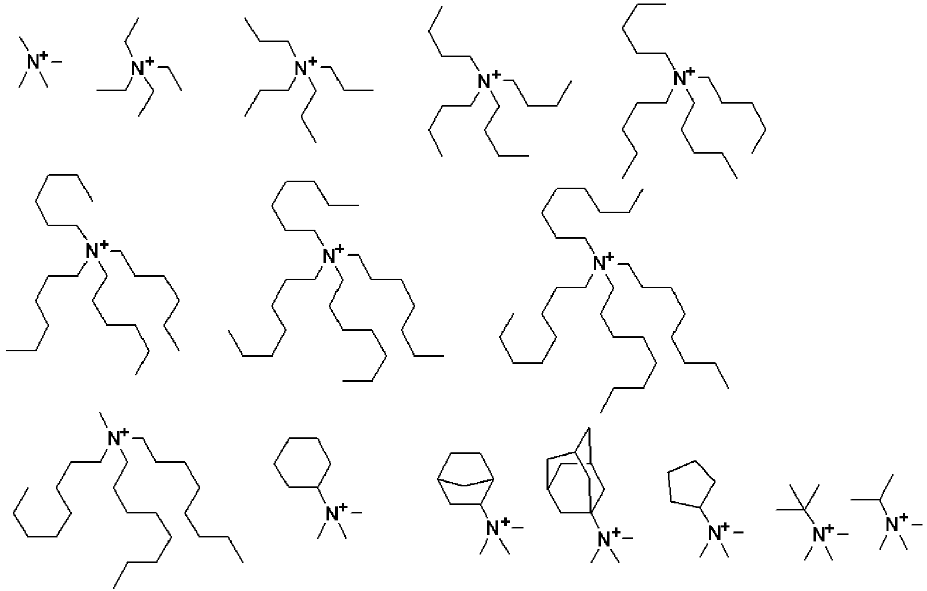

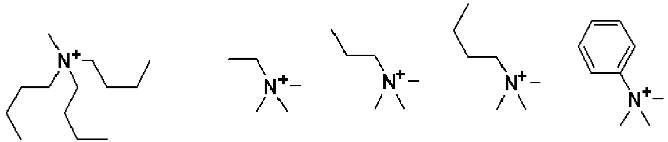

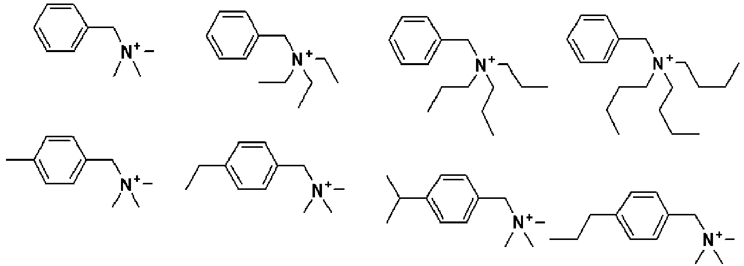

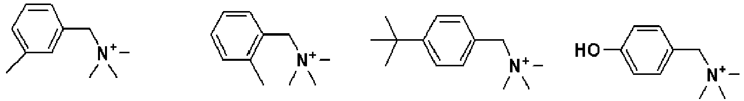

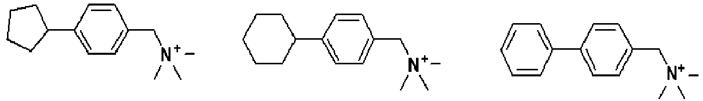

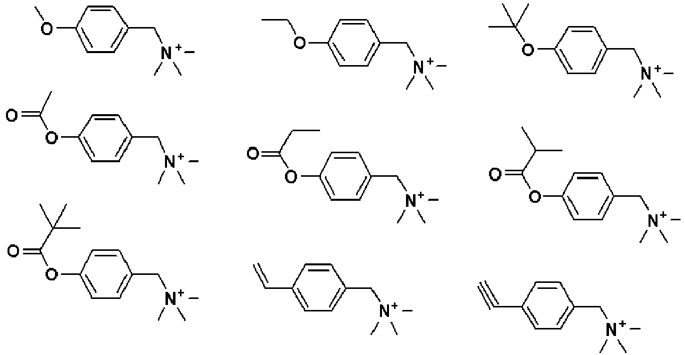

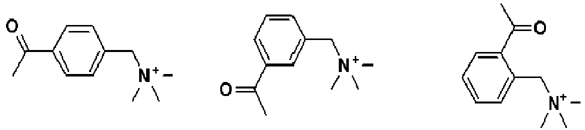

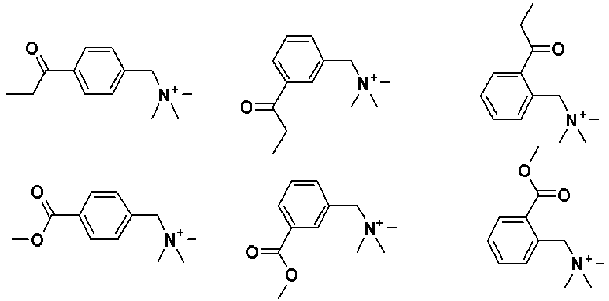

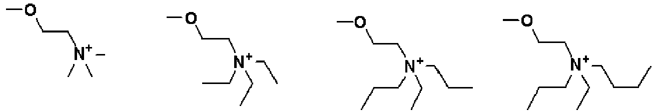

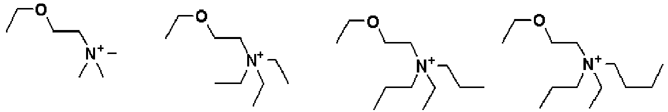

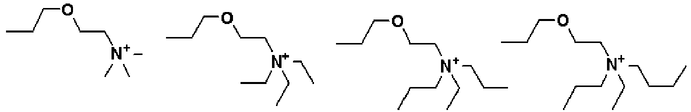

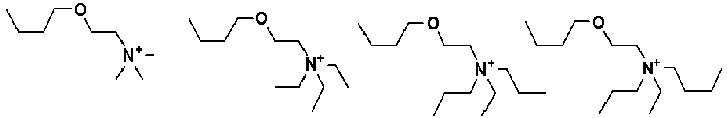

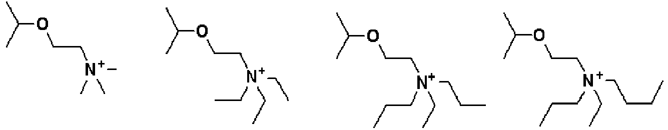

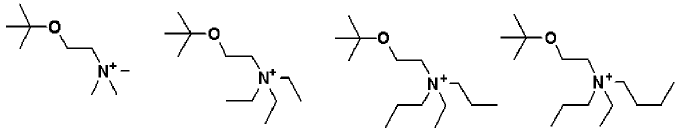

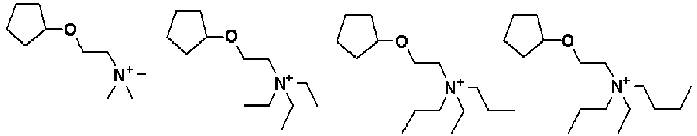

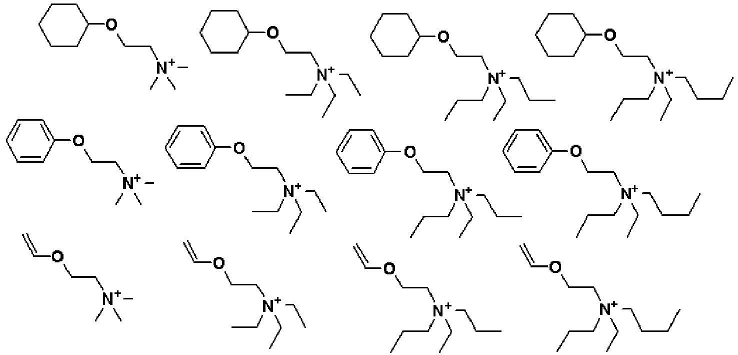

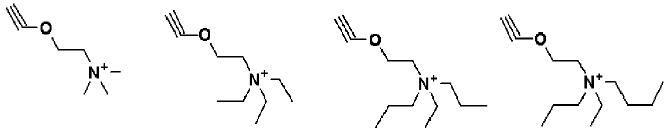

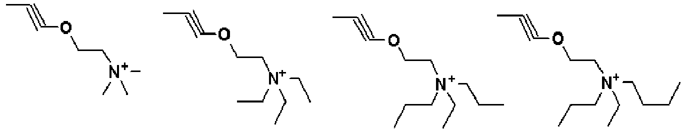

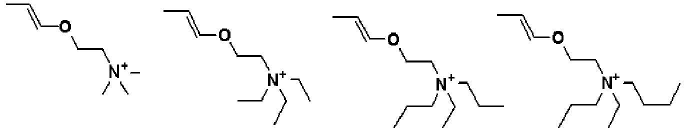

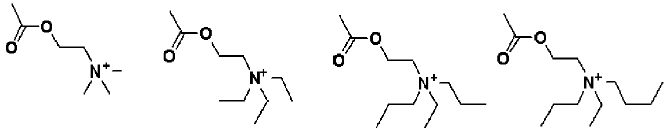

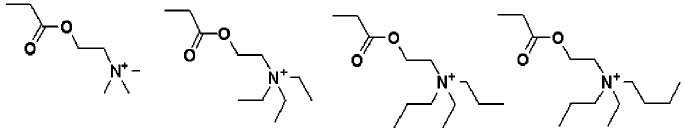

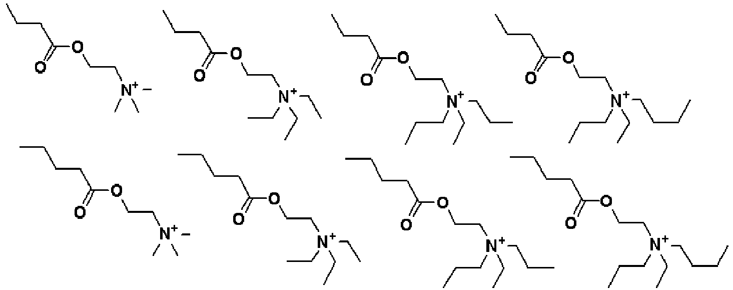

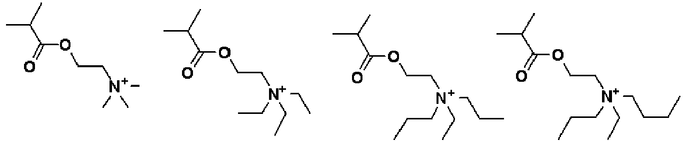

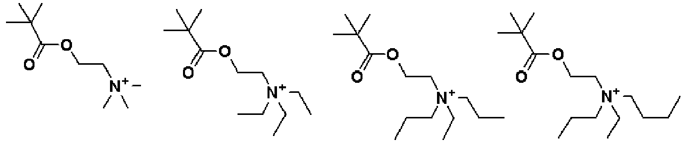

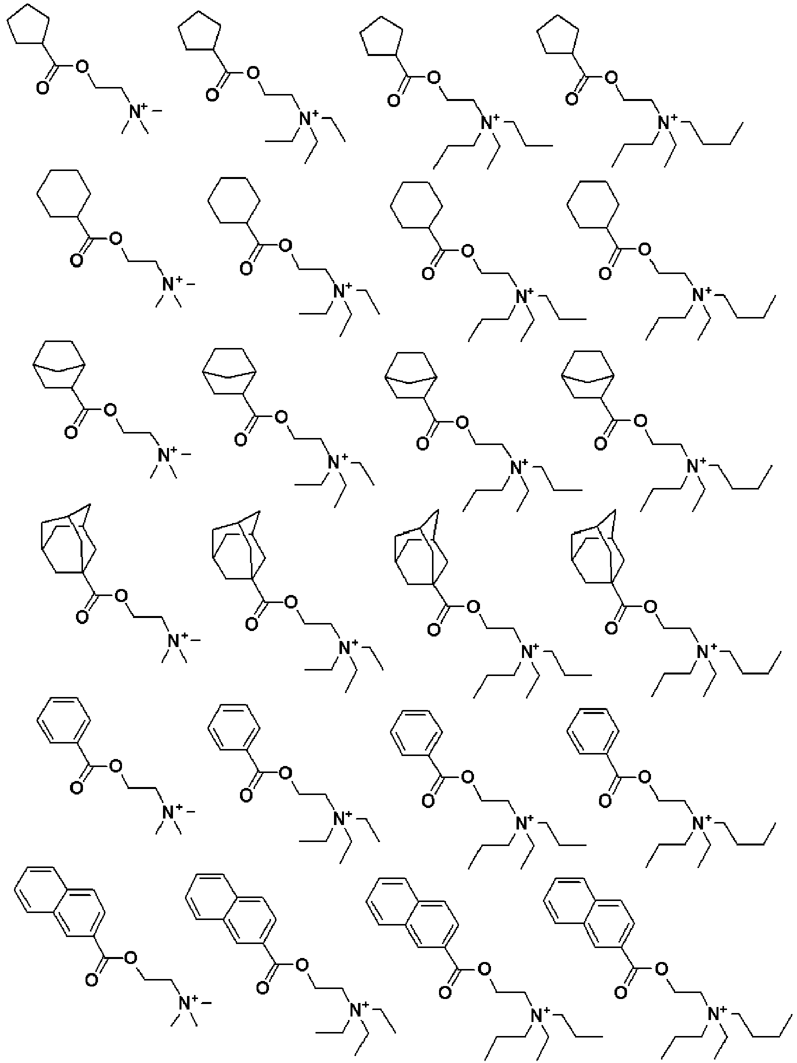

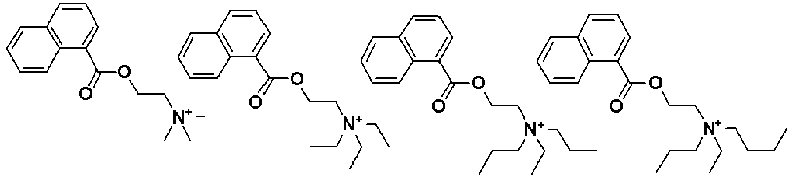

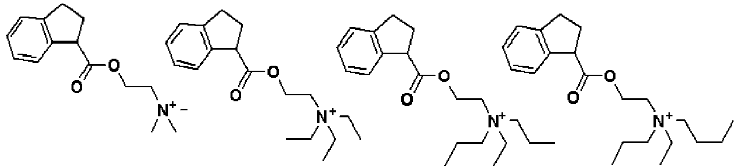

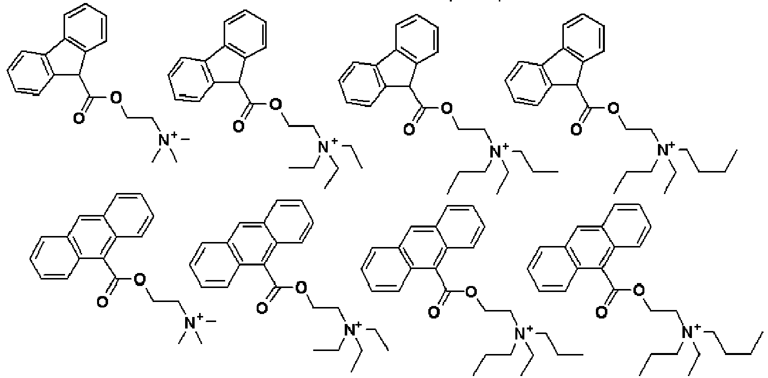

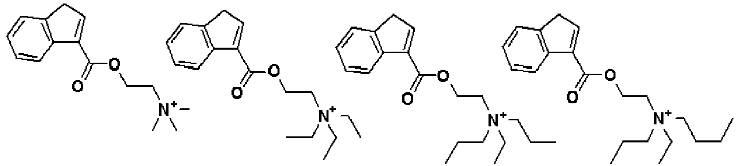

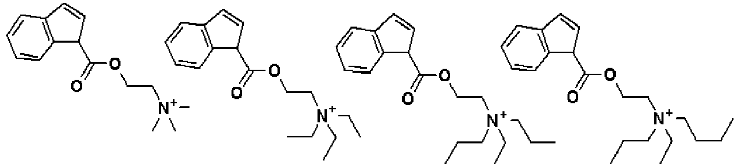

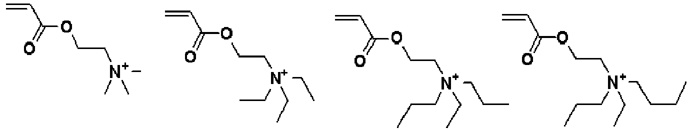

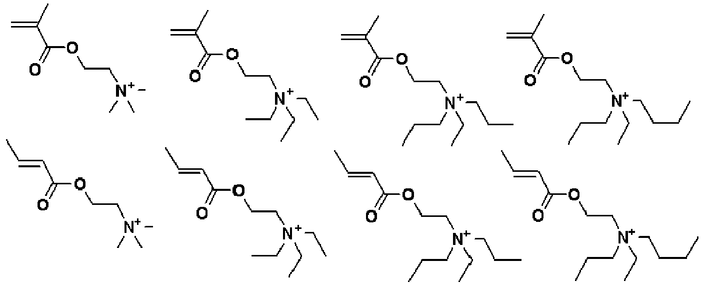

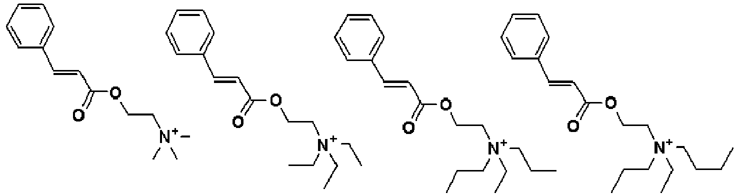

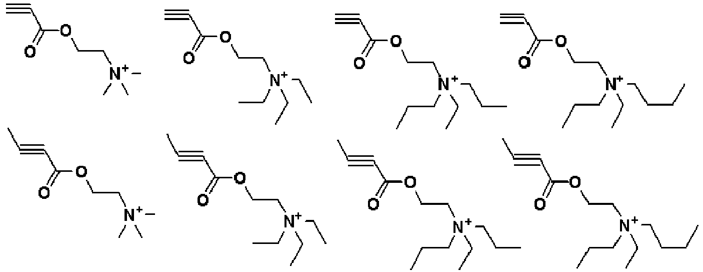

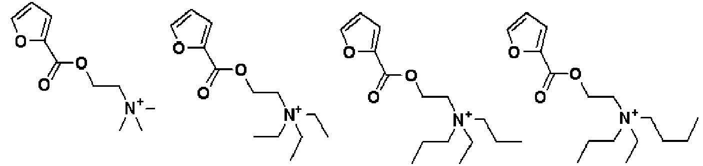

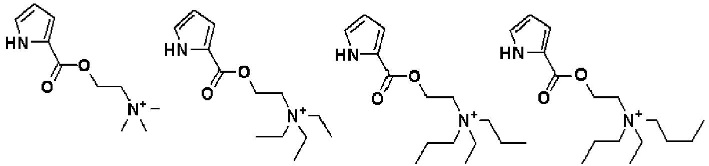

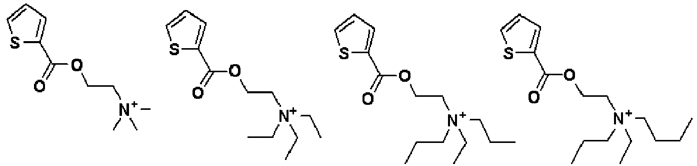

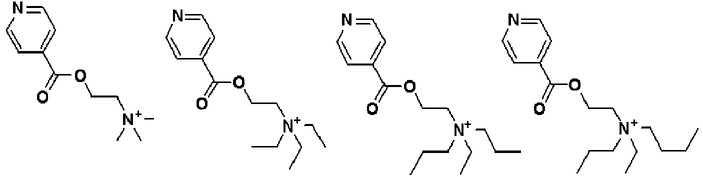

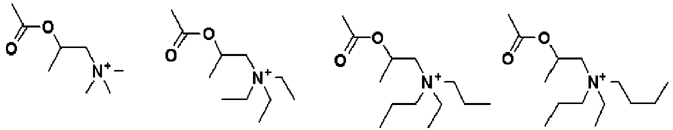

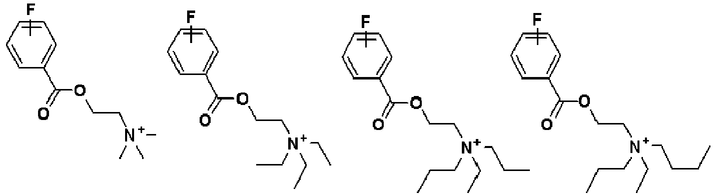

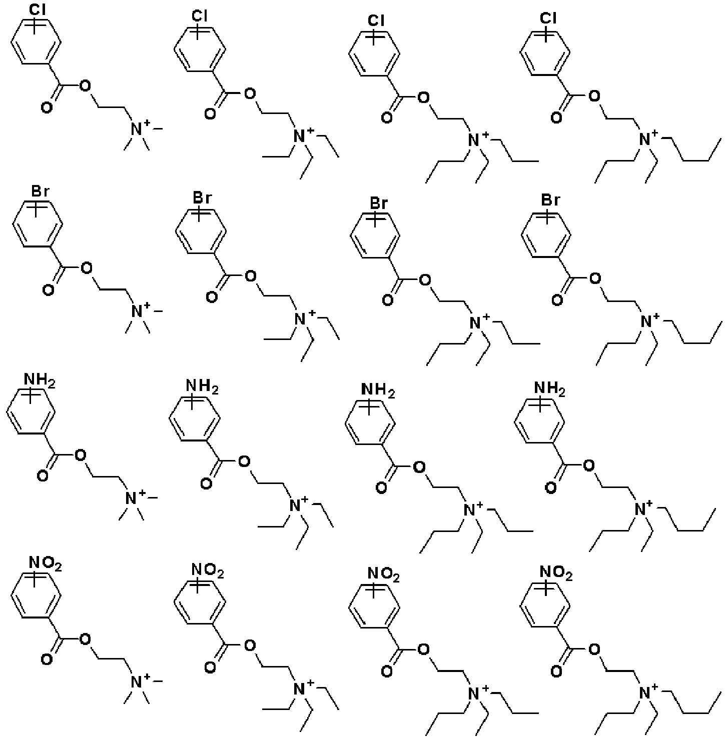

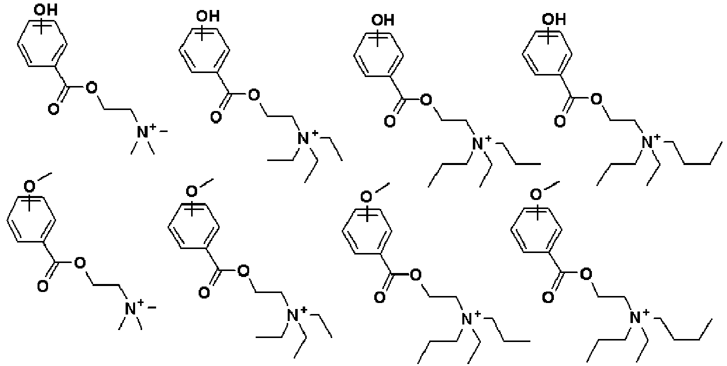

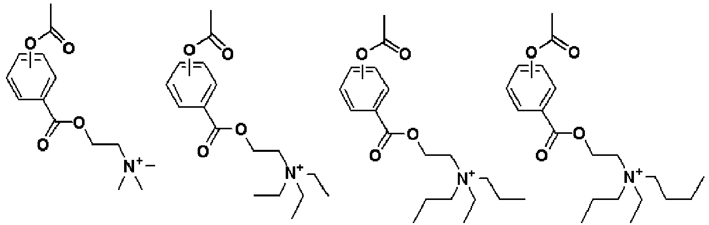

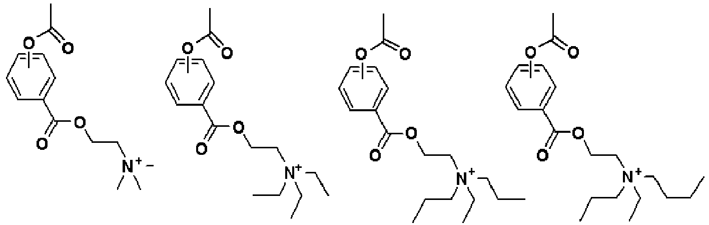

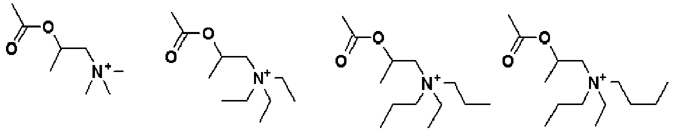

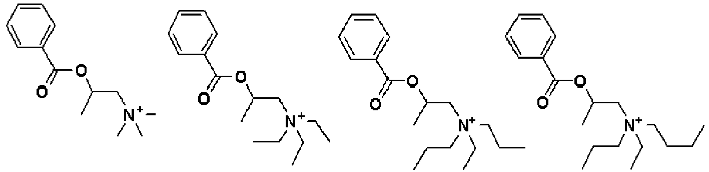

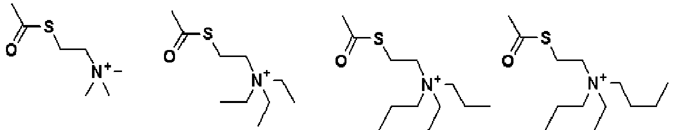

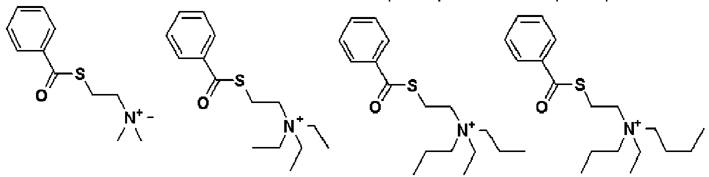

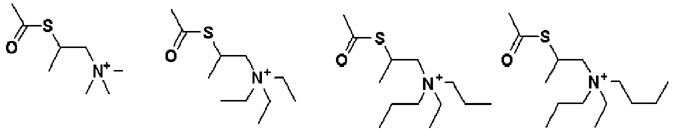

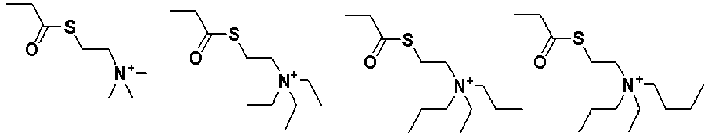

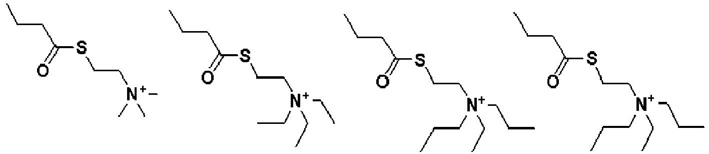

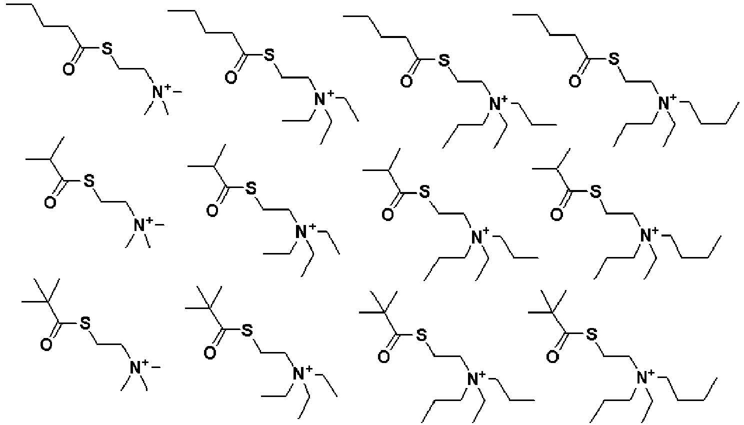

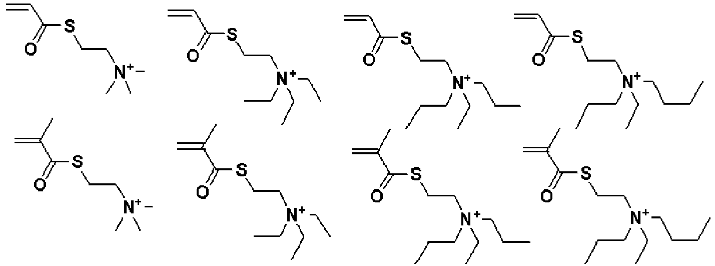

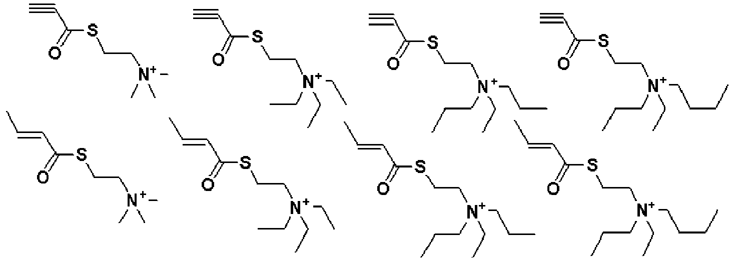

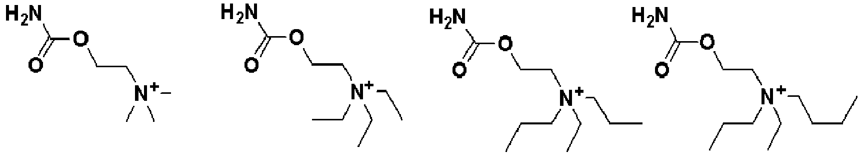

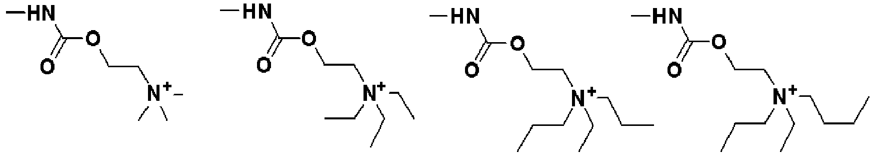

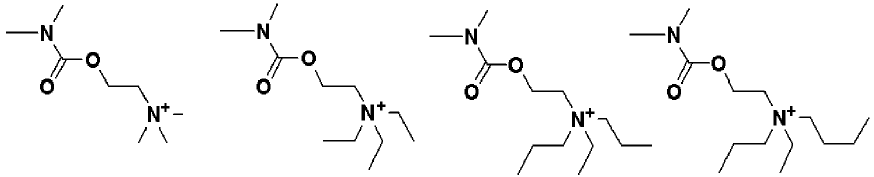

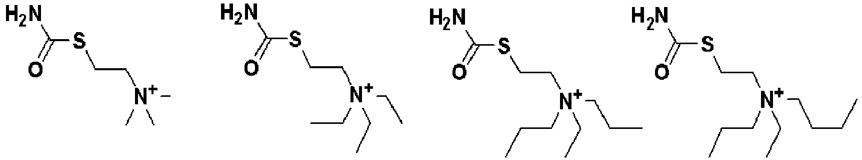

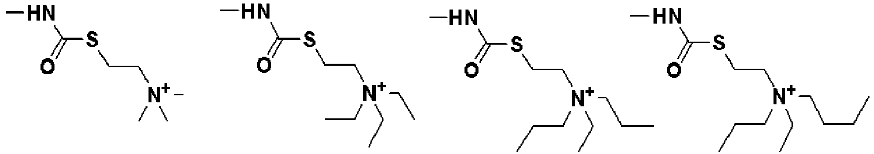

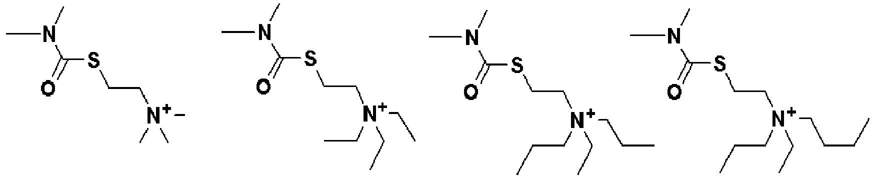

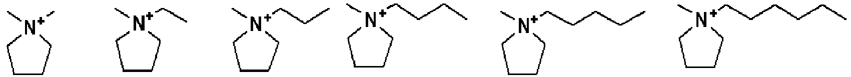

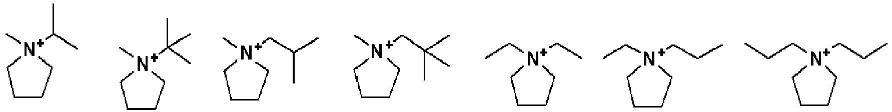

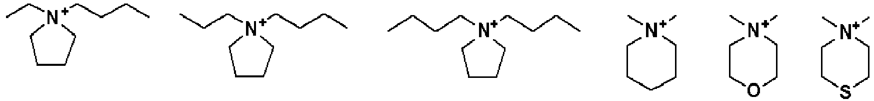

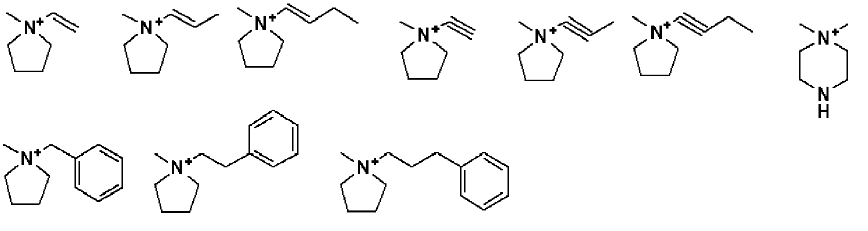

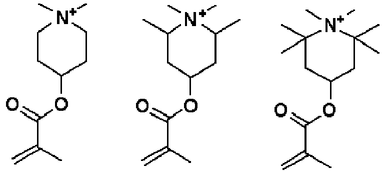

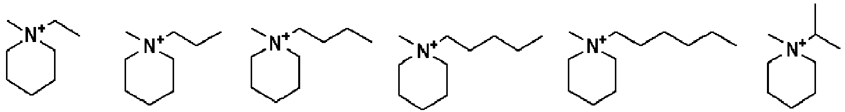

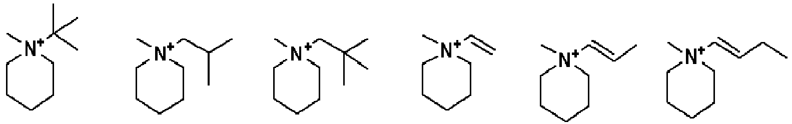

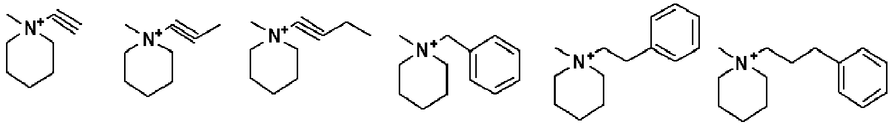

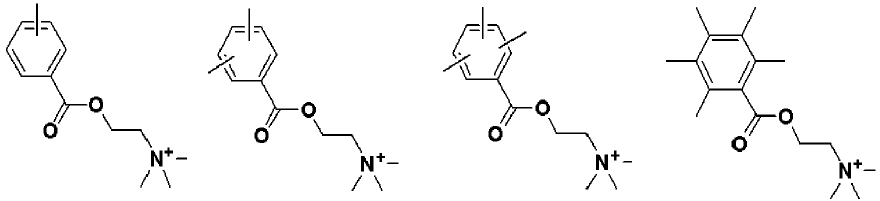

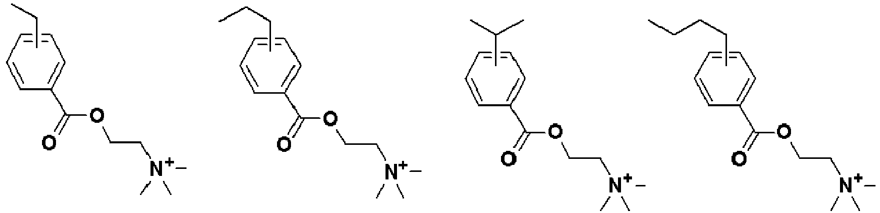

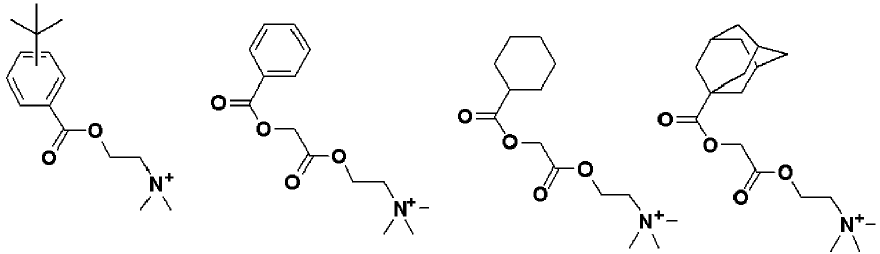

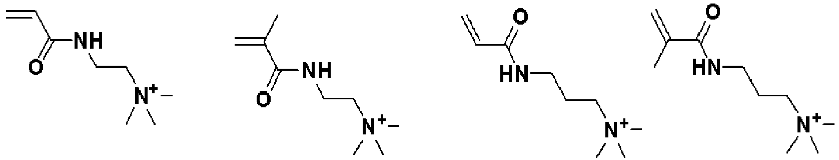

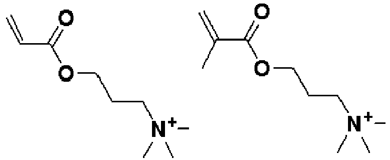

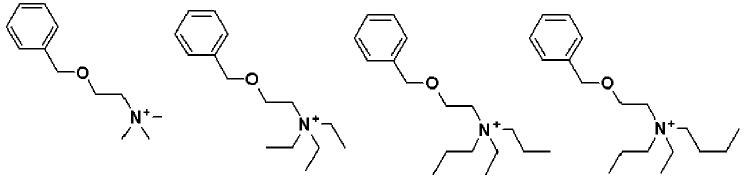

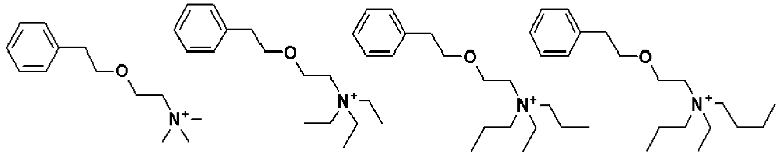

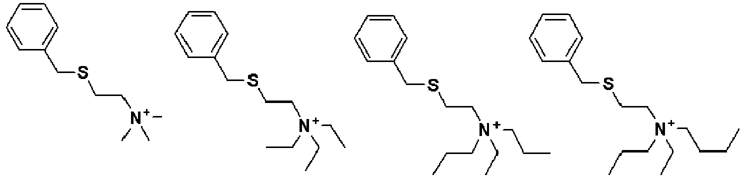

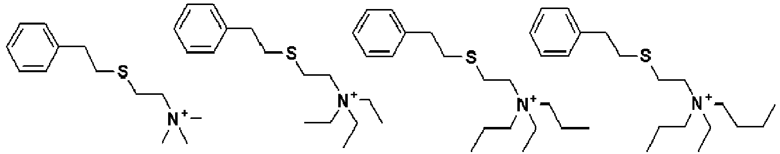

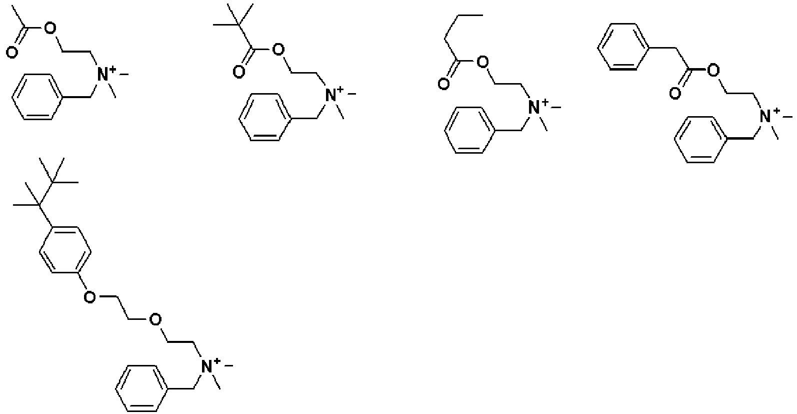

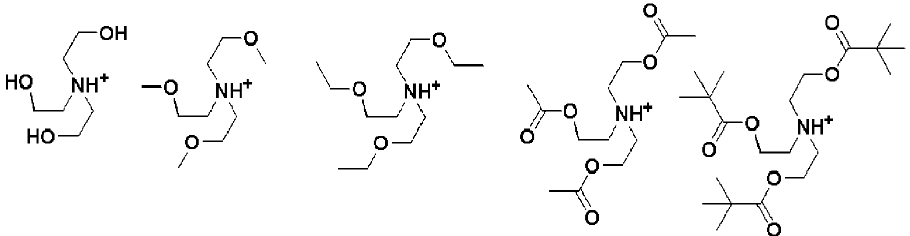

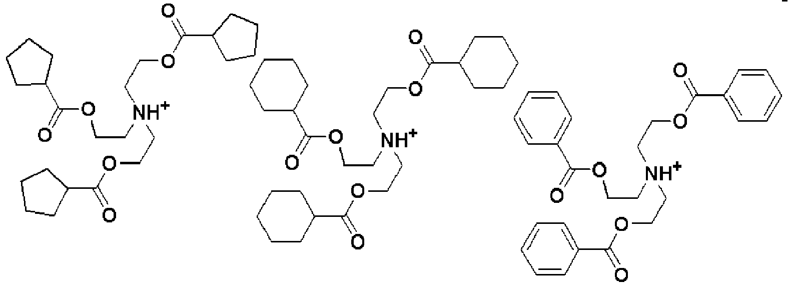

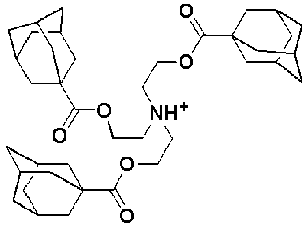

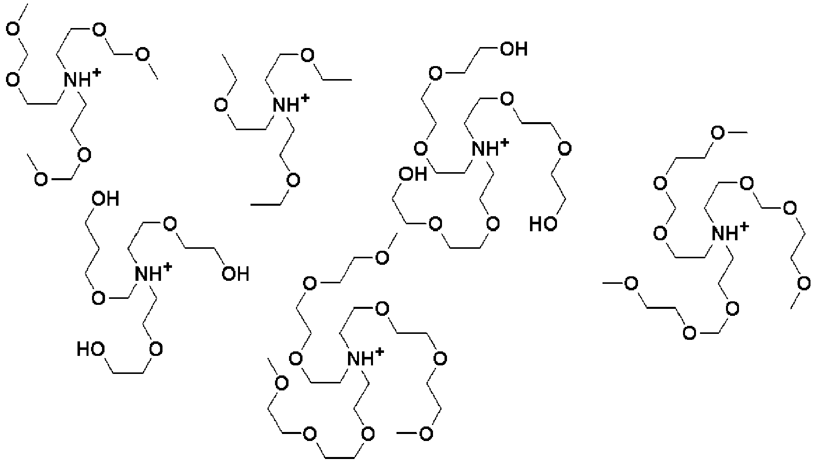

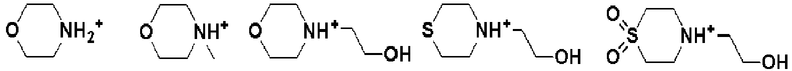

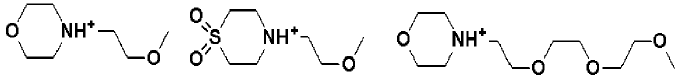

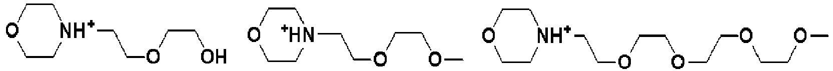

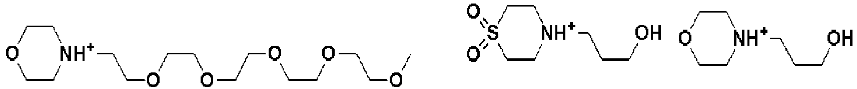

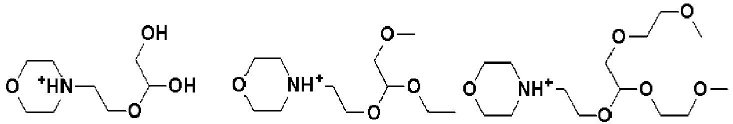

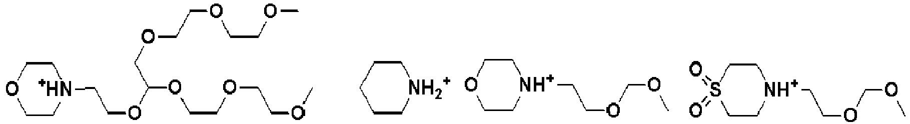

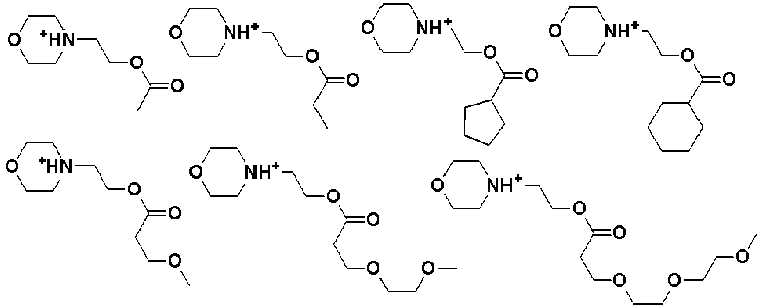

It is especially preferable that the ammonium ion represented by the above general formula (2) is a tertiary ammonium or a quaternary ammonium.

The repeating unit represented by the above general formula (1) can be obtained by a reaction between a monomer represented by the above general formula (1)-1 having a dihydroxy group and a compound having an isocyanate group. Examples of the compound having an isocyanate group can be exemplified below.

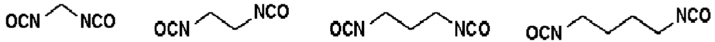

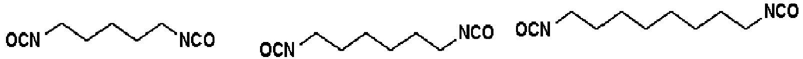

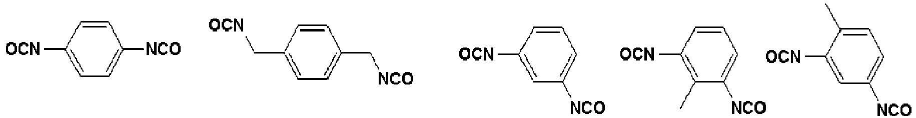

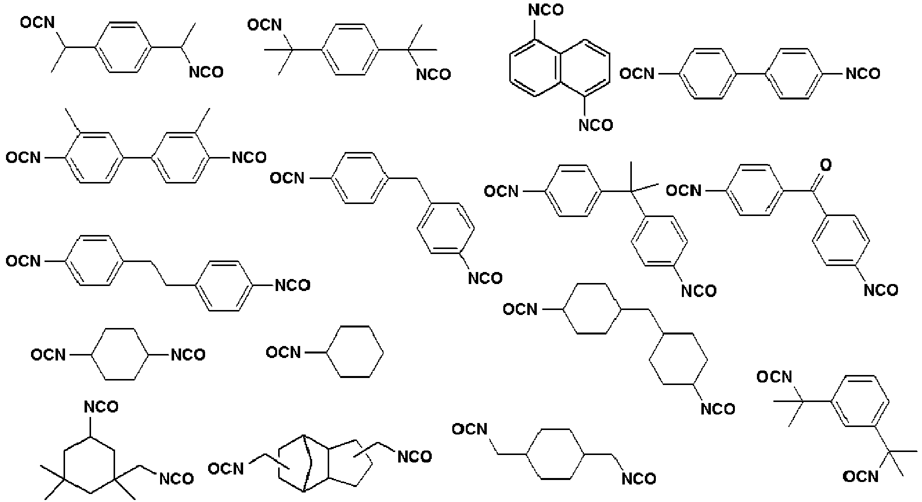

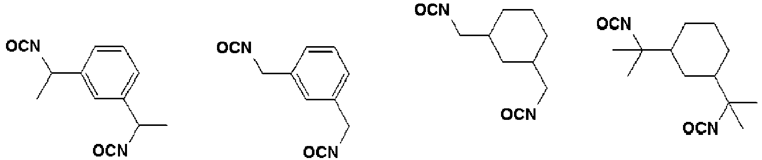

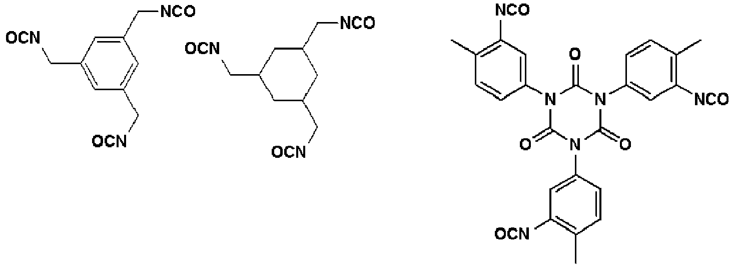

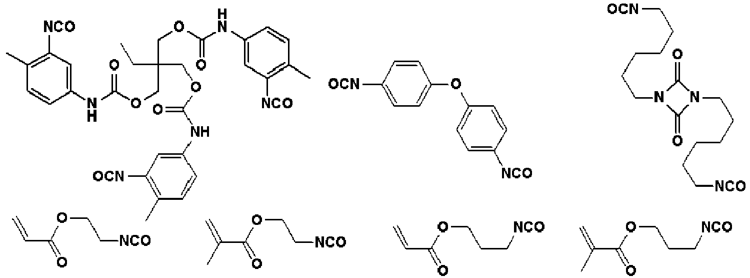

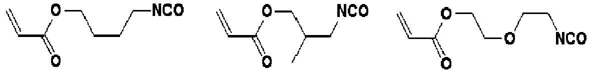

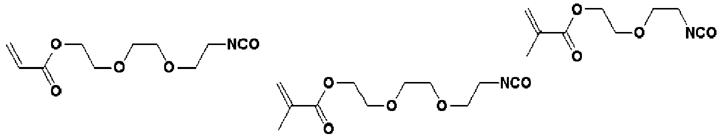

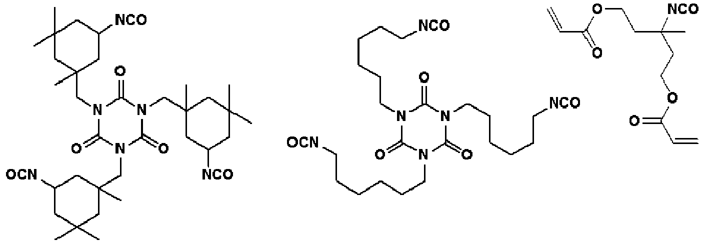

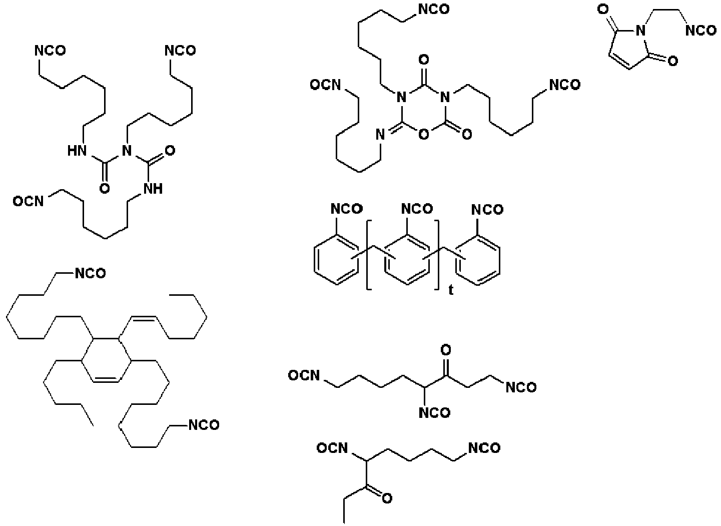

In the formula, "t" is an integer of 1 or more.

Although the repeating unit represented by the above general formula (1) can be obtained by a reaction between a monomer represented by the above general formula (1)-1 having a dihydroxy group and a compound having an isocyanate group, it is also possible to react with compounds having a dihydroxy group other than the monomer represented by the above general formula (1)-1.

Examples of the compounds having a dihydroxy group other than the monomer represented by the above general formula (1)-1 can include compounds having polyol at the terminal as exemplified below.

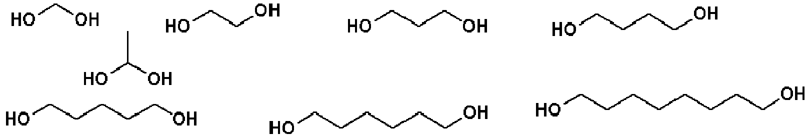

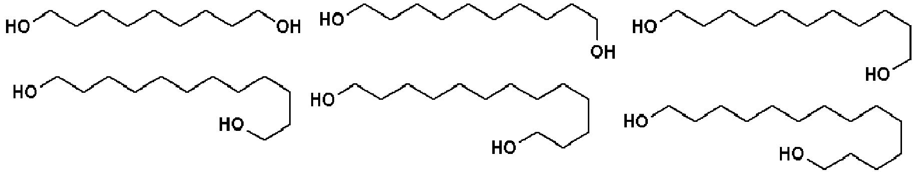

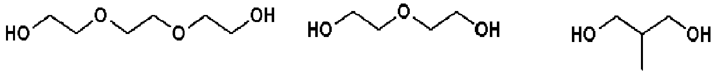

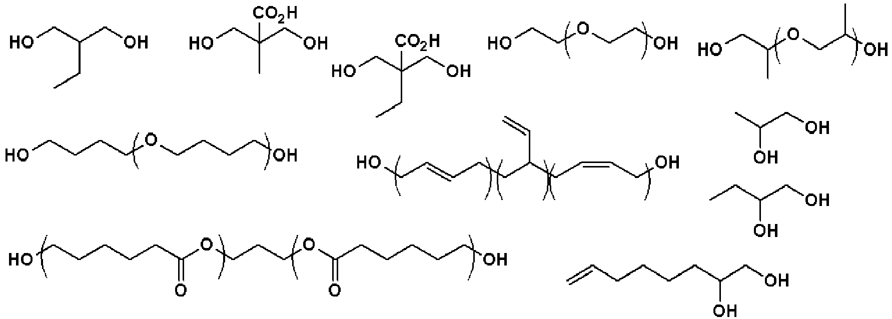

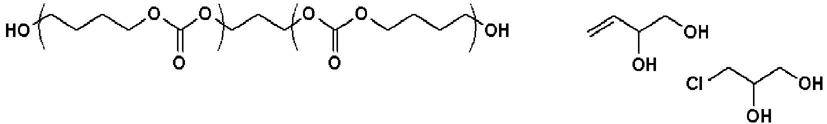

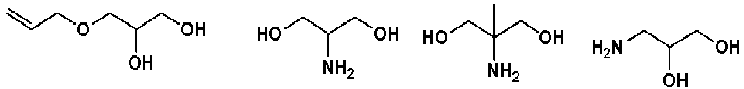

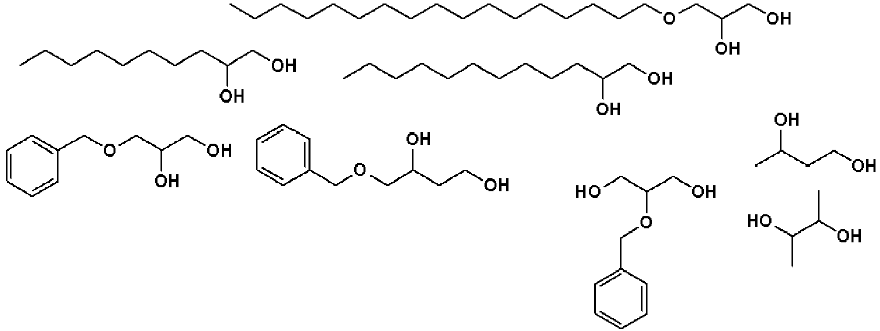

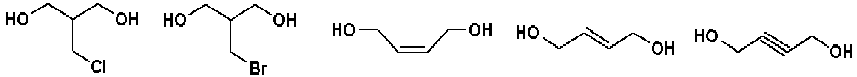

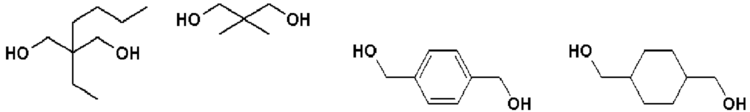

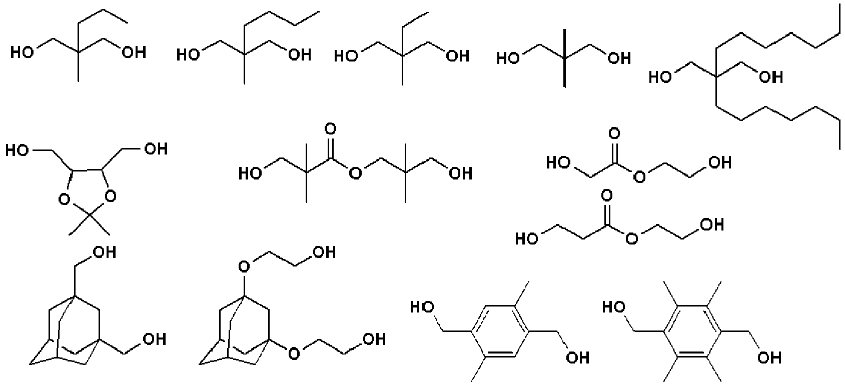

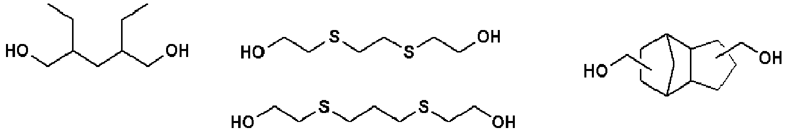

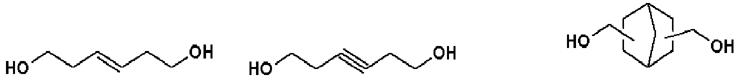

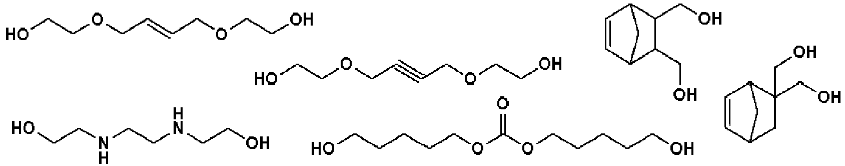

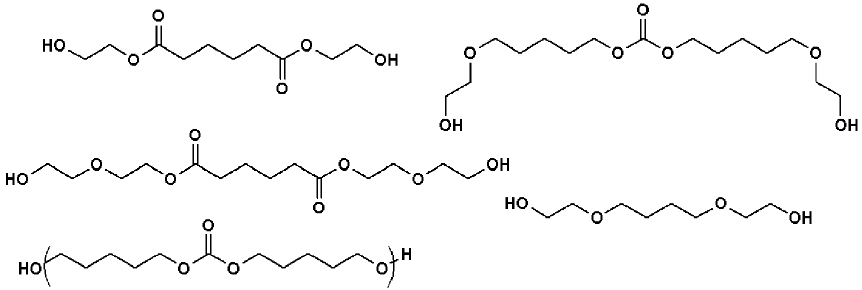

Here, the parentheses are a repeating unit.

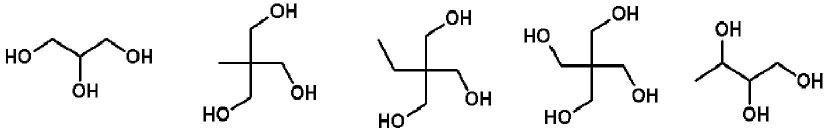

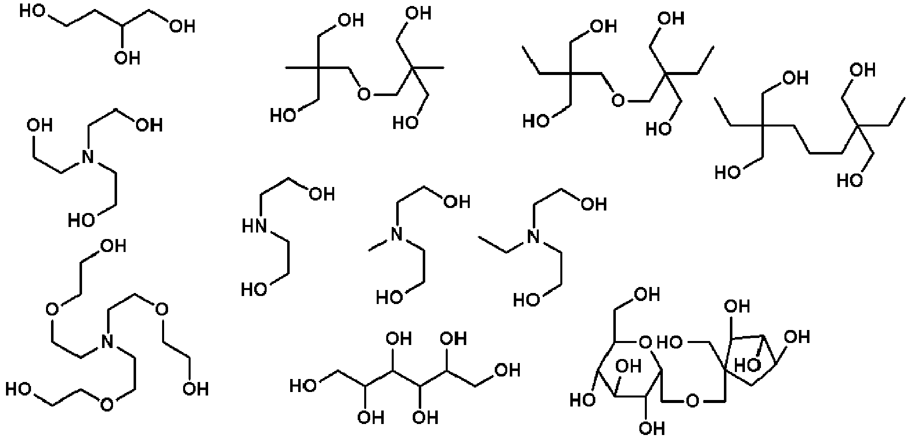

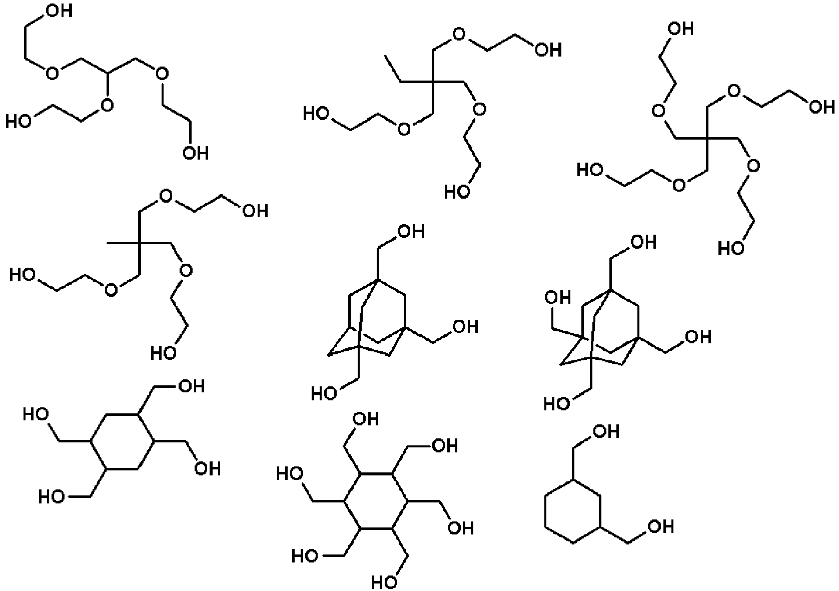

Diol compounds of which raw material is a polyester compound having diol at a copolymerizable terminal can be exemplified below.

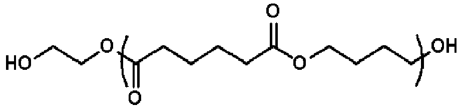

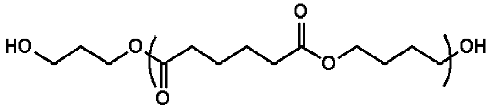

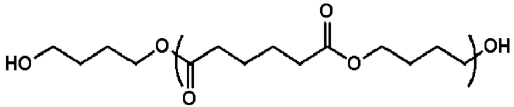

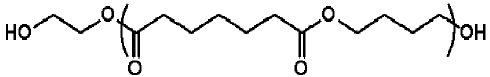

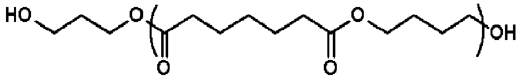

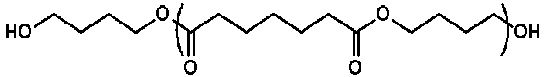

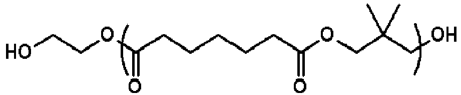

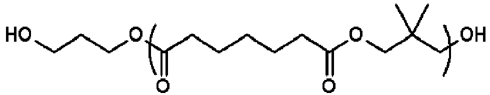

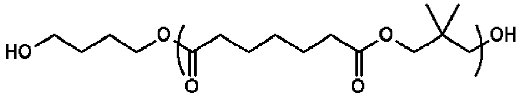

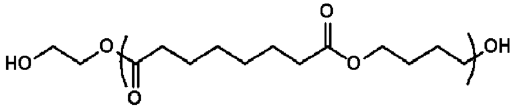

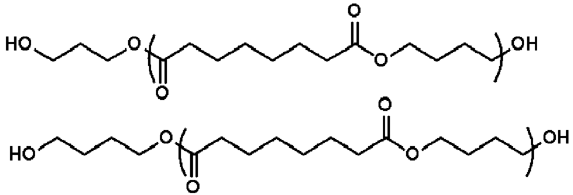

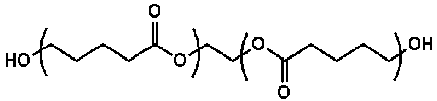

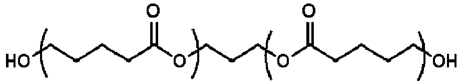

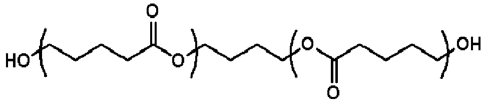

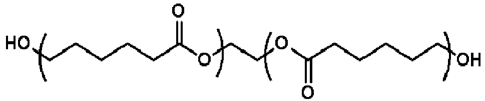

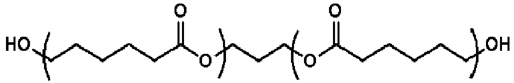

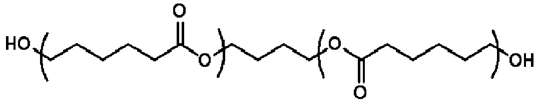

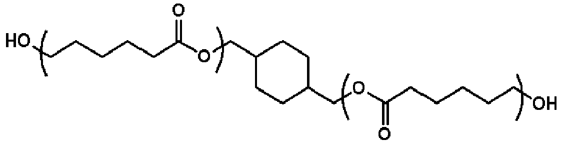

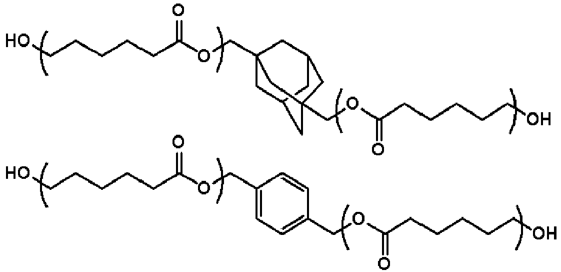

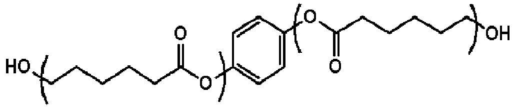

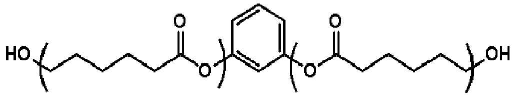

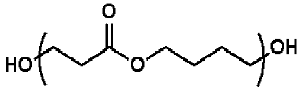

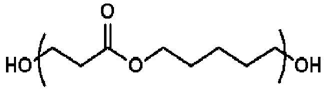

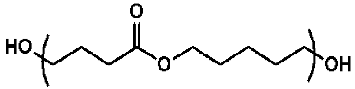

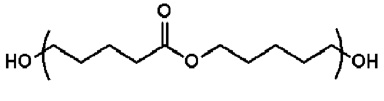

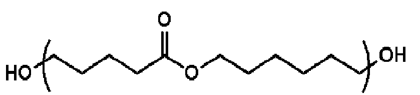

Here, the parentheses are a repeating unit.

It is also possible to copolymerize with a polycarbonate compound having diol at the terminal shown below.

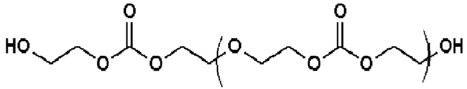

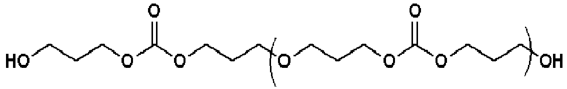

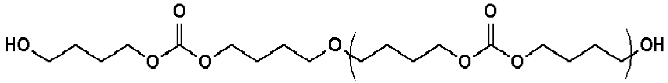

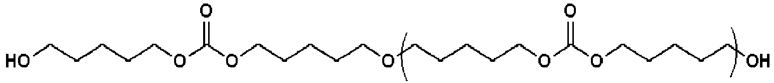

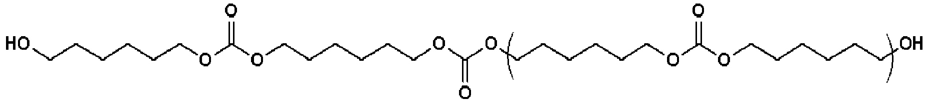

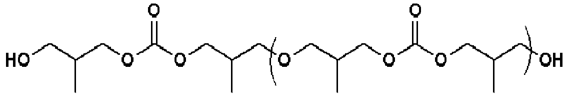

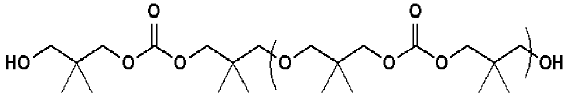

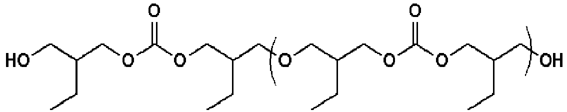

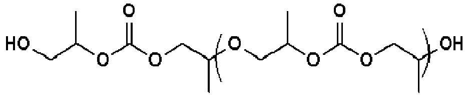

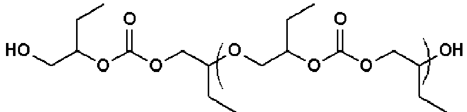

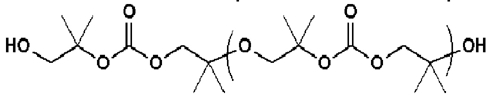

Here, the parentheses are a repeating unit.

Further, it is also possible to copolymerize with a diol compound having a fluoroalkyl group or a fluoroalkylene group shown below.

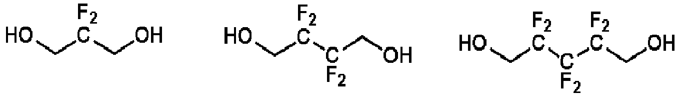

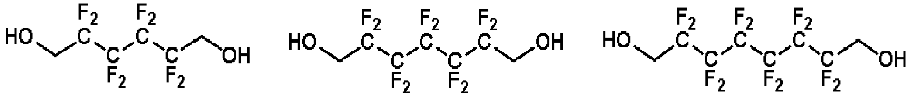

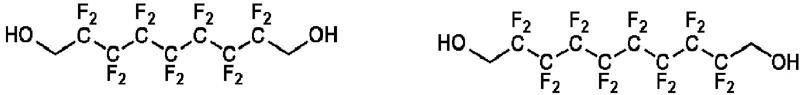

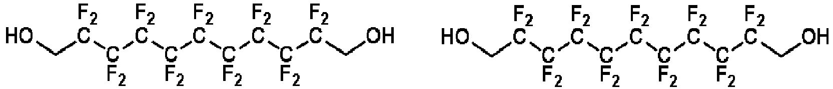

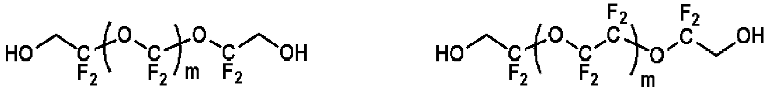

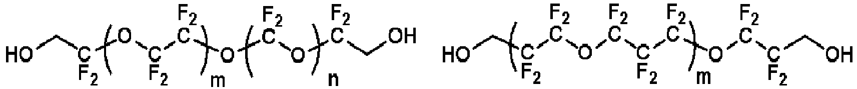

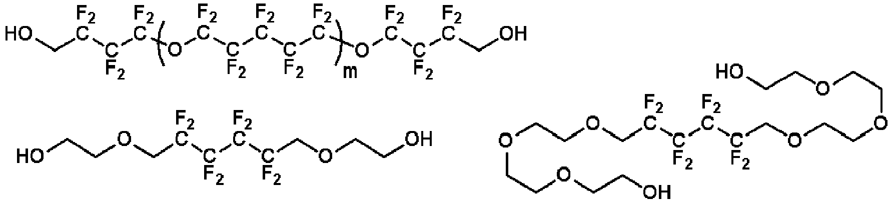

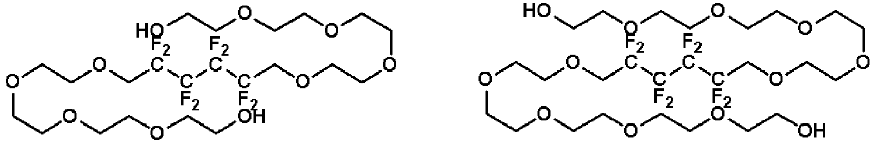

Here, "m" and "n" are in the range of 0 to 20.

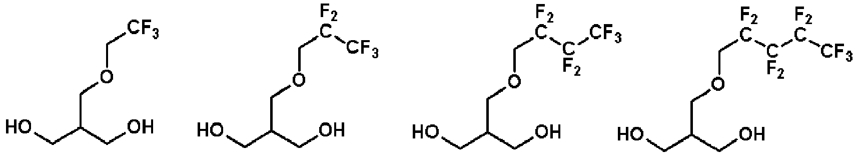

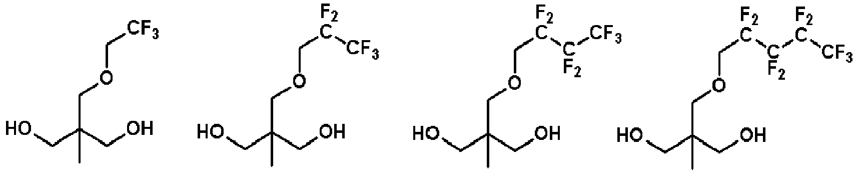

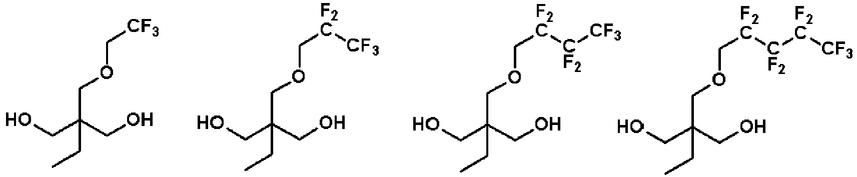

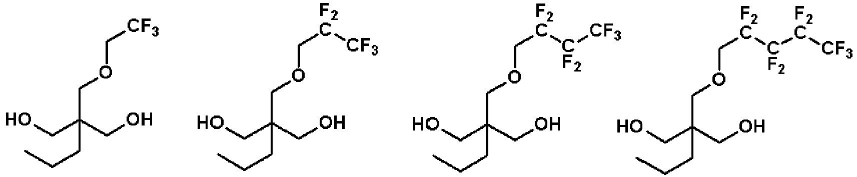

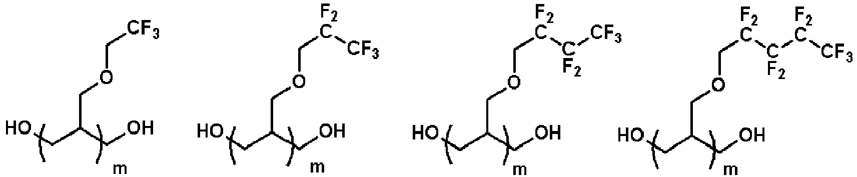

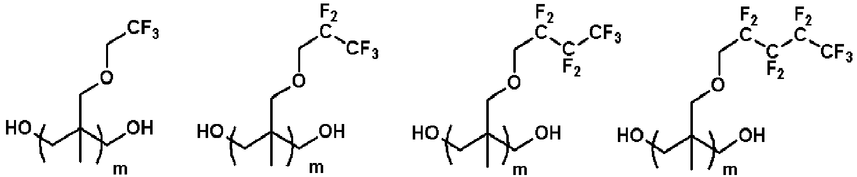

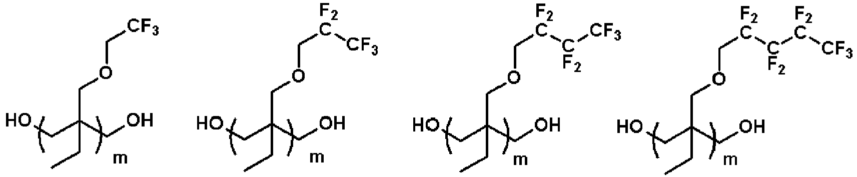

Here, "m" is in the range of 1 to 20.

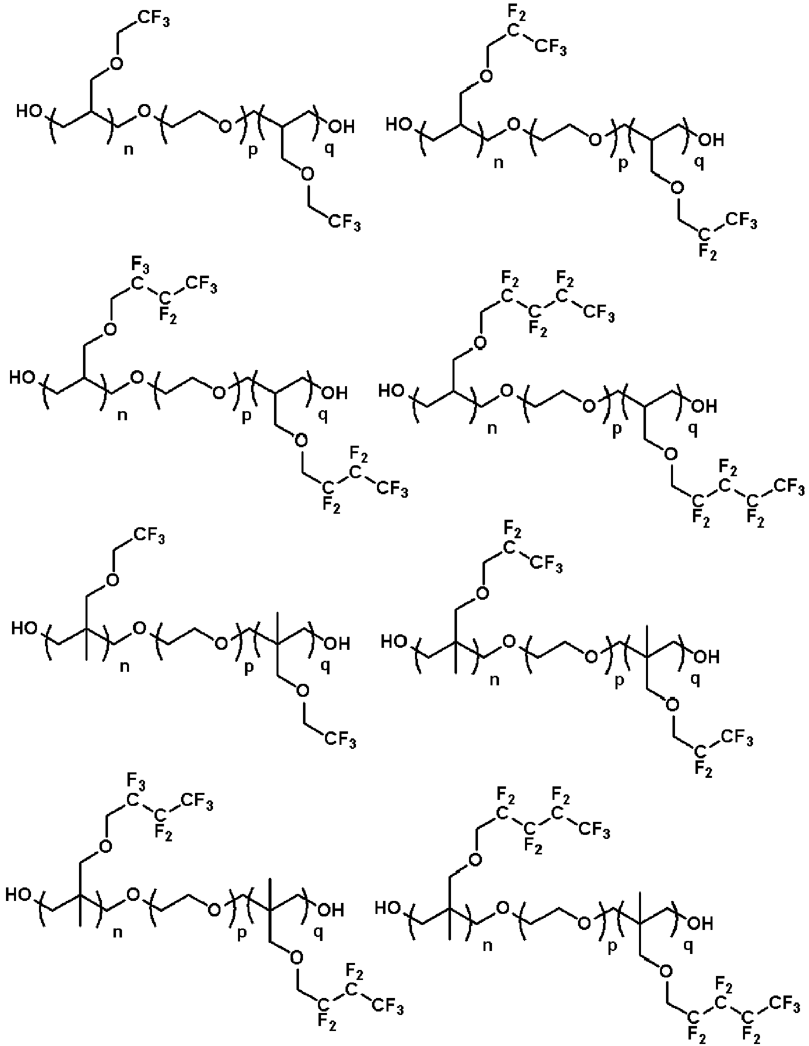

Here, "n" and "q" are in the range of 1 to 20, 2≤n+q≤21, and 1≤p≤40.

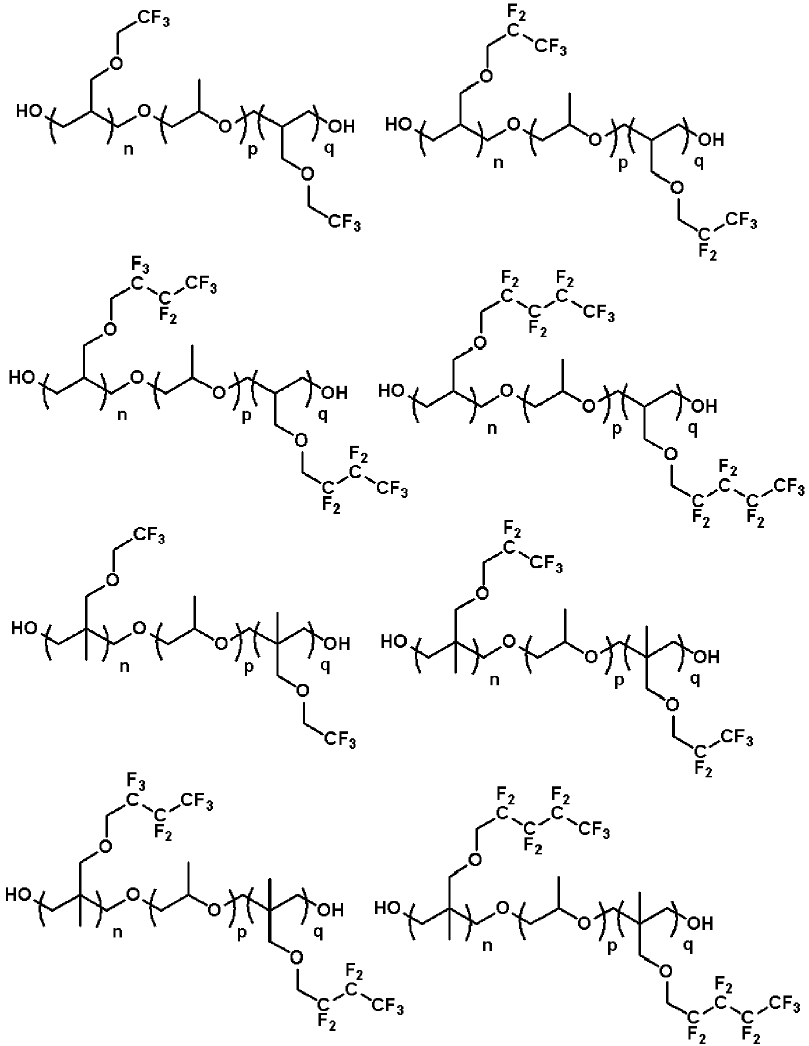

Here, "n" and "q" are in the range of 1 to 20, 2≤n+q≤21, and 1≤p≤40.

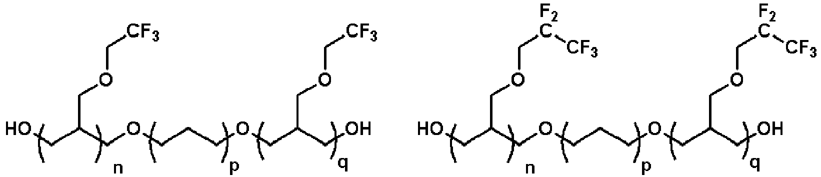

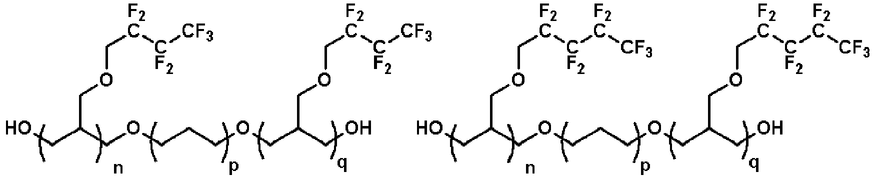

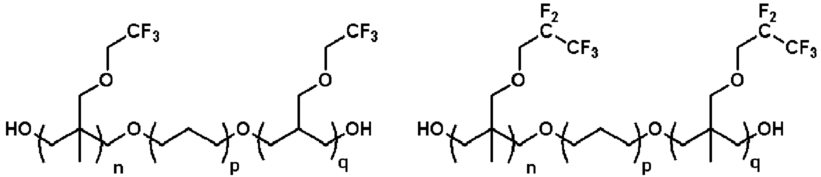

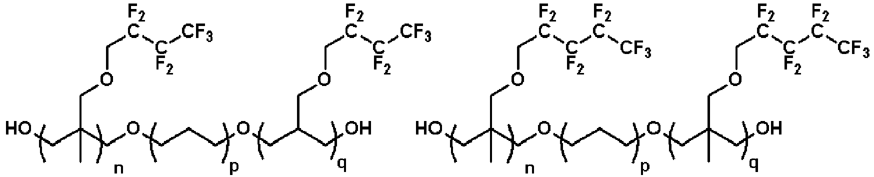

Here, "n" and "q" are in the range of 1 to 20, 2≤n+q≤21, and 1≤p≤40.

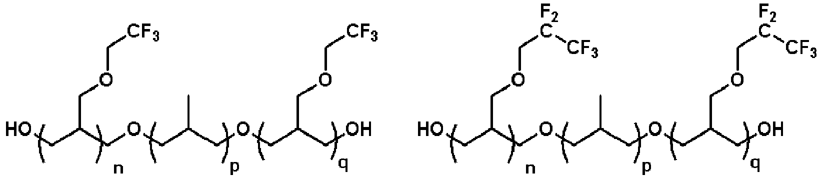

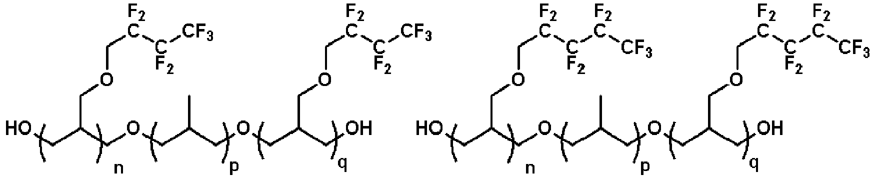

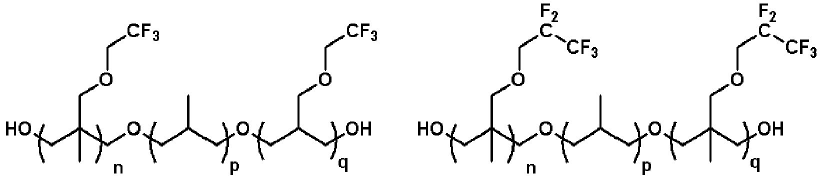

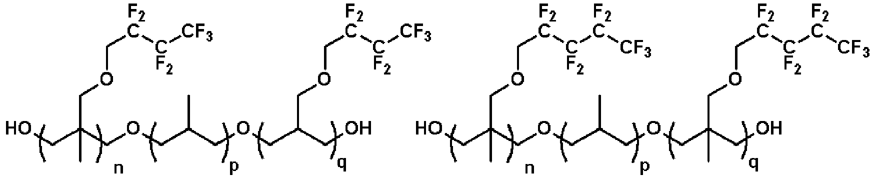

Here, "n" and "q" are in the range of 1 to 20, 2≤n+q≤21, and 1≤p≤40.

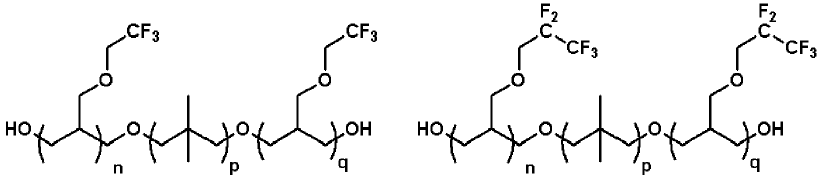

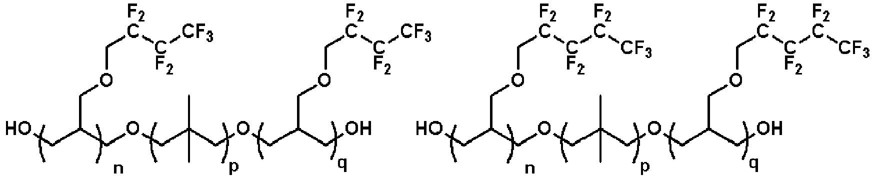

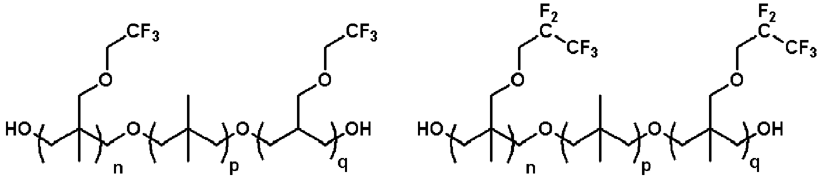

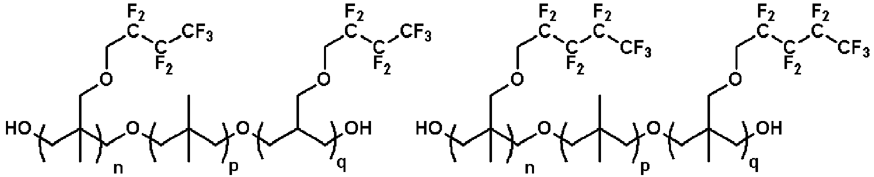

Here, "n" and "q" are in the range of 1 to 20, 2≤n+q≤21, and 1≤p≤40.

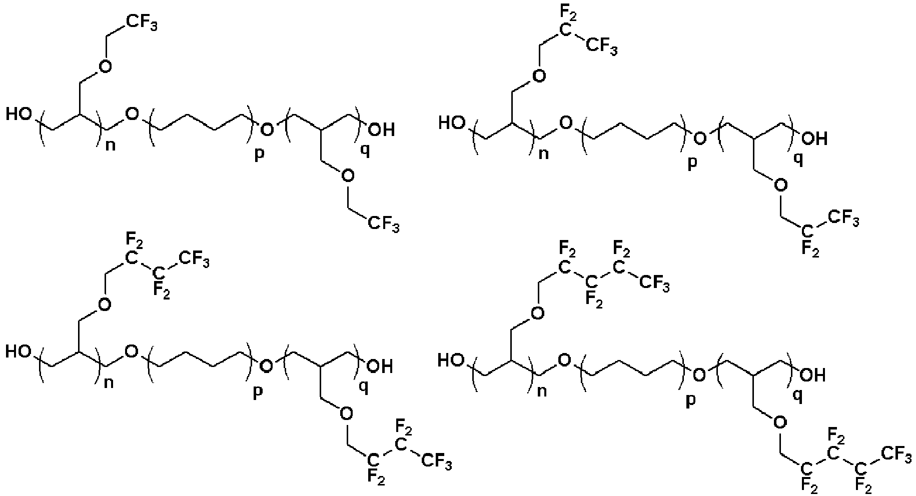

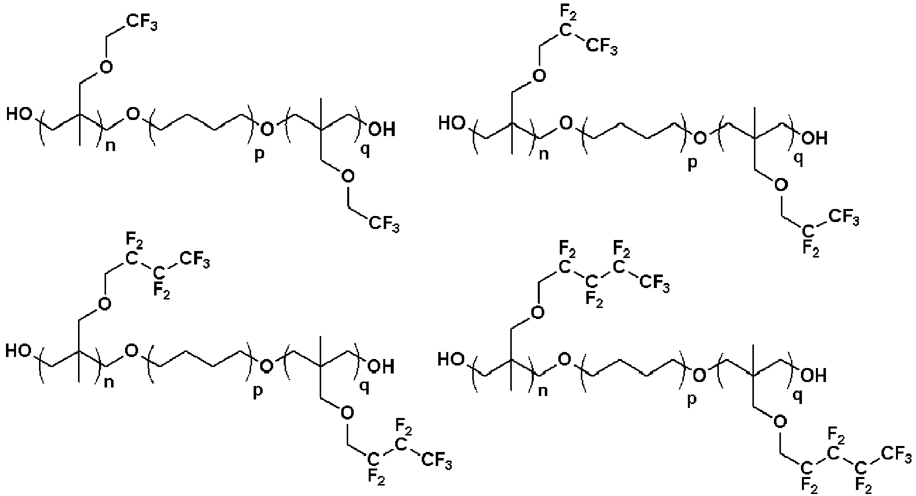

Here, "n" and "q" are in the range of 1 to 20, 2≤n+q≤21, and 1≤p≤40.

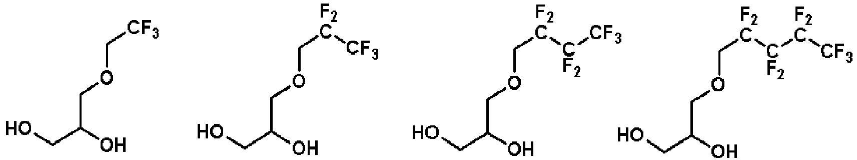

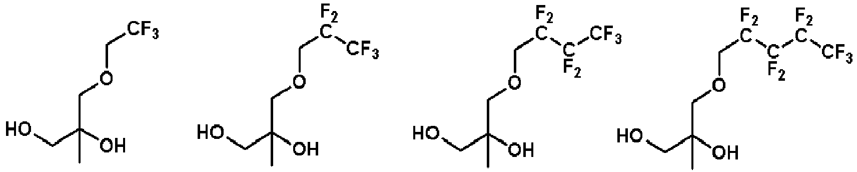

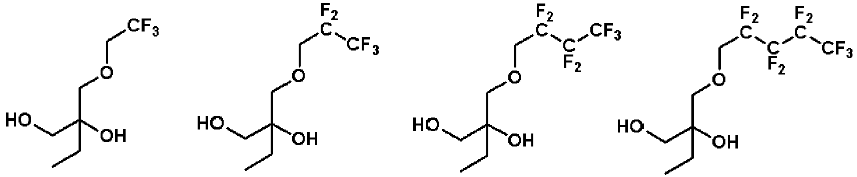

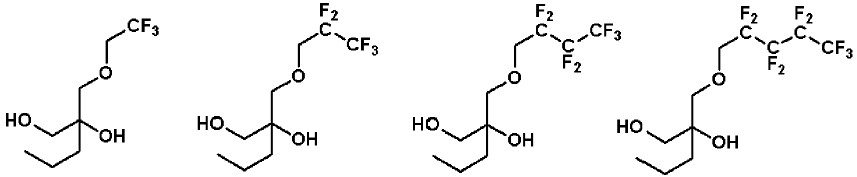

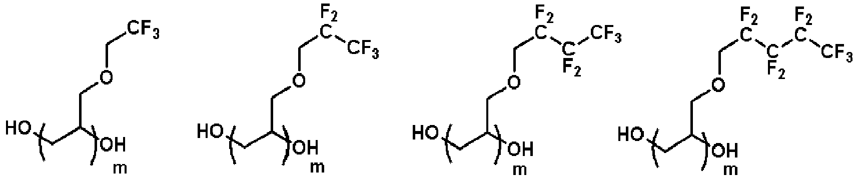

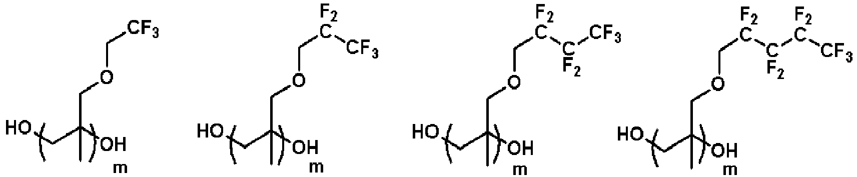

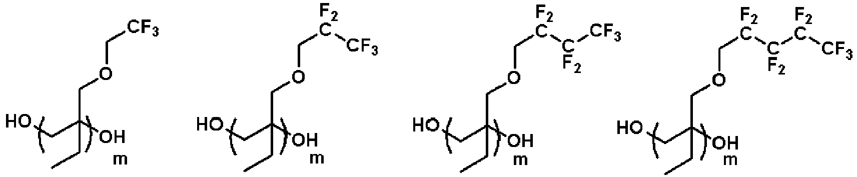

Here, "m" is in the range of 1 to 20.

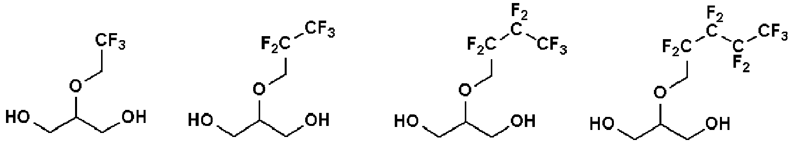

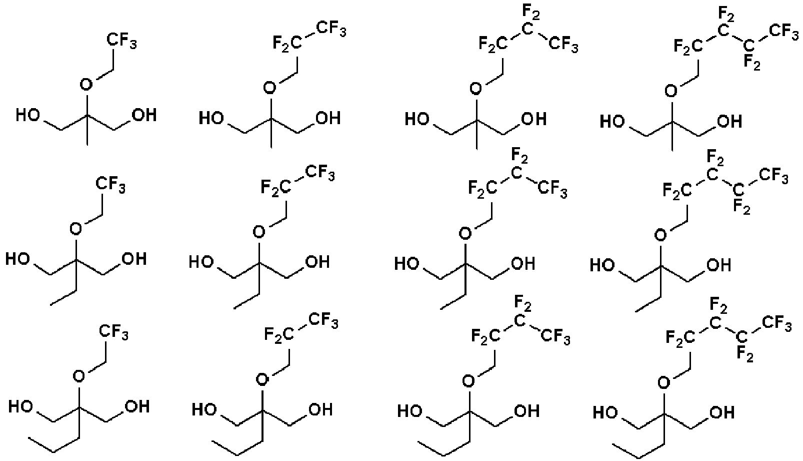

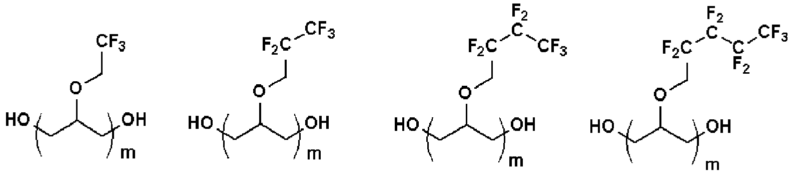

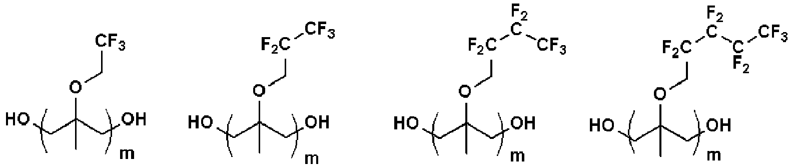

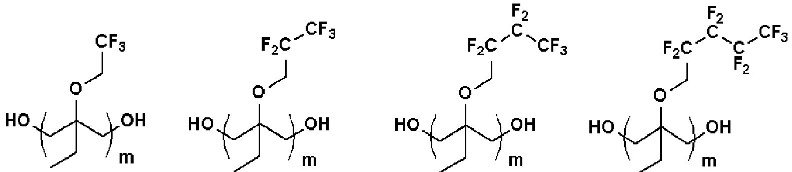

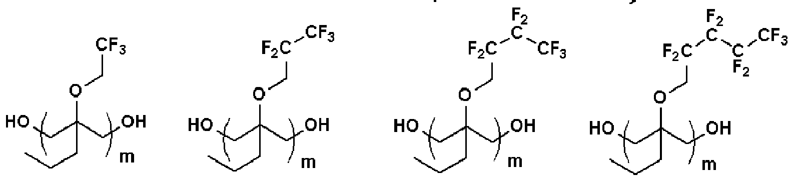

Here, "m" is in the range of 1 to 20.

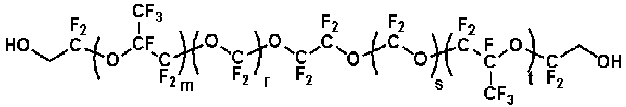

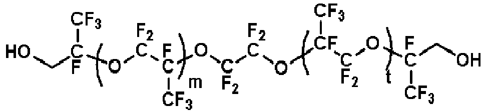

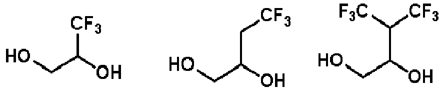

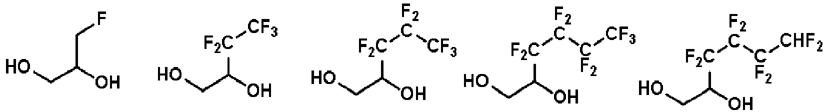

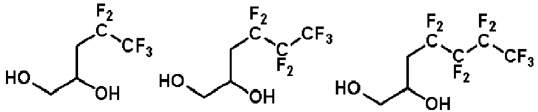

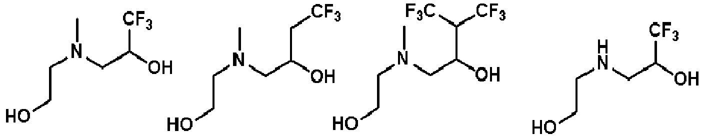

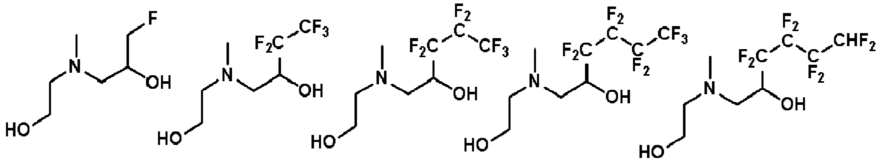

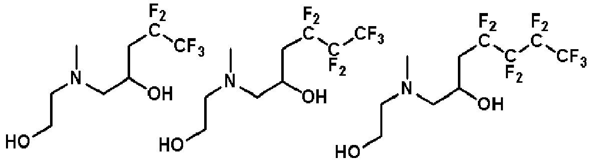

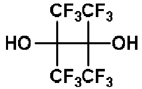

Here, "m", "r", "s", and "t" are in the range of 0 to 20.

Further, it is also possible to copolymerize with a diol compound having a fluoroalcohol group or a sulfonamide group disclosed in JP 2022-078861 A.

It is also possible to copolymerize with a diol compound having the following silicon in a pendant form.

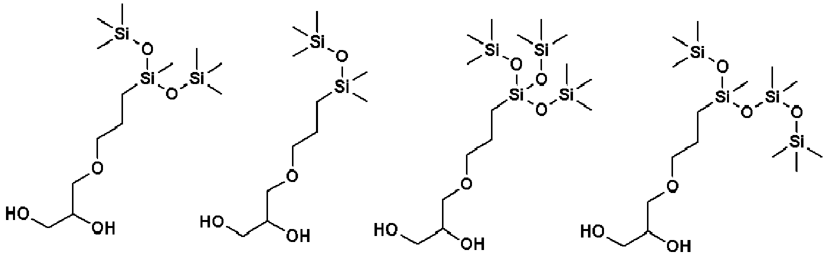

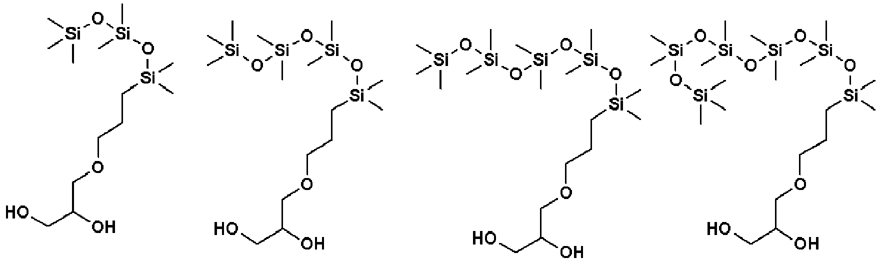

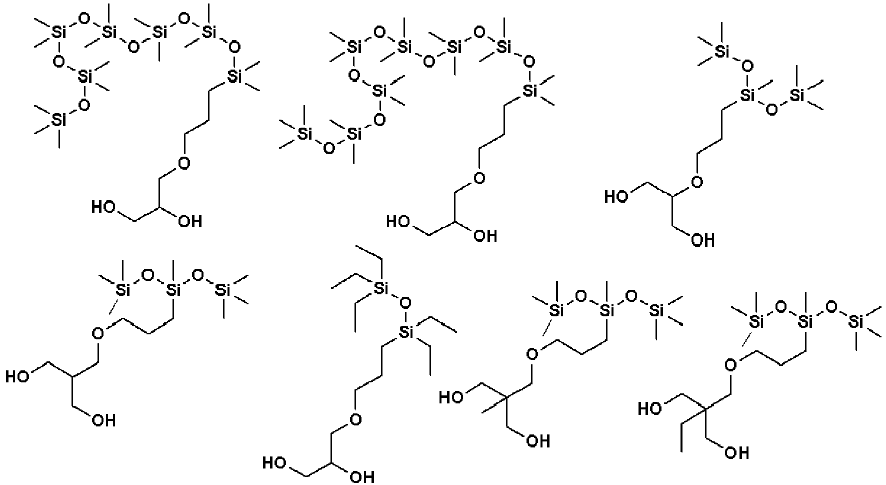

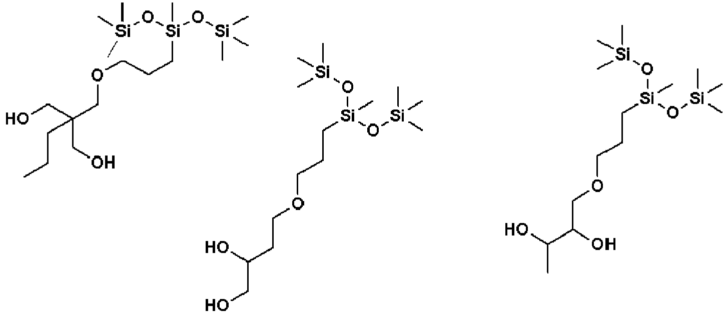

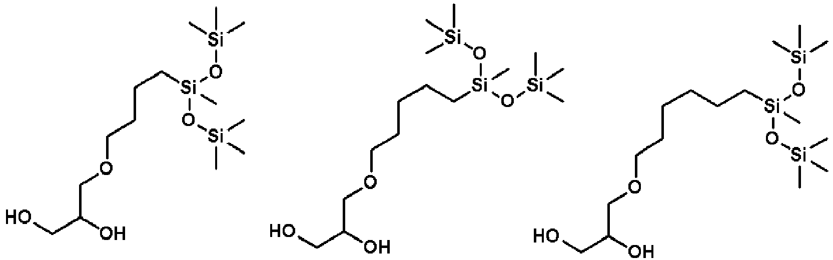

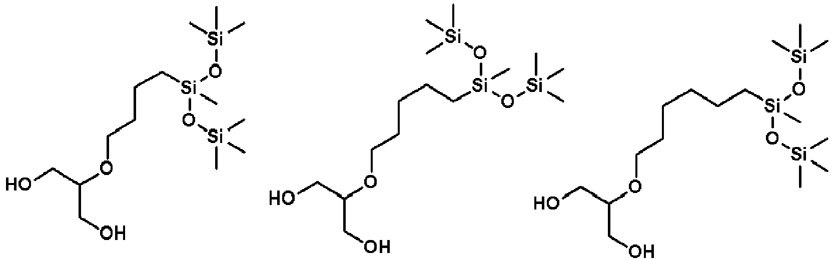

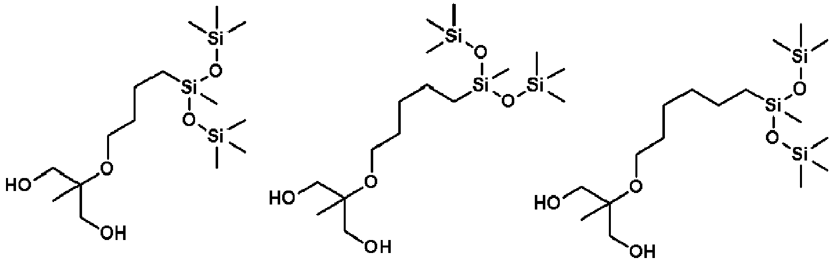

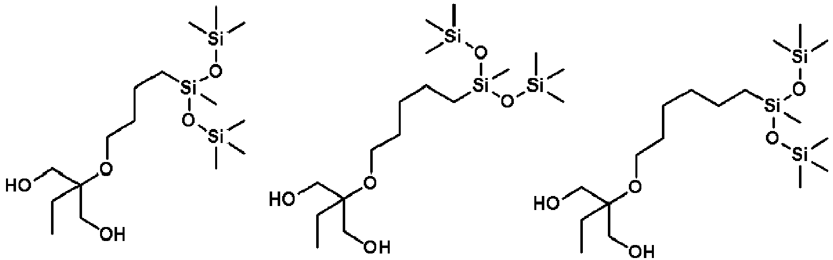

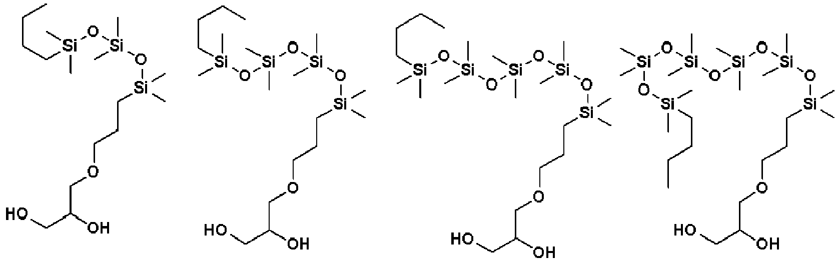

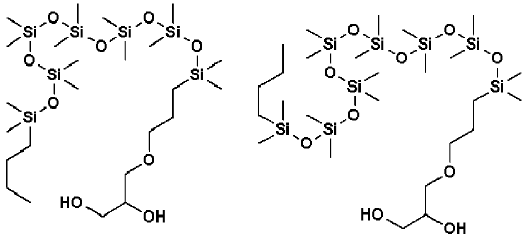

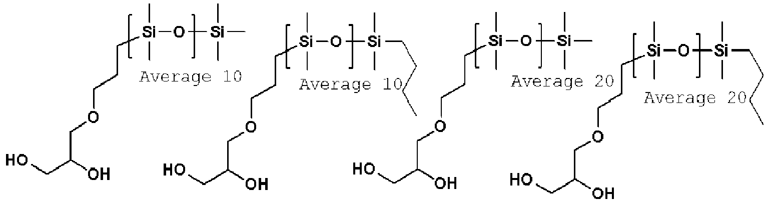

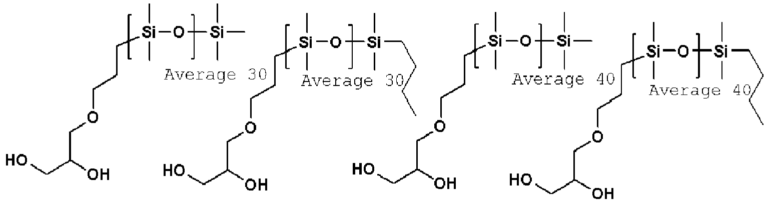

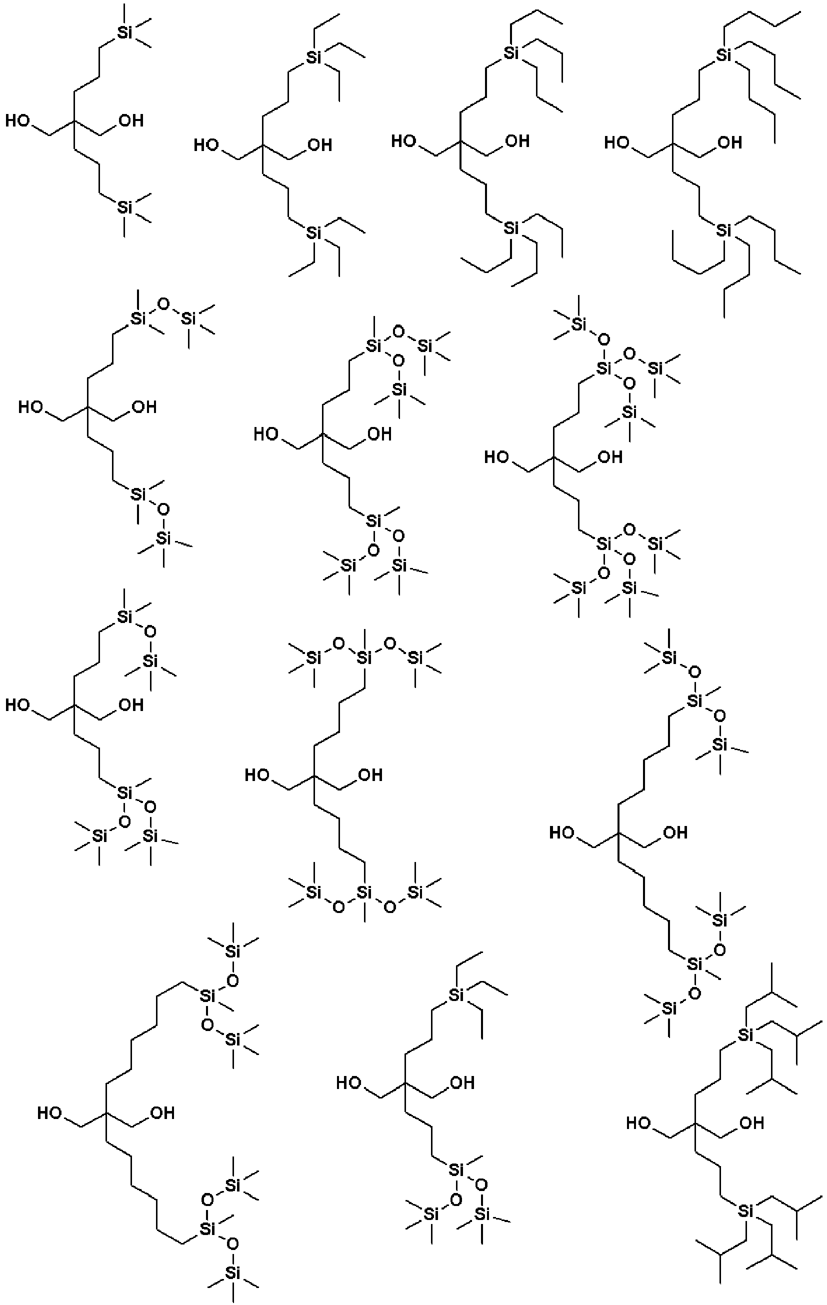

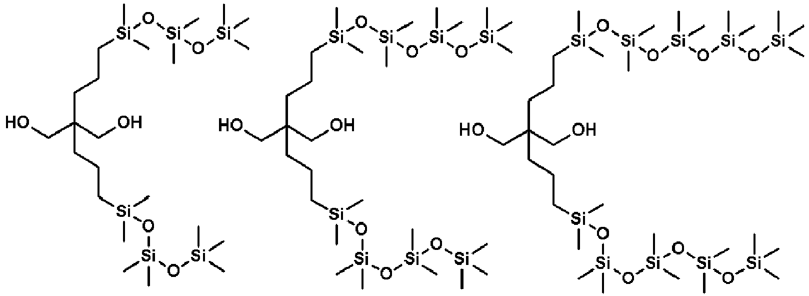

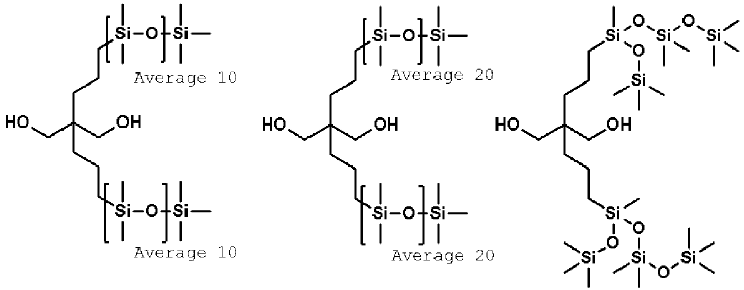

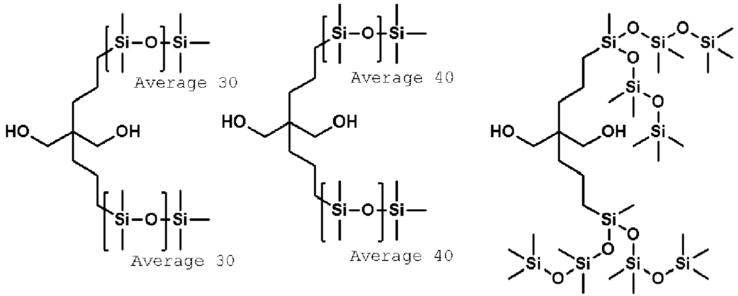

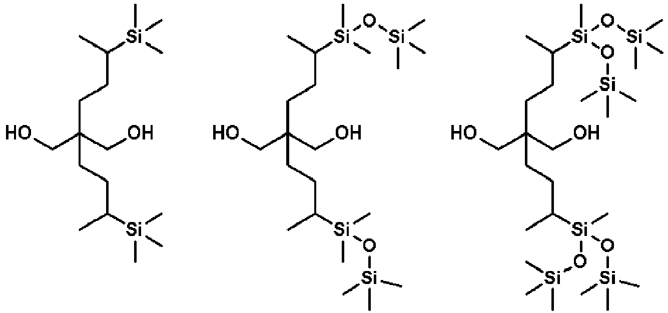

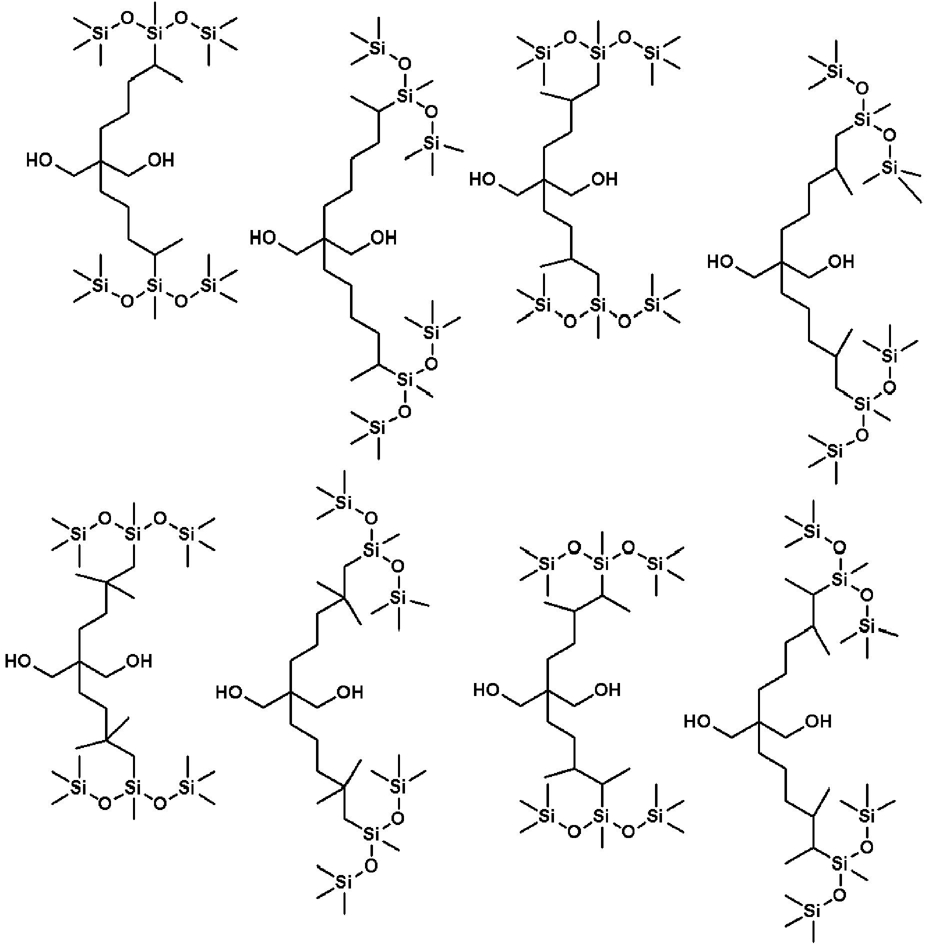

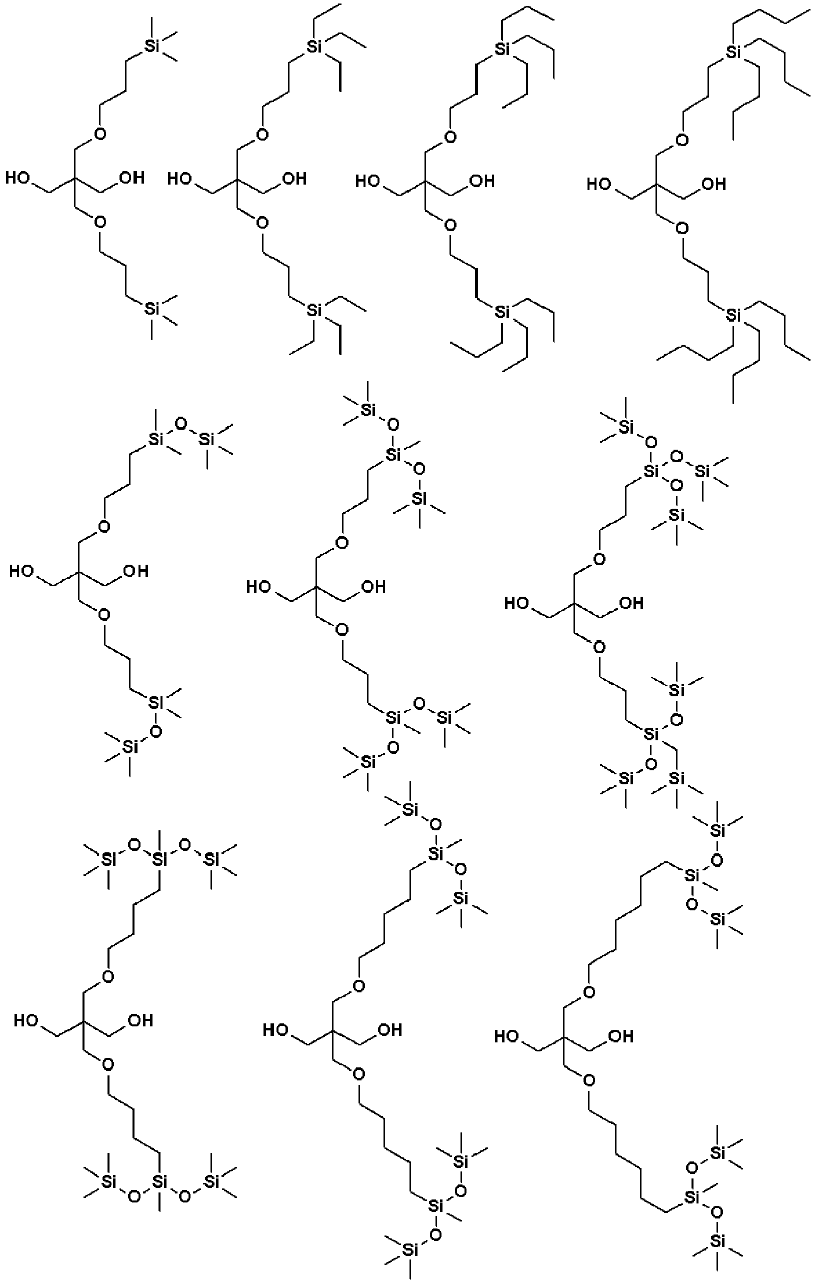

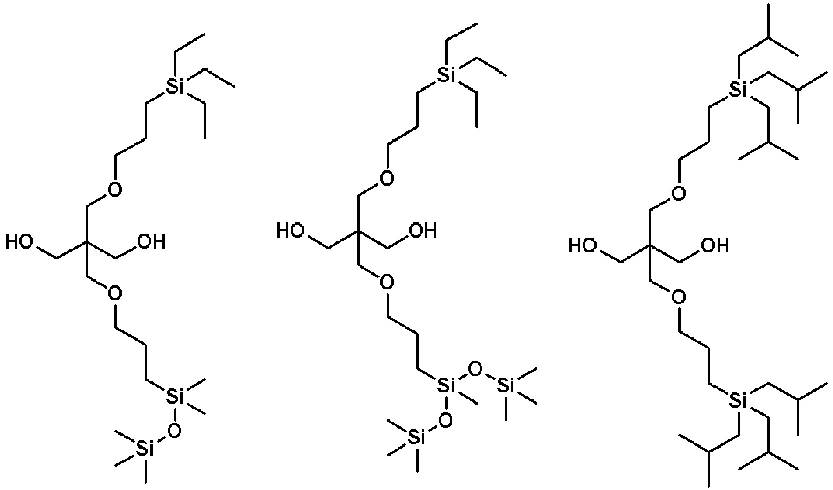

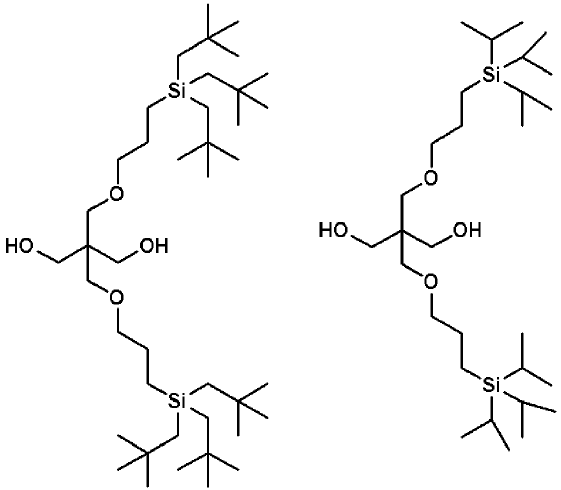

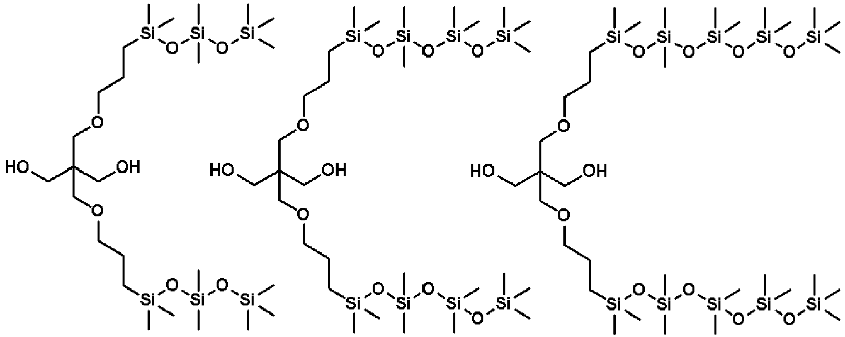

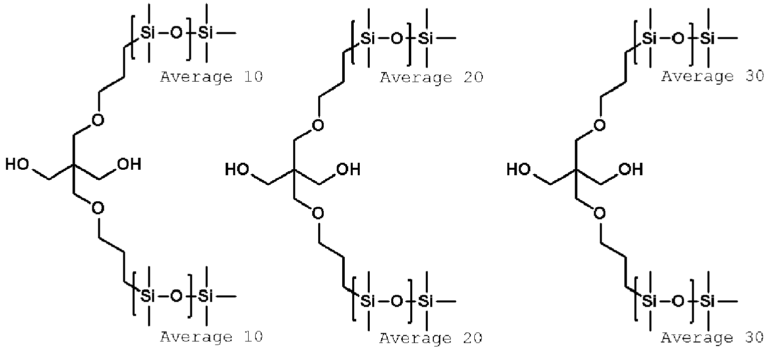

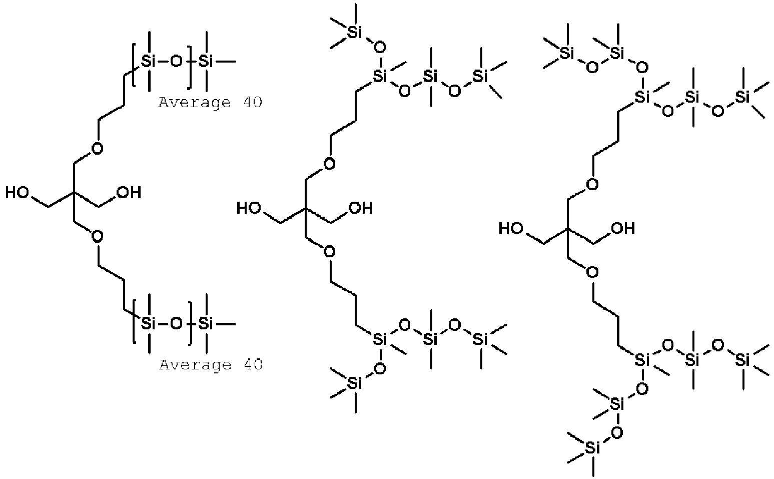

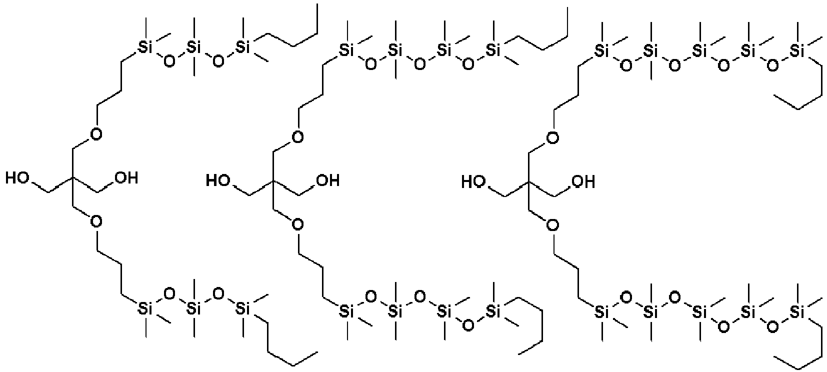

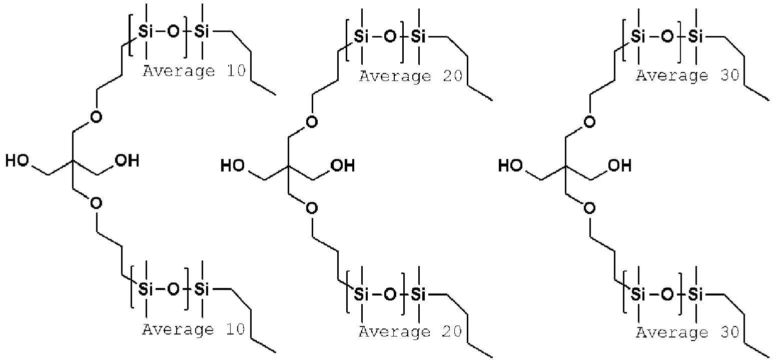

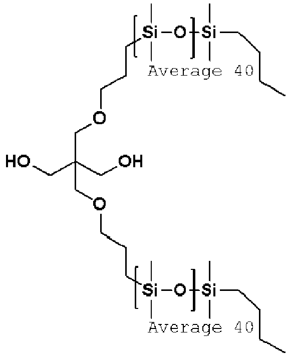

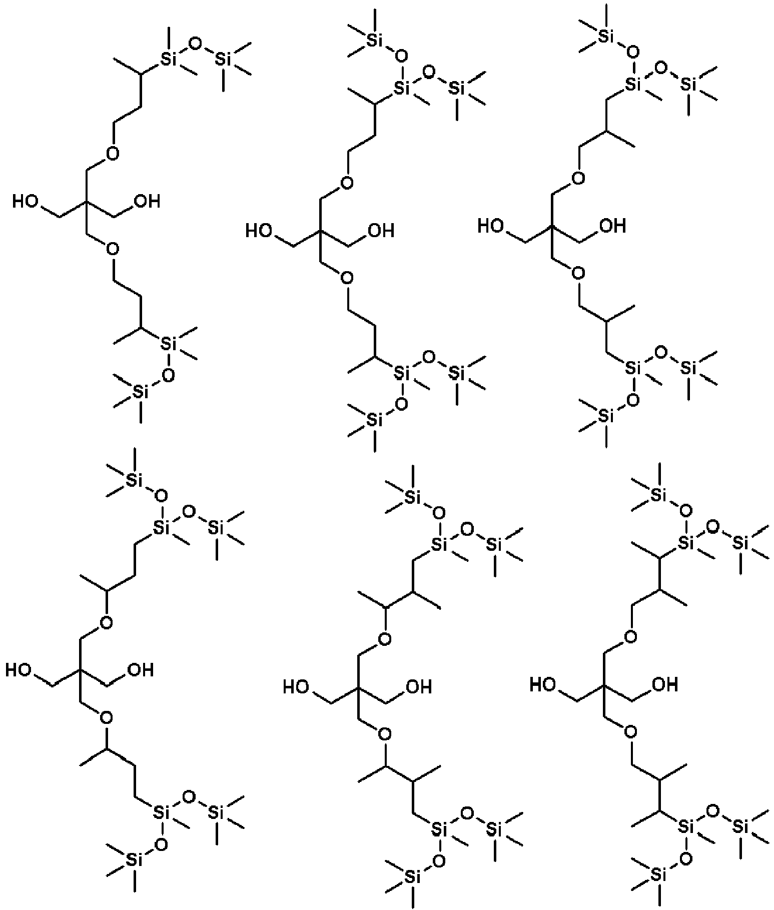

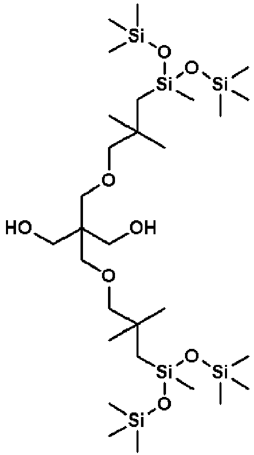

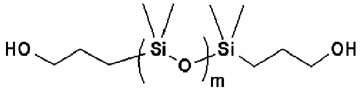

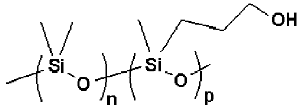

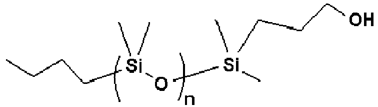

Here, "m", "n", and "p" are in the range of 1 to 20.

Among the above isocyanate compounds, especially when an isocyanate compound having a (meth)acrylate group is reacted with a compound represented by the general formula (1)-1 or another hydroxy compound, the compound having a (meth)acrylate group at the terminal can be obtained. The compound having a (meth)acrylate group at the terminal can be obtained also by reacting a compound having both a hydroxy group and a (meth)acrylate group with an isocyanate compound.

Among the above isocyanate compounds, especially when an isocyanate compound having a maleimide group is reacted with a compound represented by the general formula (1)-1 or another hydroxy compound, the compound having a maleimide group at the terminal can be obtained. The compound having a maleimide group at the terminal can be obtained also by reacting a compound having both a hydroxy group and a maleimide group with an isocyanate compound.

Because the above isocyanate compounds have high reactivity to a hydroxy group-containing compound, it is impossible to control the reaction in some cases. Additionally, an isocyanate compound acts with moisture in the air during storage and its isocyanate group is deactivated in some cases, therefore, sufficient care must be taken during storage, such as sufficiently preventing humidity. For that, to prevent such a phenomenon, a compound having a blocked isocyanate group where the isocyanate group is protected by a substituent, is used in some cases.

The blocked isocyanate group is deprotected by heating to become an isocyanate group, and the specific examples include an isocyanate group substituted with alcohols, phenols, thioalcohols, imines, ketimines, amines, lactams, pyrazoles, oximes, β-diketones, etc.

In order to lower the deprotection temperature of the blocked isocyanate group, it is possible to add a catalyst. The catalysts for this are known to include: organic tins such as dibutyltin dilaurate, etc.; bismuth salts; and zinc carboxylates such as zinc 2-ethylhexanoate and zinc acetate, etc.

Especially, JP 2012-152725 A discloses that the deprotection temperature can be lowered by containing α,β-unsaturated carboxylic acid as a carboxylic acid and as a blocked isocyanate dissociation catalyst.

In polyurethane polymerization, in order to promote a formation reaction of urethane bonding, a catalyst is preferably added as the occasion demands.

The urethanization catalyst can be selected from publically known catalysts accordingly, and examples thereof include amine catalysts, ammonium salt catalysts, potassium salt catalysts, organometallic catalysts, etc.

Examples of amine catalysts include triethylamine, N,N-dimethylcyclohexylamine, triethylenediamine, 2-methyltriethylenediamine, N,N,N',N'-tetramethylethylenediamine, N,N,N',N'-tetramethylpropylenediamine, N,N,N',N",N"-pentamethyldiethylenetriamine, N,N,N',N",N"-pentamethyl-(3-aminopropyl)ethylenediamine, N,N,N',N",N"-pentamethyldipropylenetriamine, N,N,N',N'-tetramethylhexamethylenediamine, bis(2-dimethylaminoethyl)ether, dimethylethanolamine, dimethylisopropanolamine, dimethylaminoethoxyethanol, N,N-dimethylhexanolamine, N,N-dimethyl-N'-(2-hydroxyethyl)ethylenediamine, N,N-dimethyl-N'-(2-hydroxyethyl)propanediamine, N,N,N'-trimethylaminoethylethanolamine, bis(dimethylaminopropyl)amine, bis(dimethylaminopropyl)isopropanolamine, N-methyl-N'-(2-dimethylaminoethyl)piperazine, N-methyl-N'-(2-hydroxyethyl)piperazine, N-methylmorpholine, N-ethylmorpholine, 1-methylimidazole, 1,2-dimethylimidazole, 1-isobutyl-2-methylimidazole, 1-dimethylaminopropylimidazole, 1-(2-hydroxyethyl)imidazole, 1-(2-hydroxypropyl)imidazole, 1-(2-hydroxyethyl)-2-methylimidazole, 1-(2-hydroxypropyl)-2-methylimidazole, etc.

Examples of ammonium salt catalysts include: quaternary ammonium salts such as tetraethylhydroxylammonium, etc.; ammonium salts of 1,8-diazabicyclo(5,4,0)-undecene-7 or 1,5-diazabicyclo(4,3,0)-nonene-5, formed with octylic acid, olein acid, p-toluenesulfonic acid, formic acid, phenolic acid, orthophthalic acid, acetic acid, maleic acid, or boric acid; etc.

Examples of potassium salt catalysts include potassium carbonate, potassium acetate, potassium octylate, etc.

Examples of organometallic catalysts include: organic tin compounds such as tin acetate, tin octylate (tin 2-ethylhexanoate), tin oleate, tin laurate, dibutyltin diacetate, dimethyltin dilaurate, dibutyltin dilaurate, dibutyltin dimercaptide, dibutyltin maleate, dibutyltin dineodecanoate, dioctyltin dimercaptide, dioctyltin dilaurylate, and dibutyltin dichloride; organic lead compounds such as lead octoate, lead naphthenate, etc.; organic nickel compounds such as nickel naphthenate, etc.; organic cobalt compounds such as cobalt naphthenate, etc.; organocopper compounds such as copper octenoate, etc.; and organic bismuth compounds such as bismuth octylate, bismuth neodecanoate, etc.

These urethanization catalyst can be used solely or in combination of two kinds or more. The amount urethanization catalyst used is preferably 0 parts by mass or more and 5 parts by mass or less, more preferably 0.1 parts by mass or more and 2 parts by mass or less relative to 100 parts by mass of entire polyurethane components.

In the inventive bio-electrode composition, the blended amount of the component (A) is not specifically limited, but preferably is 10 to 10,000 parts by mass, more preferably 20 to 2,000 parts by mass relative to 100 parts by mass of the component (B). The component (A) may be used solely, or may be used in combination of two kinds or more.

### [(B) Resin]

The resin (B) contained in the inventive bio-electrode composition is a component that is compatible with the above component (A) to prevent elution of salts, holds an electric conductivity improver such as metal powder, carbon powder, silicon powder, lithium titanate powder, etc., and enhances adhesiveness further. When the component (A) has sufficient adhesiveness, the resin (B) is not always necessary. Note that, the resin (B) may be any resin other than the above-described component (A), and is preferably either or both of a thermosetting resin and a photo-curable resin. Particularly it is preferable that the resin (B) is one or more resins selected from the group consisting of silicone resins, (meth)acrylate resins, and urethane resins.

Examples of the adhesive silicone resin include an addition-curable type and a radical crosslinking reaction-curable type. As the addition reaction-curable type, for example, it is possible to use one that contains diorganosiloxane having an alkenyl group(s), an MQ resin having a R₃SiO_{0.5} unit and a SiO₂ unit, organohydrogenpolysiloxane having multiple SiH groups, a platinum catalyst, an addition-reaction inhibitor, and an organic solvent, as disclosed in JP 2015-193803 A. As the radical crosslinking reaction-curable type, for example, it is possible to use one that contains diorganopolysiloxane with or without an alkenyl group, an MQ resin having a R₃SiO_{0.5} unit and SiO₂ unit, organic peroxide, and an organic solvent, as disclosed in JP 2015-193803 A. Here, R represents a substituted or unsubstituted monovalent hydrocarbon group having 1 to 10 carbon atoms.

It is also possible to use a polysiloxane-resin integrated compound that is formed by condensation reaction of an MQ resin and polysiloxane having silanol at the terminal or the side chain of the polymer. The MQ resin contains many silanols, and improves adhesive strength when contained, but does not bond to the polysiloxane in molecular level because it is not crosslinkable. The adhesive strength can be increased by integrating the polysiloxane and the resin as described above.

The silicone resin may contain modified siloxane that has a group selected from the group consisting of an amino group, an oxirane group, an oxetane group, a polyether group, a hydroxy group, a carboxy group, a mercapto group, a methacryl group, an acryl group, a phenol group, a silanol group, a carboxylic anhydride group, an aryl group, an aralkyl group, an amide group, an ester group, and a lactone ring. The addition of the modified siloxane improves dispersibility of the component (A) in the silicone resin. The modified siloxane may be modified at any part such as one terminal, both terminals, or a side chain of the siloxane.

As the adhesive (meth)acrylic-based resin, for example, it is possible to use one having hydrophilic (meth)acrylic ester and hydrophobic long chain (meth)acrylic ester as a repeating unit, as disclosed in JP 2016-011338 A. In some cases, it is also possible to copolymerize (meth)acrylic ester having a functional group or (meth)acrylic ester having a siloxane bond.

As the adhesive urethane resin, for example, it is possible to use one having a urethane bond with polyether, a polyester bond, a polycarbonate bond, or a siloxane bond, as disclosed in JP 2016-065238 A.

In the inventive bio-electrode composition, the resin (B) preferably has high compatibility with the component (A) to prevent lowering of the electric conductivity due to separation of the component (A) from the living body contact layer. Further, in the inventive bio-electrode composition, the resin (B) preferably has high adhesion to the electro-conductive base material to prevent peeling of the living body contact layer from the electro-conductive base material. In order to increase the compatibility with the electro-conductive base material and the salt, the use of a resin with high polarity is effective. Examples of such a resin include: resins having one or more moieties selected from an ether bond, an ester bond, an amide bond, an imide bond, a urethane bond, a thiourethane bond, and a thiol group; a polyacrylic resin; a polyamide resin; a polyimide resin; a polyurethane resin, a polythiourethane resin; etc. On the other hand, the living body contact layer is to be in contact with the living body and thus is susceptible to the perspiration of the living body. Accordingly, in the inventive bio-electrode composition, the resin (B) preferably has high repellency and is hardly hydrolyzed. To make the resin (B) highly repellent and hardly hydrolyzed, the use of a silicon atom-containing resin is effective.

The silicon atom-containing polyacrylic resin includes a polymer that has a silicone in a main chain and a polymer that has a silicon atom(s) in a side chain, and either thereof can be suitably used. As the polymer that has a silicone in a main chain, siloxane, silsesquioxane, or the like having a (meth)acrylpropyl group can be used. In this case, an addition of a photoradical generator can allow the (meth)acryl moiety to be polymerized to cured.

As the silicon atom-containing polyamide resin, for example, it is possible to suitably use polyamide silicone resins or the like disclosed in JP 2011-079946 A and US 5981680 A. Such polyamide silicone resins can be synthesized, for example, by combining a silicone or non-silicone compound having amino groups at both terminals and a non-silicone or silicone compound having carboxy groups at both terminals, so as to contain a silicon atom.

Further, it is also possible to use polyamic acid before cyclization thereof, which is obtained by reacting carboxylic anhydride and amine. The carboxy group of the polyamic acid may be crosslinked by using for a crosslinking agent such as an epoxy type and an oxetane type. It is also possible to esterify a carboxy group with hydroxyethyl (meth)acrylate to perform photoradical crosslinking of the (meth)acrylate moiety.

As the silicon atom-containing polyimide resin, it is possible to suitably use polyimide silicone resins disclosed in JP 2002-332305 A, for example. Although polyimide resins have very high viscosity, the viscosity can be decreased by blending a (meth)acrylic monomer as both a solvent and a crosslinking agent.

Examples of the silicon atom-containing polyurethane resin include polyurethane silicone resins. Such polyurethane silicone resins can be crosslinked with urethane bonding by blending a compound having isocyanate groups at both terminals and a compound having a hydroxy group(s) at the terminal(s) and heating the blended. In this case, either or both of the compound having isocyanate groups at both terminals and the compound having a hydroxy group at the terminal have to contain a silicon atom(s) (a siloxane bond). Alternatively, polysiloxane and a urethane (meth)acrylate monomer can be blended and photocrosslinked as disclosed in JP 2005-320418 A. It is also possible to photo-crosslink a polymer having a (meth)acrylate group at the terminal, and both of a siloxane bond and a urethane bond. Particularly, a material having a polyurethane in the main chain and a silicone chain in the side chain as disclosed in JP 2018-123304 A and JP 2019-070109 A has properties of high strength and high stretchability, and is preferable.

The silicon atom-containing polythiourethane resin can be obtained by reaction of a compound having a thiol group and a compound having an isocyanate group, provided that at least one of them contains a silicon atom(s). The resin can also be photo-cured when a (meth)acrylate group are contained at the terminal.

The compatibility of a silicone resin with the component (A) can be improved by adding modified siloxane that has a group selected from the group consisting of an amino group, an oxirane group, an oxetane group, a polyether group, a hydroxy group, a carboxy group, a mercapto group, a methacryl group, an acryl group, a phenol group, a silanol group, a carboxylic anhydride group, an aryl group, an aralkyl group, an amide group, an ester group, and a lactone ring, in addition to the above diorganopolysiloxane having an alkenyl group(s), the above MQ resin having a R₃SiO_{0.5} unit and a SiO₂ unit, and the above organohydrogenpolysiloxane having multiple SiH groups.

The diorganopolysiloxane having an alkenyl group and the organohydrogenpolysiloxane having multiple SiH groups can be crosslinked by an addition reaction with a platinum catalyst.

Examples of the platinum catalyst include: platinum-based catalysts such as chloroplatinic acid, alcohol solution of chloroplatinic acid, reaction product of chloroplatinic acid and alcohol, reaction product of chloroplatinic acid and an olefin compound, reaction product of chloroplatinic acid and vinyl group-containing siloxane, a platinum-olefin complex, a complex of platinum and vinyl group-containing siloxane, etc.; and platinum group metal-based catalysts such as a rhodium complex, a ruthenium complex, etc. These catalysts may be used after being dissolved or dispersed in alcohol solvent, hydrocarbon solvent, or siloxane solvent.

Note that, the platinum catalyst is contained in an amount within preferably 5 to 2,000 ppm, particularly preferably 10 to 500 ppm, based on 100 parts by mass of the total resin including the components (A) and (B).

In the inventive bio-electrode composition, the component (B) is preferably contained in an amount of 0 to 2,000 parts by mass, more preferably 10 to 1000 parts by mass, based on 100 parts by mass of the component (A). Each of the components (A) and (B) may be used in one kind solely, or may be used in combination of two or more kinds in mixture.

When the addition-curable silicone resin is used, an addition-reaction inhibitor may be added. This addition-reaction inhibitor is added as a quencher to prevent the action of the platinum catalyst in the solution and under a low temperature circumstance before heat-curing the coating film after forming the coating film. Specific examples of the addition-reaction inhibitor include 3-methyl-1-butyn-3-ol, 3-methyl-1-pentyn-3-ol, 3,5-dimethyl-1-hexyn-3-ol, 1-ethynylcyclohexanol, 3-methyl-3-trimethylsiloxy-1-butyne, 3-methyl-3-trimethylsiloxy-1-pentyne, 3,5-dimethyl-3-trimethylsiloxy-1-hexyne, 1-ethynyl-1-trimethylsiloxycyclohexane, bis(2,2-dimethyl-3-butynoxy)dimethylsilane, 1,3,5,7-tetramethyl-1,3,5,7-tetravinylcyclotetrasiloxane, 1,1,3,3-tetramethyl-1,3-divinyldisiloxane, etc.

The addition-reaction inhibitor is preferably contained in an amount of 0 to 10 parts by mass, particularly preferably 0.05 to 3 parts by mass, based on 100 parts by mass of total resin including the components (A) and (B).

When the component (B) has a double bond capable of radical crosslinking, it is effective to add a radical generator. Examples of the radical generators include photoradical generators and thermal-radical generators.

Examples of the photoradical generator include acetophenone, 4,4'-dimethoxybenzyl, benzyl, benzoin, benzophenone, 2-benzoylbenzoic acid, 4,4'-bis(dimethylamino)benzophenone, 4,4'-bis(diethylamino)benzophenone, benzoin methyl ether, benzoin ethyl ether, benzoin isopropyl ether, benzoin butyl ether, benzoin isobutyl ether, 4-benzoylbenzoic acid, 2,2'-bis(2-chlorophenyl)-4,4',5,5'-tetraphenyl-1,2'-biimidazole, methyl 2-benzoylbenzoate, 2-(1,3-benzodioxole-5-yl)-4,6-bis(trichloromethyl)-1,3,5-triazine, 2-benzyl-2-(dimethylamino)-4'-morpholinobutyrophenone, 4,4'-dichlorobenzophenone, 2,2-diethoxyacetophenone, 2,2-dimethoxy-2-phenylacetophenone, 2,4-diethylthioxanthene-9-one, diphenyl(2,4,6-trimethylbenzoyl)phosphine oxide (BAPO), 1,4-dibenzoylbenzene, 2-ethylanthraquinone, 1-hydroxycyclohexyl phenyl ketone, 2-hydroxy-2-methylpropiophenone, 2-hydroxy-4'-(2-hydroxyethoxy)-2-methylpropiophenone, 2-isonitrosopropiophenone, and 2-phenyl-2-(p-toluenesulfonyloxy)acetophenone.

The curing can also be performed by adding a radical generator of a heat decomposition type. Examples of such a thermal-radical generator can include 2,2'-azobis(isobutyronitrile), 2,2'-azobis(2,4-dimethylvaleronitrile), 2,2'-azobis(2-methylbutyronitrile), 2,2'-azobis(methylpropionamidine) hydrochloride, 2,2'-azobis[2-(2-imidazolin-2-yl)propane] hydrochloride, 2,2'-azobis(4-methoxy-2,4-dimethylvaleronitrile), 2,2'-azobis(cyclohexane-1-carbonitrile), 1[(1-cyano-1-methylethyl)azo]formamide, 2,2'-azobis[2-methyl-N-(2-hydroxyethyl)propionamide], 2,2'-azobis[N-(2-propenyl)-2-methylpropionamide], 2,2'-azobis(N-butyl-2-methylpropionamide), dimethyl-2,2'-azobis(isobutylate), 4,4'-azobis(4-cyanopentanoic acid), dimethyl-2,2'-azobis(2-methylpropionate), benzoyl peroxide, tert-butyl hydroperoxide, cumene hydroperoxide, di-tert-butyl peroxide, di-tert-amyl peroxide, di-n-butyl peroxide, dicumyl peroxide, etc.

Note that, the added amount of the radical generator is preferably in the range of 0.1 to 50 parts by mass based on 100 parts by mass of the total resin of the components (A) and (B) combined.

As described below, the living body contact layer is a cured product of the bio-electrode composition. By curing the bio-electrode composition, the resulting living body contact layer has favorable adhesion to both the skin and the electro-conductive base material. The curing means is not particularly limited, and common means can be used, including crosslinking reaction by either or both of heat and light, or with an acid catalyst or a base catalyst, for example. The crosslinking reaction can be performed, for example, by appropriately selecting methods described in "Kakyou han-nou handbook (handbook of crosslinking reaction)", Chapter 2, pages 51-371, Yasuharu Nakayama, Maruzen Publishing Co., Ltd. (2013).

### [Ionic Polymer]

The inventive bio-electrode composition may contain an ionic polymer besides the component (A). As the ionic polymer, it is possible to use polymers disclosed in JP 2018-099504 A, JP 2018-110845 A, or JP 2018-130533 A. The ionic polymer is preferably contained in an amount of 0.1 to 100 parts by mass based on 100 parts by mass of the total resin of the components (A) and (B) combined.

### [(C) Carbon Material and/or Metal Powder]

The inventive bio-electrode composition preferably further contains a carbon material and/or a metal powder as a component (C).

### [Metal Powder]

The inventive bio-electrode composition may also contain a metal powder selected from the group consisting of gold, silver, platinum, copper, tin, titanium, nickel, aluminum, tungsten, molybdenum, ruthenium, chromium, and indium, in order to improve electron conductivity. The metal powder is preferably added in an amount of 1 to 50 parts by mass based on 100 parts by mass of the total resin of the components (A) and (B) combined.

As kind of the metal powder, gold, silver, and platinum are preferable from the viewpoint of electric conductivity. From the viewpoint of cost, silver, copper, tin, titanium, nickel, aluminum, tungsten, molybdenum, ruthenium, and chromium are preferable. From the viewpoint of biocompatibility, noble metals are preferable. From a comprehensive viewpoint including the above, silver (silver powder) is the most preferable.

Shapes of the metal powder can include spherical, disk-shaped, flaky, and needle-shaped, but the addition of flaky powder brings the highest electric conductivity and is preferable. The metal powder is preferably a flake having relatively low density and large specific surface area with a size of 100 µm or less, a tapped density of 5 g/cm³ or less, and a specific surface area of 0.5 m²/g or more.

### [Carbon Material]

A carbon material can be contained as an electric conductivity improver. Examples of the carbon material can include carbon black, graphite, carbon nanotube, carbon fiber, graphene, etc. It is especially preferable to contain either or both of carbon black and carbon nanotube. The carbon nanotube may be either single layer or multilayer, and the surface may be modified with an organic group(s). The carbon material is preferably contained in an amount of 1 to 50 parts by mass based on 100 parts by mass of the total resin of the components (A) and (B) combined.

### [Silicon Powder]

The inventive bio-electrode composition may contain a silicon powder to enhance ion reception sensitivity. Examples of the silicon powder can include powders of silicon, silicon monoxide, and silicon carbide. The particle size of the powder is preferably smaller than 100 pm, more preferably 1 µm or less. Since finer particles provide a larger surface area, the resulting bio-electrode can receive a larger number of ions and has higher sensitivity. The silicon powder is preferably contained in an amount of 1 to 50 parts by mass based on 100 parts by mass of the total resin of the components (A) and (B) combined.

### [Lithium Titanate Powder]

The inventive bio-electrode composition may contain a lithium titanate powder to enhance ion reception sensitivity. Examples of the lithium titanate powder can include powders containing a compound represented by a molecular formulae Li₂TiO₃, LiTiO₂, Li₄Ti₅O₁₂ with a spinel structure. The lithium titanate powder preferably has a spinel structure. It is also possible to use carbon-incorporated lithium titanate particles. The particle size of the powder is preferably smaller than 100 pm, more preferably 1 µm or less. Since finer particles provide a larger surface area, the bio-electrode can receive a larger number of ions has higher sensitivity. These powders may be composite powders with carbon. The lithium titanate powder is preferably contained in an amount of 1 to 50 parts by mass based on 100 parts by mass of the total resin of the components (A) and (B) combined.

### [Crosslinking Agent]

The inventive bio-electrode composition may contain an epoxy-type crosslinking agent. This crosslinking agent is a compound having multiple epoxy groups or oxetane groups in one molecule. The amount of the crosslinking agent to be contained can preferably be 1 to 30 parts by mass based on 100 parts by mass of the total resin of the components (A) and (B) combined.

### [Crosslinking Catalyst]

The inventive bio-electrode composition may also contain a catalyst for crosslinking the epoxy groups or the oxetane groups. For this catalyst, those disclosed in paragraphs [0027] to [0029] of JP 2019-503406 A can be used. The catalyst is preferably contained in an amount of 0.01 to 10 parts by mass based on 100 parts by mass of the total resin of the components (A) and (B) combined.

### [Ionic Additive]

The inventive bio-electrode composition may contain an ionic additive to enhance the ionic conductivity. In consideration of biocompatibility, examples of the ionic additive can include sodium chloride, potassium chloride, calcium chloride, magnesium chloride, saccharin sodium salt, acesulfame potassium, sodium carboxylate, potassium carboxylate, calcium carboxylate, sodium sulfonate, potassium sulfonate, calcium sulfonate, sodium phosphate, potassium phosphate, calcium phosphate, magnesium phosphate, betaines, and salts disclosed in JP 2018-044147 A, JP 2018-059050 A, JP 2018-059052 A, and JP 2018-130534 A. The ionic additive is preferably contained in an amount of 0.1 to 50 parts by mass based on 100 parts by mass of the total resin of the components (A) and (B) combined.

### [(D) Organic Solvent]

In addition, the inventive bio-electrode composition may contain an organic solvent as a component (D). Specific examples of the organic solvent can include: aromatic hydrocarbon solvents such as toluene, xylene, cumene, 1,2,3-trimethylbenzene, 1,2,4-trimethylbenzene, 1,3,5-trimethylbenzene, styrene, α-methylstyrene, butylbenzene, sec-butylbenzene, isobutylbenzene, cymene, diethylbenzene, 2-ethyl-p-xylene, 2-propyltoluene, 3-propyltoluene, 4-propyltoluene, 1,2,3,5-tetramethyltoluene, 1,2,4,5-tetramethyltoluene, tetrahydronaphthalene, 4-phenyl-1-butene, tert-amylbenzene, amylbenzene, 2-tert-butyltoluene, 3-tert-butyltoluene, 4-tert-butyltoluene, 5-isopropyl-m-xylene, 3-methylethylbenzene, tert-butyl-3-ethylbenzene, 4-tert-butyl-o-xylene, 5-tert-butyl-m-xylene, tert-butyl-p-xylene, 1,2-diisopropylbenzene, 1,3-diisopropylbenzene, 1,4-diisopropylbenzene, dipropylbenzene, pentamethylbenzene, hexamethylbenzene, hexylbenzene, 1,3,5-triethylbenzene, etc.; aliphatic hydrocarbon solvents such as n-heptane, isoheptane, 3-methylhexane, 2,3-dimethylpentane, 3-ethylpentane, 1,6-heptadiene, 5-methyl-1-hexyne, norbornane, norbornene, dicyclopentadiene, 1-methyl-1,4-cyclohexadiene, 1-heptyne, 2-heptyne, cycloheptane, cycloheptene, 1,3-dimethylcyclopentane, ethylcyclopentane, methylcyclohexane, 1-methyl-1-cyclohexene, 3-methyl-1-cyclohexene, methylenecyclohexane, 4-methyl-1-cyclohexene, 2-methyl-1-hexene, 2-methyl-2-hexene, 1-heptene, 2-heptene, 3-heptene, n-octane, 2,2-dimethylhexane, 2,3-dimethylhexane, 2,4-dimethylhexane, 2,5-dimethylhexane, 3,3-dimethylhexane, 3,4-dimethylhexane, 3-ethyl-2-methylpentane, 3-ethyl-3-methylpentane, 2-methylheptane, 3-methylheptane, 4-methylheptane, 2,2,3-trimethylpentane, 2,2,4-trimethylpentane, cyclooctane, cyclooctene, 1,2-dimethylcyclohexane, 1,3-dimethylcyclohexane, 1,4-dimethylcyclohexane, ethylcyclohexane, vinylcyclohexane, isopropylcyclopentane, 2,2-dimethyl-3-hexene, 2,4-dimethyl-1-hexene, 2,5-dimethyl-1-hexene, 2,5-dimethyl-2-hexene, 3,3-dimethyl-1-hexene, 3,4-dimethyl-1-hexene, 4,4-dimethyl-1-hexene, 2-ethyl-1-hexene, 2-methyl-1-heptene, 1-octene, 2-octene, 3-octene, 4-octene, 1,7-octadiene, 1-octyne, 2-octyne, 3-octyne, 4-octyne, n-nonane, 2,3-dimethylheptane, 2,4-dimethylheptane, 2,5-dimethylheptane, 3,3-dimethylheptane, 3,4-dimethylheptane, 3,5-dimethylheptane, 4-ethylheptane, 2-methyloctane, 3-methyloctane, 4-methyloctane, 2,2,4,4-tetramethylpentane, 2,2,4-trimethylhexane, 2,2,5-trimethylhexane, 2,2-dimethyl-3-heptene, 2,3-dimethyl-3-heptene, 2,4-dimethyl-1-heptene, 2,6-dimethyl-1-heptene, 2,6-dimethyl-3-heptene, 3,5-dimethyl-3-heptene, 2,4,4-trimethyl-1-hexene, 3,5,5-trimethyl-1-hexene, 1-ethyl-2-methylcyclohexane, 1-ethyl-3-methylcyclohexane, 1-ethyl-4-methylcyclohexane, propylcyclohexane, isopropylcyclohexane, 1,1,3-trimethylcyclohexane, 1,1,4-trimethylcyclohexane, 1,2,3-trimethylcyclohexane, 1,2,4-trimethylcyclohexane, 1,3,5-trimethylcyclohexane, allylcyclohexane, hydrindane, 1,8-nonadiene, 1-nonyne, 2-nonyne, 3-nonyne, 4-nonyne, 1-nonene, 2-nonene, 3-nonene, 4-nonene, n-decane, 3,3-dimethyloctane, 3,5-dimethyloctane, 4,4-dimethyloctane, 3-ethyl-3-methylheptane, 2-methylnonane, 3-methylnonane, 4-methylnonane, tert-butylcyclohexane, butylcyclohexane, isobutylcyclohexane, 4-isopropyl-1-methylcyclohexane, pentylcyclopentane, 1,1,3,5-tetramethylcyclohexane, cyclododecane, 1-decene, 2-decene, 3-decene, 4-decene, 5-decene, 1,9-decadiene, decahydronaphthalene, 1-decyne, 2-decyne, 3-decyne, 4-decyne, 5-decyne, 1,5,9-decatriene, 2,6-dimethyl-2,4,6-octatriene, limonene, myrcene, 1,2,3,4,5-pentamethylcyclopentadiene, α-phellandrene, pinene, terpinene, tetrahydrodicyclopentadiene, 5,6-dihydrodicyclopentadiene, dicyclopentadiene, 1,4-decadiyne, 1,5-decadiyne, 1,9-decadiyne, 2,8-decadiyne, 4,6-decadiyne, n-undecane, amylcyclohexane, 1-undecene, 1,10-undecadiene, 1-undecyne, 3-undecyne, 5-undecyne, tricyclo[6.2.1.0^{2,7}]undeca-4-ene, n-dodecane, n-tridecane, n-pentadecane, n-hexadecane, 2-methylundecane, 3-methylundecane, 4-methylundecane, 5-methylundecane, 2,2,4,6,6-pentamethylheptane, 1,3-dimethyladamantane, 1-ethyladamantane, 1,5,9-cyclododecatriene, 1,2,4-trivinylcyclohexane, isoparaffins, etc.; ketone solvents such as cyclohexanone, cyclopentanone, 2-octanone, 2-nonanone, 2-heptanone, 3-heptanone, 4-heptanone, 2-hexanone, 3-hexanone, diisobutyl ketone, methylcyclohexanone, methyl n-pentyl ketone, etc.; alcohol solvents such as 3-methoxybutanol, 3-methyl-3-methoxybutanol, 1-methoxy-2-propanol, 1-ethoxy-2-propanol, etc.; ether solvents such as propylene glycol monomethyl ether, ethylene glycol monomethyl ether, propylene glycol monoethyl ether, ethylene glycol monoethyl ether, propylene glycol dimethyl ether, diethylene glycol monomethyl ether, diethylene glycol monoethyl ether, diethylene glycol monopropyl ether, diethylene glycol monobutyl ether, diethylene glycol monopentyl ether, diethylene glycol monoheptyl ether, diethylene glycol diethyl ether, diethylene glycol dipropyl ether, diethylene glycol dibutyl ether, diisopropyl ether, diisobutyl ether, diisopentyl ether, di-n-pentyl ether, methyl cyclopentyl ether, methyl cyclohexyl ether, di-n-butyl ether, di-sec-butyl ether, di-sec-pentyl ether, di-tert-amyl ether, di-n-hexyl ether, anisole, etc.; ester solvents such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, propylene glycol mono-tert-butyl ether acetate, etc.; lactone solvents such as γ-butyrolactone, etc.; and water, etc.

Note that, (D) organic solvent is preferably contained in an amount of 10 to 50,000 parts by mass based on 100 parts by mass of the total resin of the components (A) and (B) combined.

### [Other Additives]

The inventive bio-electrode composition can also be mixed with silica particles, polyether silicone, polyether, polyglycerin, polyglycerin silicone. Silica particles have hydrophilic surfaces and favorable compatibility with hydrophilic ion polymer, polyether silicone, and polyglycerin silicone. Therefore, silica particles can improve the dispersibility of the ion polymer, polyether silicone, and polyglycerin silicone in a hydrophobic silicone adhesive. The silica particles can preferably be used either in a dry type or a wet type.

### [Silicone Compound Having Polyglycerin Structure]

The inventive bio-electrode composition may also contain a silicone compound having a polyglycerin structure in order to improve the moisture-holding property of the film and improve the sensitivity to ions released from the skin and the ionic conductivity. The silicone compound having a polyglycerin structure is preferably contained in an amount of 0.01 to 100 parts by mass, more preferably 0.5 to 60 parts by mass, based on 100 parts by mass of the total resin in the components (A) and (B) combined. One kind of the silicone compound having a polyglycerin structure may be used solely, or two or more kinds may be used in mixture.

The silicone compound having a polyglycerin structure is preferably shown by any of the following general formulae (4)' and (5)'.

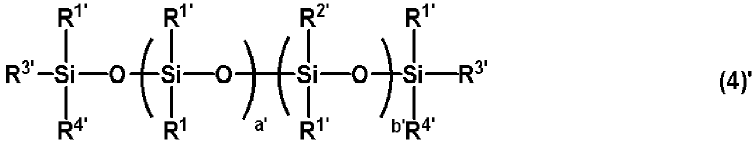

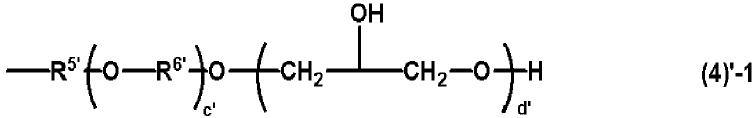

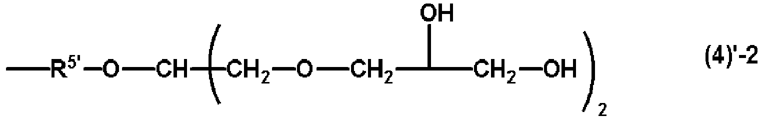

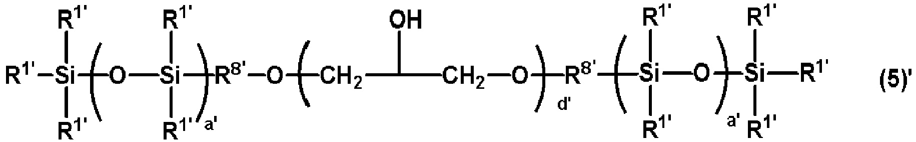

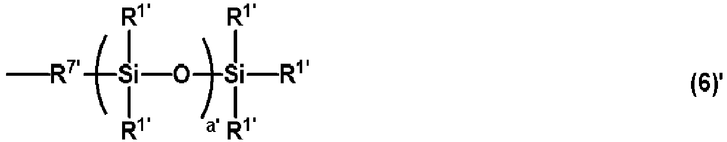

In the formulae, each R¹' is independent and may be identical to or different from one another, and independently represents a hydrogen atom, a linear or branched alkyl group having 1 to 50 carbon atoms, or phenyl group, may have an ether group, may be a silicone chain represented by the general formula (6)'. R²' represents a group having a polyglycerin group structure shown by the general formula (4)'-1 or (4)'-2. Each R³' is independent, may be identical to or different from the other, and is the R¹' or the R²'. Each R⁴' is independent, may be identical to or different from the other, and is the R¹', the R²', or an oxygen atom. When the R⁴' is an oxygen atom, the two R⁴' moieties optionally bond to each other to constitute an ether group to form a ring together with silicon atoms. Each "a'" may be identical to or different from one another and is 0 to 100, "b'" is 0 to 100, and a'+b' is 0 to 200. Nevertheless, when "b'" is 0, at least one R³' is the R²'. R⁵' represents an alkylene group having 2 to 10 carbon atoms or an aralkylene group having 7 to 10 carbon atoms. R⁶', R⁷', and R⁸' each represent an alkylene group having 2 to 6 carbon atoms, and R⁷' may be an ether group. "c'" is 0 to 20. "d'" is 1 to 20.

Examples of such a silicone compound having a polyglycerin structure can include the following.

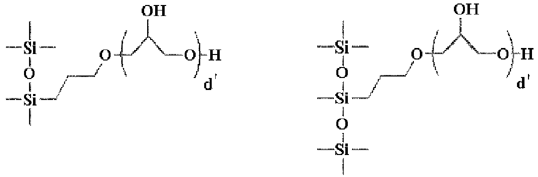

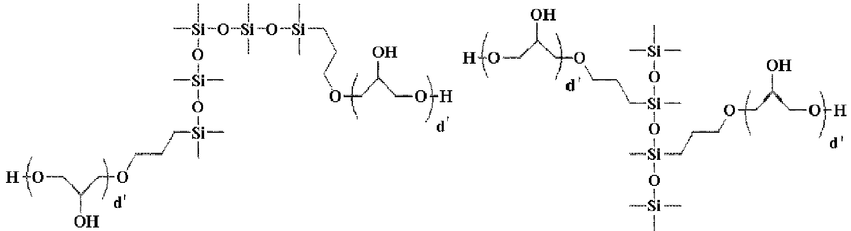

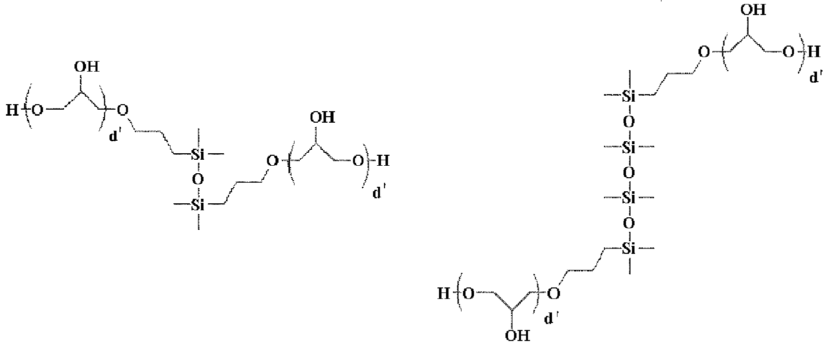

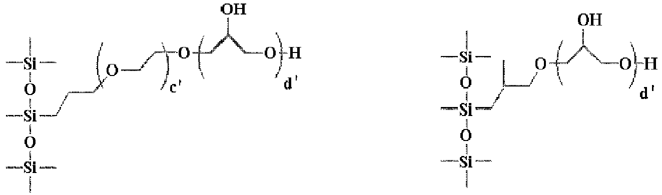

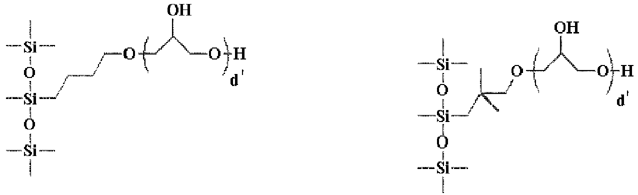

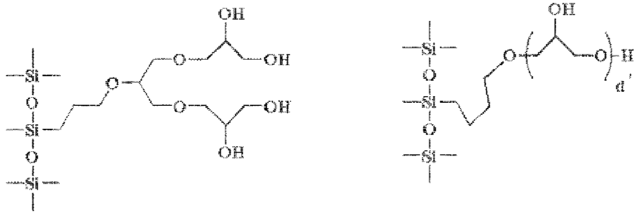

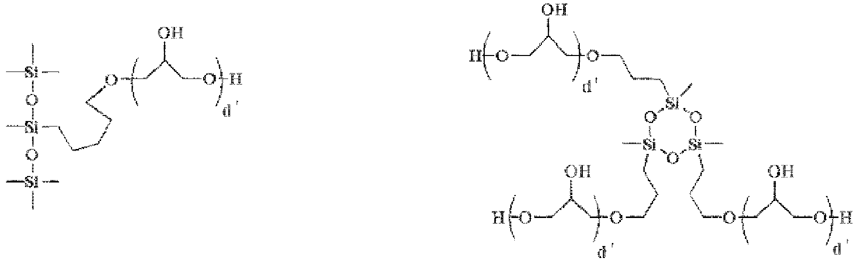

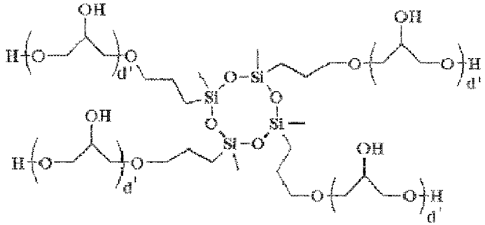

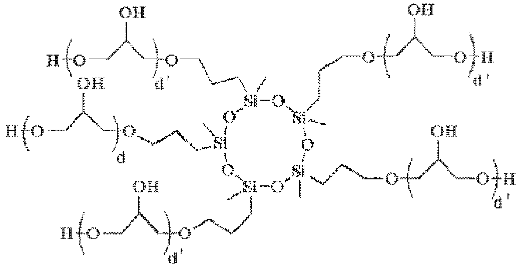

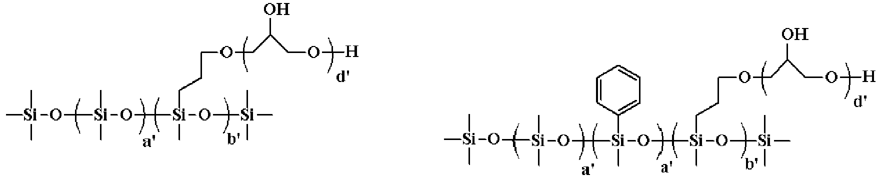

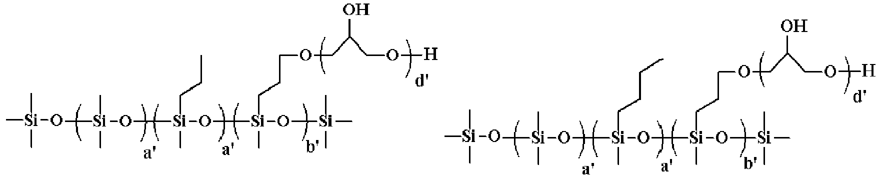

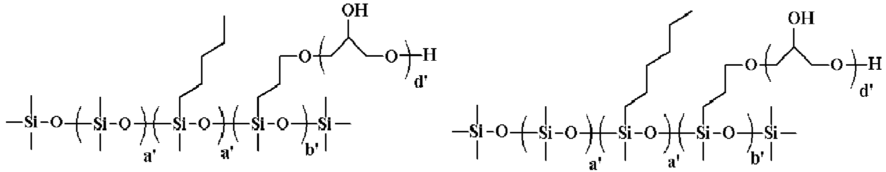

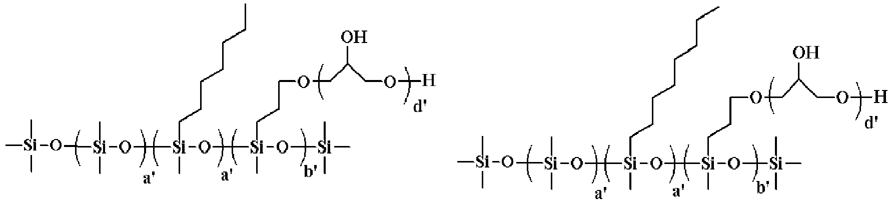

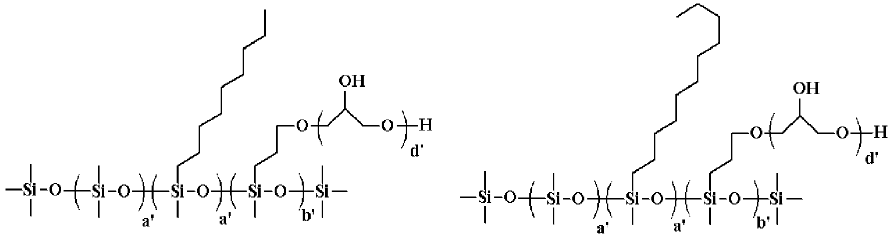

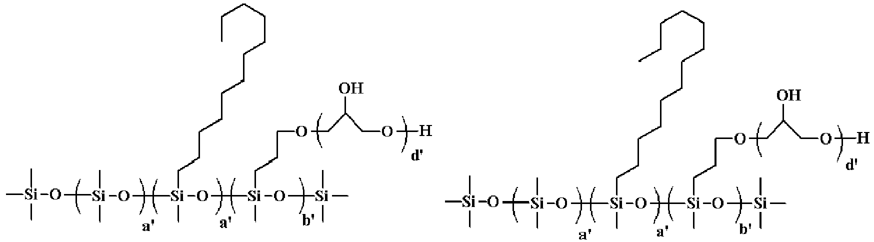

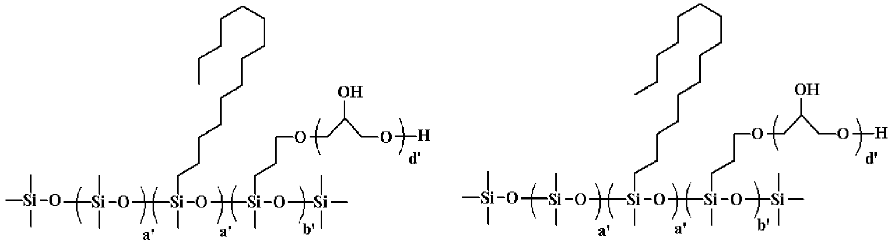

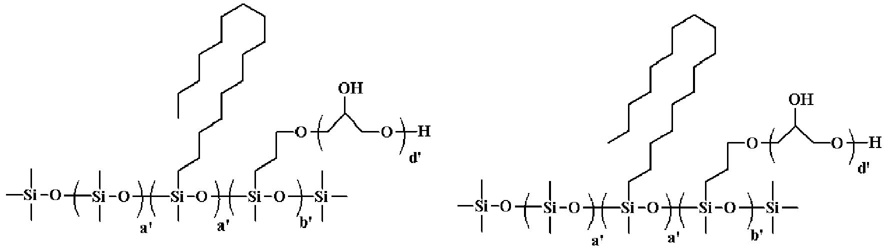

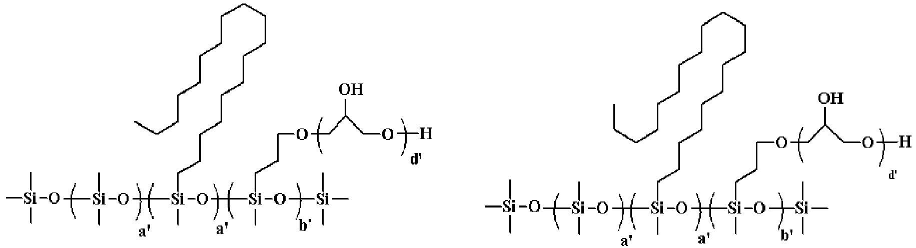

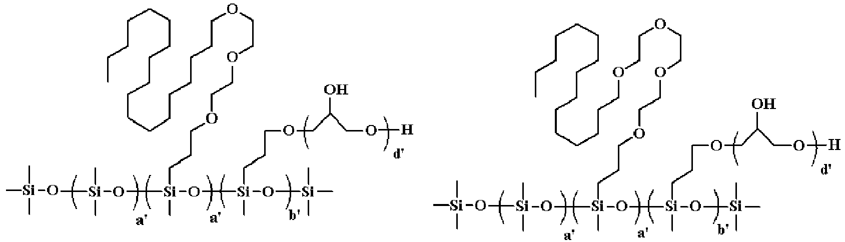

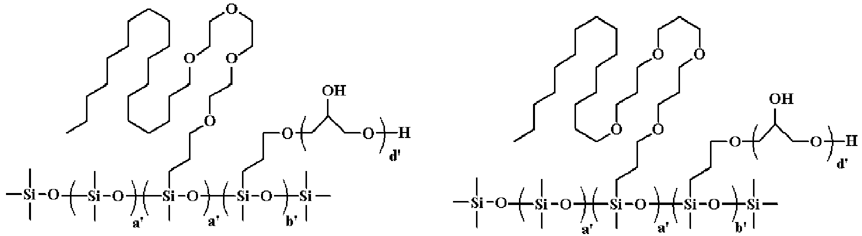

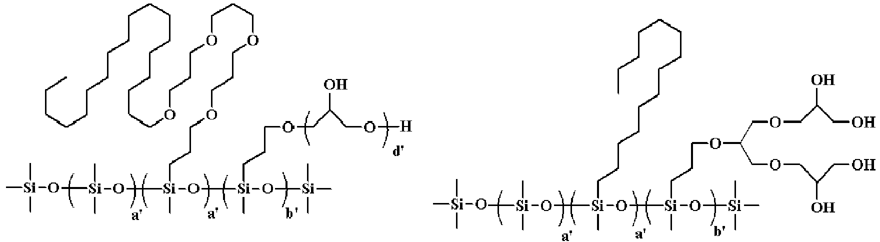

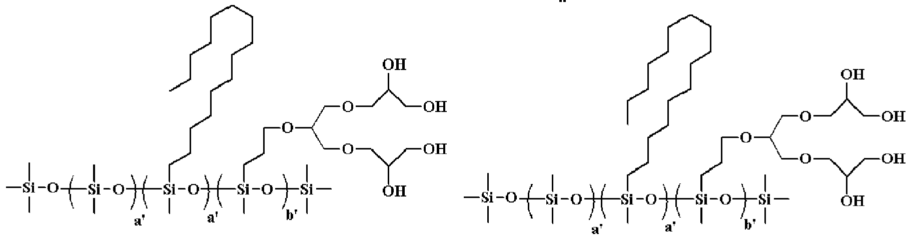

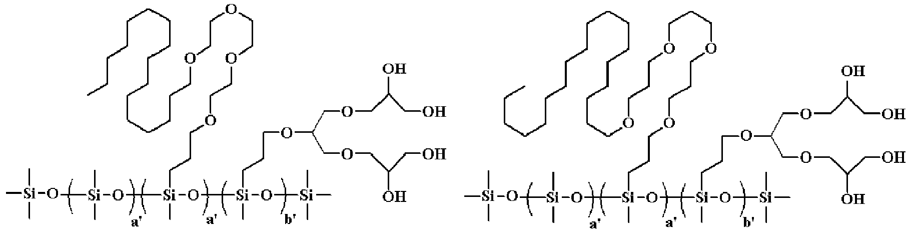

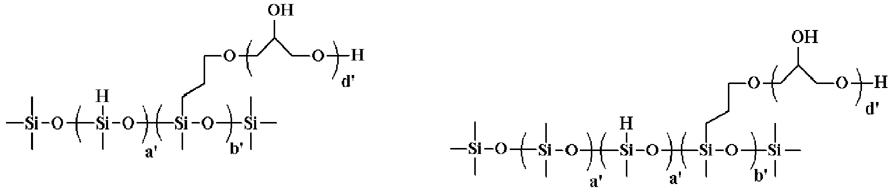

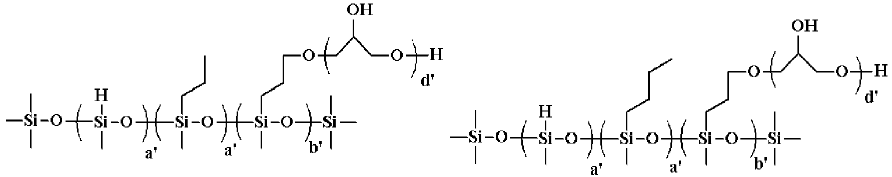

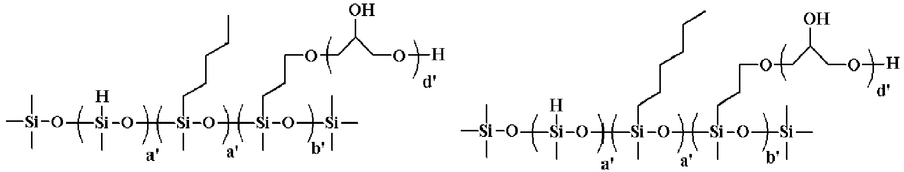

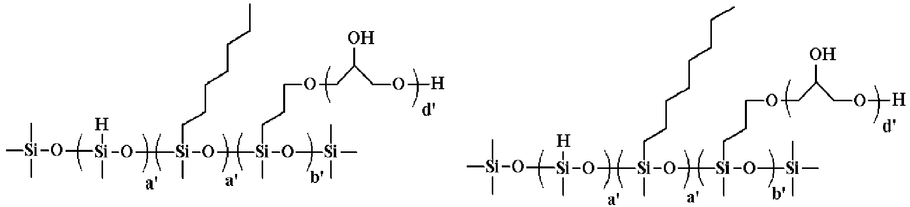

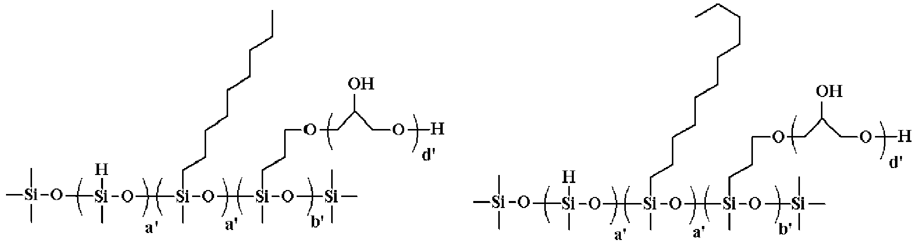

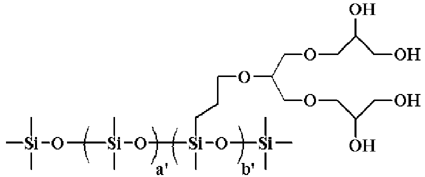

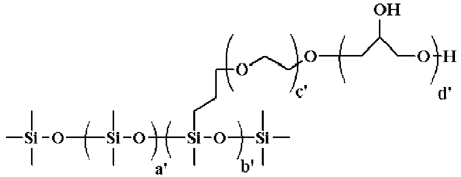

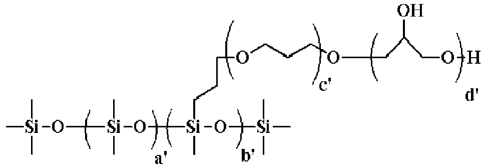

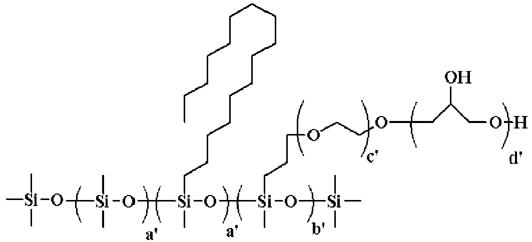

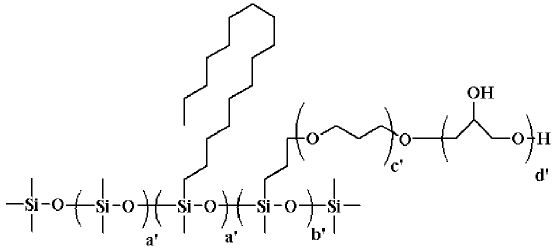

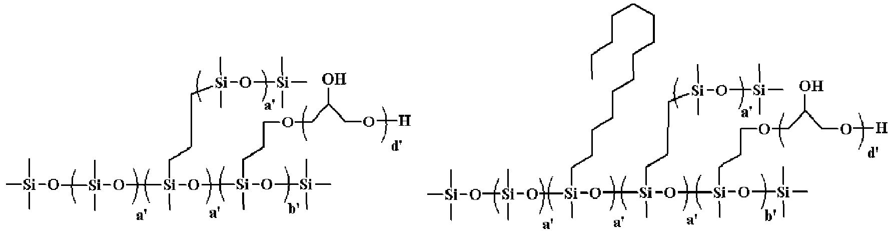

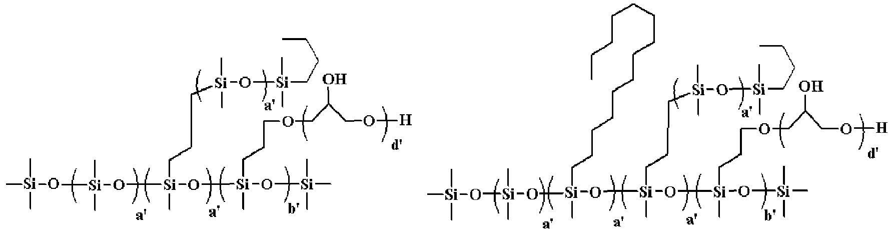

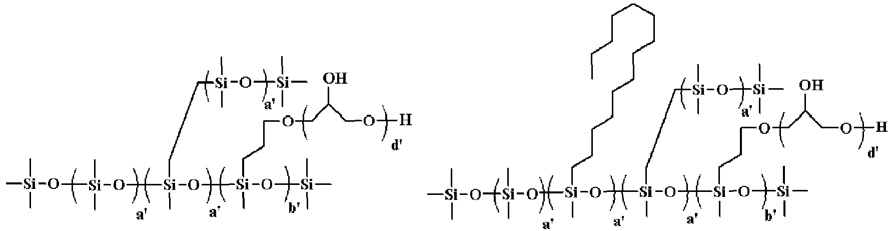

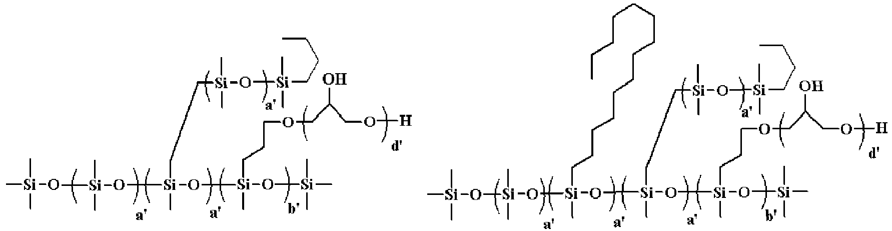

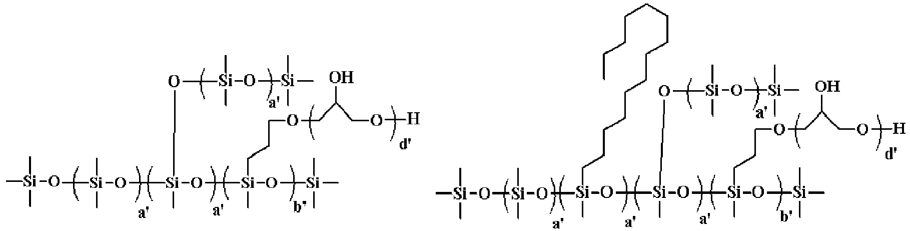

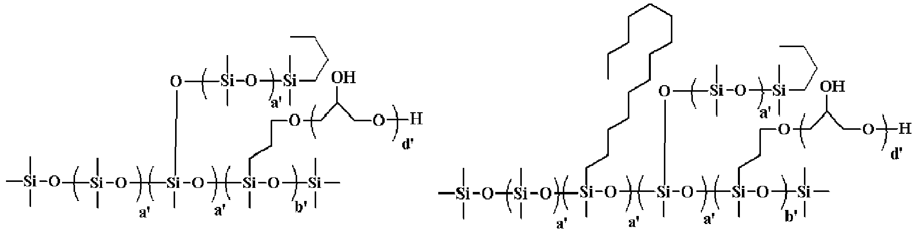

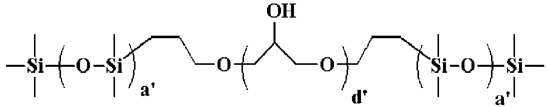

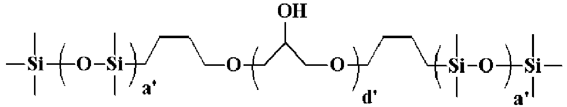

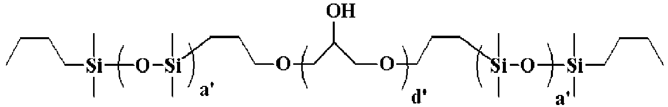

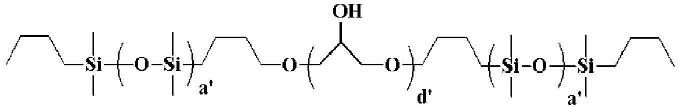

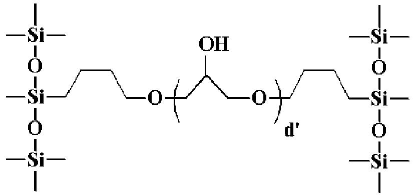

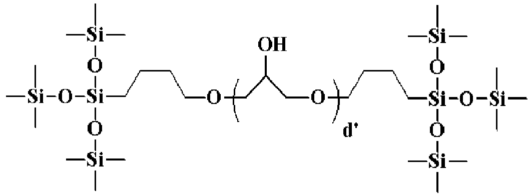

In the formulae, "a'", "b'", "c'", and "d'" are as defined above.

When such a silicone compound having a polyglycerin structure is contained, the resulting bio-electrode composition is capable of forming a living body contact layer that can exhibit more excellent moisture-holding properties and consequently exhibit more excellent sensitivity to ions released from the skin.

As described above, the inventive bio-electrode composition makes it possible to form a living body contact layer for a bio-electrode that is highly adhesive and has sufficient adhesiveness even when detached from the skin and then reattached, capable of efficiently conducting electric signals from the skin to a device (i.e., excellent in electric conductivity), free from risk of causing allergies even when the bio-electrode is attached to the skin for a long period (i.e., excellent in biocompatibility), light-weight, manufacturable at low cost, and free from significant reduction in the electric conductivity even when the bio-electrode is wetted with water or dried. Moreover, it is possible to further enhance the electric conductivity by adding a carbon material, and it is possible to manufacture a bio-electrode with particularly high adhesive strength and high stretchability by combining the inventive bio-electrode composition with a resin having adhesion and stretchability. Further, the stretchability and adhesion to the skin can be enhanced with additives, etc. The stretchability and adhesion can also be controlled by appropriately adjusting the composition of the resin and the thickness of the living body contact layer.

### <Bio-Electrode>

The present invention provides a bio-electrode including an electro-conductive base material and a living body contact layer formed on the electro-conductive base material, the living body contact layer being a cured product of the inventive bio-electrode composition described above.

Hereinafter, the inventive bio-electrode will be described in detail with reference to the drawings, but the present invention is not limited thereto.

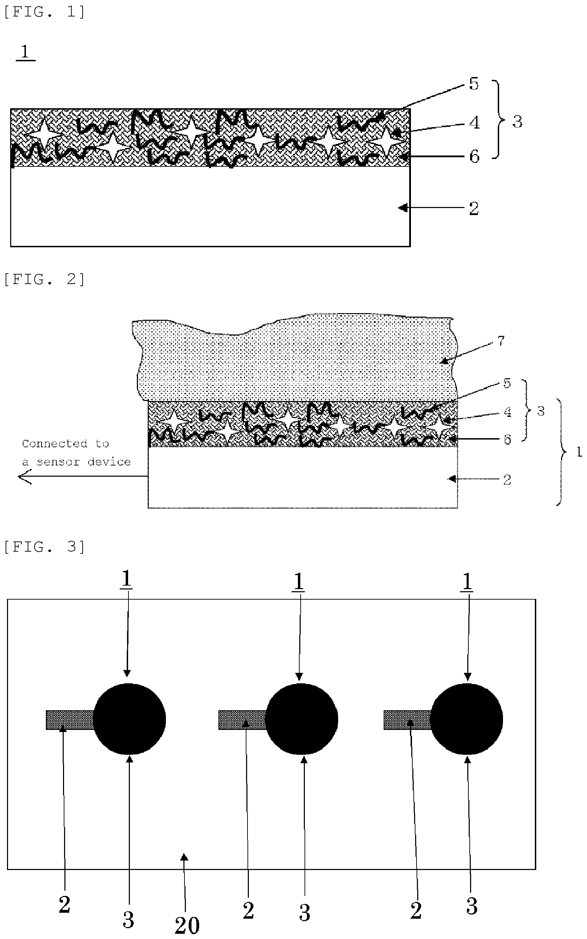

FIG. 1 is a schematic sectional view showing an example of the inventive bio-electrode. In FIG. 1, a bio-electrode 1 has an electro-conductive base material 2 and a living body contact layer 3 formed on the electro-conductive base material 2. The living body contact layer 3 is a cured product of the inventive bio-electrode composition. The living body contact layer 3 contains an ionic urethane resin 5. The living body contact layer 3 can further contain resin 6 other than the ionic urethane resin 5 and an electro-conductive powder 4. Hereinbelow, with reference to FIG.s 1 and 2, the living body contact layer 3 is described as a layer in which the ionic urethane resin 5 and the electro-conductive powder 4 are dispersed in the resin 6. Nevertheless, the inventive bio-electrode is not limited to this embodiment.

When the bio-electrode 1 as shown in FIG. 1 is used, the living body contact layer 3 (i.e., the layer in which the ionic urethane resin 5 and the electro-conductive powder 4 are dispersed in the resin 6) is brought into contact with the living body 7 as shown in FIG. 2. By the ionic urethane resin 5 and the electro-conductive powder 4, electric signals are picked from the living body 7 and conducted to a sensor device or the like (not shown) via the electro-conductive base material 2. As described above, the inventive bio-electrode can achieve both electric conductivity and biocompatibility by using the above-described ionic urethane resin (the component (A)) and obtain electric signals from the skin stably with high sensitivity because the bio-electrode also has adhesiveness and thus the contact area with the skin is kept constant.

Hereinafter, each component of the inventive bio-electrode will be described further in detail.

### [Electro-Conductive Base Material]

The inventive bio-electrode has an electro-conductive base material. This electro-conductive base material is usually connected electrically with a sensor device etc., and conducts electrical signals picked from the living body through the living body contact layer to the sensor device etc.

The electro-conductive base material is not particularly limited, as long as it has electric conductivity. The electro-conductive base material preferably contains one or more selected from the group consisting of gold, silver, silver chloride, platinum, aluminum, magnesium, tin, tungsten, iron, copper, nickel, stainless steel, chromium, titanium, carbon, and electro-conductive polymer, for example.

The electro-conductive base material is not particularly limited, and may be a hard electro-conductive substrate, an electro-conductive film having flexibility, a cloth with the surface coated with electro-conductive paste, a cloth into which electro-conductive polymer is kneaded. The electro-conductive base material may be flat, uneven, or mesh-form of woven metal wires, and can be appropriately selected in accordance with the use of the bio-electrode, etc.

### [Living Body Contact Layer]

The inventive bio-electrode has a living body contact layer formed on the electro-conductive base material. This living body contact layer is a part to be actually in contact with the living body when the bio-electrode is used, and are conductive. In some cases, the living body contact layer has adhesive properties also. The living body contact layer is a cured product of the inventive bio-electrode composition described above, and, that is, an adhesive resin layer formed of a cured composition containing the component (A) described above, and, if necessary, the components (B), (C), and (D), and the other component(s).

Although the living body contact layer may or may not have adhesiveness, but when having adhesiveness, the living body contact layer preferably has an adhesive strength in a range of 0.01 N/25 mm or more and 20 N/25 mm or less. The adhesive strength is commonly measured by the method described in JIS Z 0237, in which a metal substrate such as a stainless steel (SUS) substrate or a polyethylene terephthalate (PET) substrate can be used as a base material. Alternatively, human skin can also be used for measuring. The human skin has lower surface energy than metals and various plastics. Its surface energy is as low as that of Teflon (registered trademark), and thus the human skin has difficulty to be adhered to.

The living body contact layer of the bio-electrode preferably has a thickness of 1 µm or more and 5 mm or less, more preferably 2 µm or more and 3 mm or less. When the living body contact layer is thinner, the adhesive strength lowers, but its flexibility is improved, the weight decreases, and thus the degree of contact is improved. The thickness of the living body contact layer can be selected in consideration of the balance of adhesiveness and texture on the skin.

The inventive bio-electrode may be additionally provided with an adhesive film on the living body contact layer additionally as in conventional bio-electrodes (e.g., the bio-electrode disclosed in JP 2004-033468 A) in order to prevent the bio-electrode peeling off from the living body during use. When the adhesive film is provided additionally, the adhesive film may be formed by using a raw material for the adhesive film such as an acrylic type, a urethane type, and a silicone type, etc. Particularly, the silicone type is suitable because: its high oxygen permeability enables dermal respiration while the electrode is attached to the skin; its high water repellency prevents lowering of adhesion due to perspiration; and irritation to skin is low. Note that, the inventive bio-electrode does not necessarily require to have the adhesive film additionally, because peeling off from the living body can be prevented by adding a tackifier to the bio-electrode composition or using a resin having good adhesion to the living body as described above.

When the inventive bio-electrode is used as a wearable device, wiring between the bio-electrode and a sensor device, and other components are not particularly limited. For example, it is possible to employ ones disclosed in JP 2004-033468 A.

As described above, because the inventive bio-electrode includes the living body contact layer formed of the cured product of the above-described inventive bio-electrode composition, the inventive bio-electrode is capable of efficiently conducting electric signals from skin to a device (i.e., excellent in electric conductivity), does not cause allergies even after long-period attachment to the skin (i.e., excellent in biocompatibility), is light-weight and manufacturable at low cost, and prevents significant reduction in the electric conductivity even when wetted with water or dried. In addition, it is possible to further improve the electric conductivity by adding an electro-conductive powder, and it is possible to manufacture a bio-electrode with particularly high adhesive strength and high stretchability by combining the inventive bio-electrode composition with a resin having adhesion and stretchability. Further, the stretchability against the skin and adhesion to the skin can be improved with use of an additive, etc. The stretchability and the adhesion can also be controlled by appropriately adjusting the composition of the resin and the thickness of the living body contact layer. Accordingly, the inventive bio-electrode as described above is particularly suitable as a bio-electrode used for a medical wearable device.

### <Method for Manufacturing Bio-Electrode>

The present invention also provides a method for manufacturing a bio-electrode having an electro-conductive base material and a living body contact layer formed on the electro-conductive base material, the method including:
applying the inventive bio-electrode composition described above onto the electro-conductive base material; and
curing the bio-electrode composition to form the living body contact layer.

Note that, the electro-conductive base material, etc. used in the inventive method for manufacturing a bio-electrode may be the same as those described above. The electro-conductive base material is preferably one or more kinds selected from the group consisting of gold, silver, silver chloride, platinum, aluminum, magnesium, tin, tungsten, iron, copper, nickel, stainless steel, chromium, titanium, carbon, and electro-conductive polymer, for example.

The method for applying the bio-electrode composition onto the electro-conductive base material is not particularly limited. It is preferable, for example, to use methods such as dip coating, spray coating, spin coating, roll coating, flow coating, doctor coating, screen printing, flexographic printing, gravure printing, inkjet printing, etc.

The method for curing the resin is not particularly limited and can be appropriately selected based on the kind of the components (A) and (B) used for the bio-electrode composition. For example, the bio-electrode composition is preferably cured by either or both of heat and light. The foregoing bio-electrode composition can also be cured through a generated crosslinking reaction caused by a catalyst which is added in advance to generate acid or base to the bio-electrode composition.

The heating temperature is not particularly limited and may be appropriately selected based on the kind of the components (A) and (B) used for the bio-electrode composition, but is preferably about 50 to 250°C, for example.

When the heating and light irradiation are combined, it is possible to perform the heating and the light irradiation simultaneously, to perform the light irradiation and then the heating, or to perform the heating and then the light irradiation. It is also possible to perform air-drying to evaporate the solvent before heating after coating film.

Putting water droplets on the surface of the cured film or spraying water vapor or mist improve the compatibility with the skin, and enable quick collection of biological signals. It is also possible to use water mixed with alcohol in order to reduce the size of the droplets of the water vapor or mist. The film surface may be wetted by bringing an absorbent cotton or cloth containing water into contact therewith.

The water for making the surface of the cured film wet may contain a salt. The water-soluble salt mixed with the water is selected from the group consisting of sodium salts, potassium salts, calcium salts, magnesium salts, and betaines.

Specifically, the water-soluble salt can be a salt selected from the group consisting of sodium chloride, potassium chloride, calcium chloride, magnesium chloride, saccharin sodium salt, acesulfame potassium, sodium carboxylate, potassium carboxylate, calcium carboxylate, sodium sulfonate, potassium sulfonate, calcium sulfonate, sodium phosphate, potassium phosphate, calcium phosphate, magnesium phosphate, and betaines. Note that, the component (A) described above is excluded from the water-soluble salt.

More specific examples of the water-soluble salt include, besides the aforementioned examples, sodium acetate, sodium propionate, sodium pivalate, sodium glycolate, sodium butyrate, sodium valerate, sodium caproate, sodium enanthate, sodium caprylate, sodium pelargonate, sodium caprate, sodium undecylate, sodium laurate, sodium tridecylate, sodium myristate, sodium pentadecylate, sodium palmitate, sodium margarate, sodium stearate, sodium benzoate, disodium adipate, disodium maleate, disodium phthalate, sodium 2-hydroxybutyrate, sodium 3-hydroxybutyrate, sodium 2-oxobutyrate, sodium gluconate, sodium methanesulfonate, sodium 1-nonanesulfonate, sodium 1-decanesulfonate, sodium 1-dodecanesulfonate, sodium 1-undecanesulfonate, sodium cocoyl isethionate, sodium lauroyl methylalanine, sodium methyl cocoyl taurate, sodium cocoyl glutamate, sodium cocoyl sarcosinate, sodium lauroyl methyl taurate, lauramidopropyl betaine, potassium isobutyrate, potassium propionate, potassium pivalate, potassium glycolate, potassium gluconate, potassium methanesulfonate, calcium stearate, calcium glycolate, calcium gluconate, calcium 3-methyl-2-oxobutyrate, and calcium methanesulfonate. The term betaines are a general term for inner salts. Specifically, it is amino acid compounds of which amino groups are added with three methyl groups. More specific examples can include trimethylglycine, carnitine, and proline betaines.

The water-soluble salt can further contain a monohydric alcohol or polyhydric alcohol having 1 to 4 carbon atoms. The alcohol is preferably selected from the group consisting of ethanol, isopropyl alcohol, ethylene glycol, diethylene glycol, triethylene glycol, glycerin, polyethylene glycol, polypropylene glycol, polyglycerin, diglycerin, and a silicone compound having a polyglycerin structure. It is more preferable that the silicone compound having the polyglycerin structure is shown by the general formulae (4)' to (5)' described abave.

In the pretreatment methods with the aqueous solution containing the water-soluble salt, the cured bio-electrode film can be wetted by a spraying method, a droplet-dispensing method, etc. The bio-electrode film can also be wetted under a high-temperature, high-humidity condition, like sauna. To prevent drying after the wetting, it is possible to cover the permeated layer by piling a protective film further thereon. Because the protective film needs to be removed immediately before the bio-electrode is attached to the skin, the protective film may be coated with a release agent, or a peelable fluorine resin film may be used as the protective film. For long-time storage, the dry electrode covered with the peelable film is preferably sealed in a bag that is covered with aluminum etc. To prevent drying in the bag covered with aluminum, it is preferable to include water therein.

Before the inventive bio-electrode is attached to skin, the skin may be moisturized with water, alcohol, etc., or the skin may be wiped with a cloth or absorbent cotton containing water, alcohol, etc. The water and the alcohol may contain the above-described salts.

As described above, the inventive method for manufacturing a bio-electrode makes it possible to manufacture the inventive bio-electrode which is excellent in electric conductivity and biocompatibility, light-weight, and capable of preventing significant reduction in the electric conductivity even when wetted with water or dried, easily and at low cost.

### EXAMPLE

Hereinafter, the present invention will be specifically described with reference to Examples and Comparative Examples, but the present invention is not limited thereto.

With using the following dihydroxy group-containing sulfonamide compounds 1 to 20, an isocyanate compound, in some cases a dihydroxy compound for elongating chain length, a monohydroxy compound or a monoisocyanate compound for sealing a terminal, a solvent propylene glycol monomethyl ether acetate in an amount twice the mass of the raw material compounds, and 0.02% by mass of catalyst XK-640 (manufactured by King Industries, Inc.) based on 100 parts by mass of the raw material compounds, were mixed. The mixture was reacted at 90°C for 9 hours to synthesize the ionic urethane resins 1 to 28.

The composition of obtained polymer is shown in a proportion of the raw materials used. The molecular weight (Mw) and the dispersity (Mw/Mn) of the obtained polymer were determined by gel permeation chromatography (GPC) using tetrahydrofuran (THF) as a solvent.

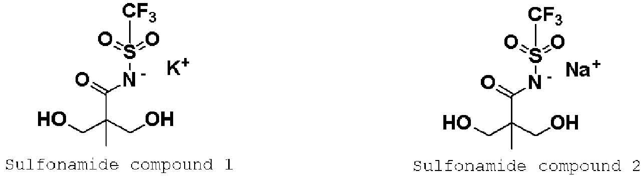

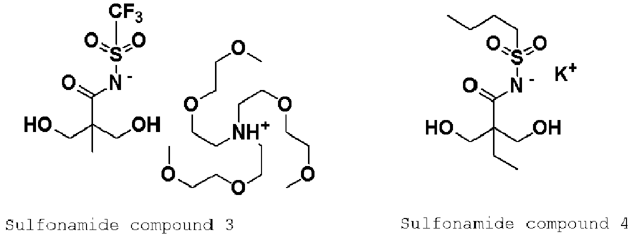

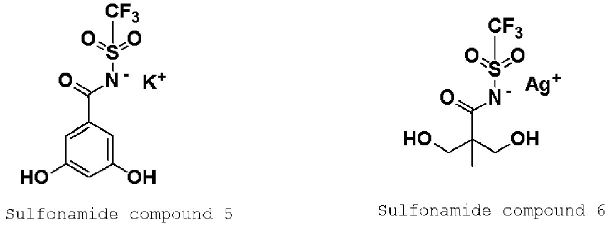

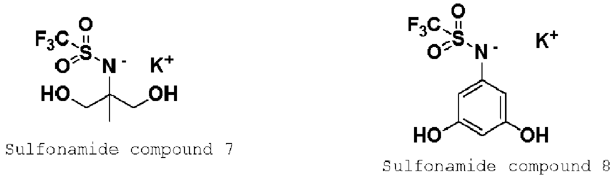

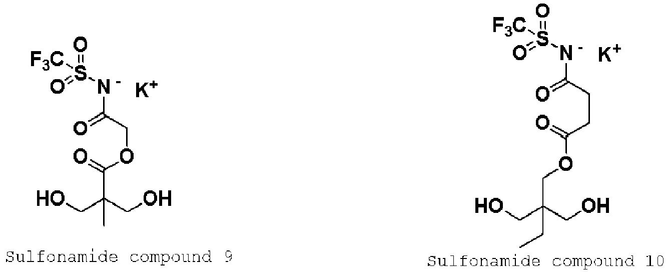

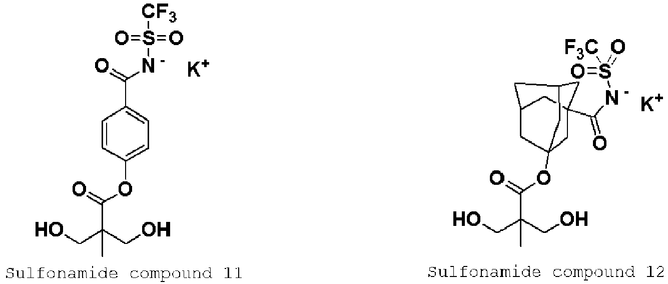

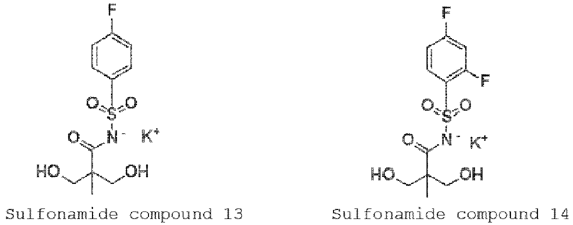

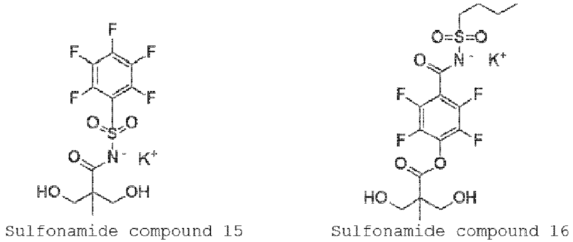

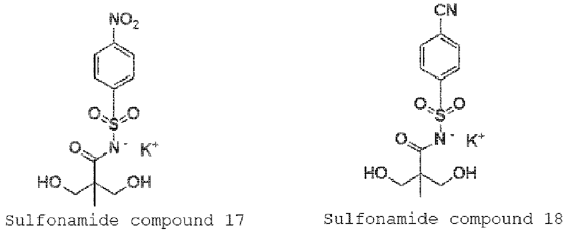

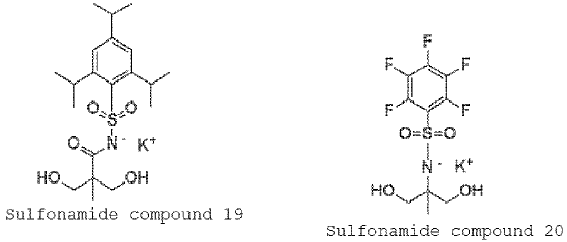

### Ionic urethane resin 1 (derived from dihydroxy group-containing sulfonamide compound 4)

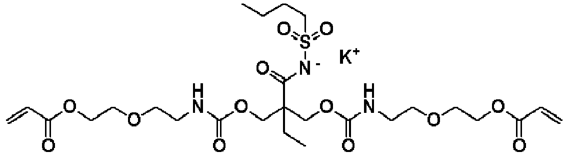

### Ionic urethane resin 2 (derived from dihydroxy group-containing sulfonamide compound 1)

Mw=7,700
Mw/Mn=2.01

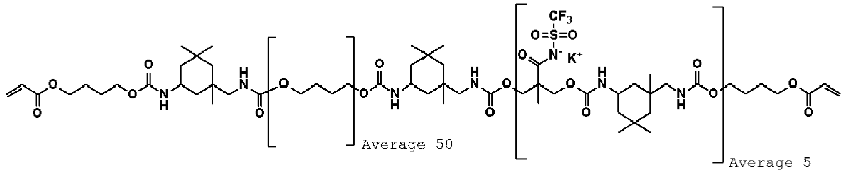

### Ionic urethane resin 3 (derived from dihydroxy group-containing sulfonamide compound 2)

Mw=17,600
Mw/Mn=2.11

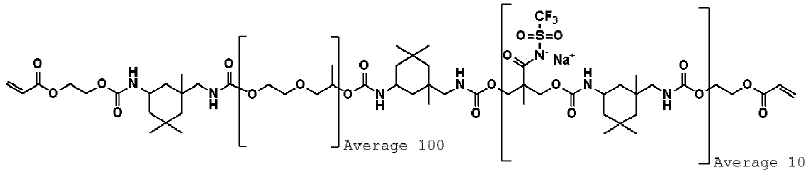

### Ionic urethane resin 4 (derived from dihydroxy group-containing sulfonamide compound 7)

Mw=16,600
Mw/Mn=2.09

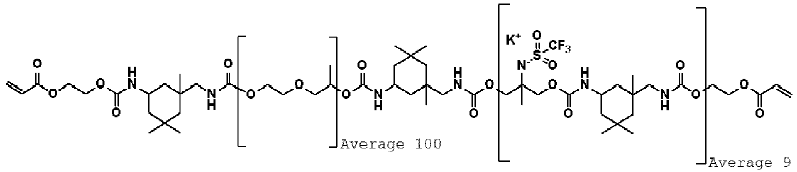

### Ionic urethane resin 5 (derived from dihydroxy group-containing sulfonamide compound 1)

Mw=7,600
Mw/Mn=2.00

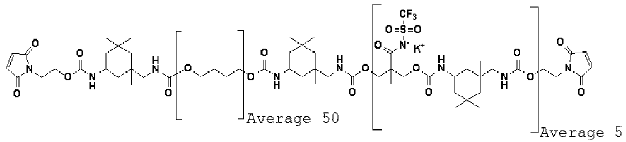

### Ionic urethane resin 6 (derived from dihydroxy group-containing sulfonamide compound 1)

Mw=8,800
Mw/Mn=2.11

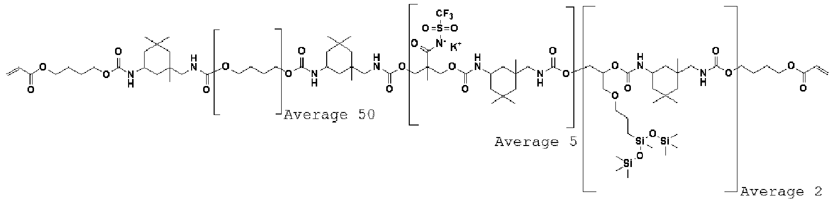

### Ionic urethane resin 7 (derived from dihydroxy group-containing sulfonamide compound 1)

Mw=9,500
Mw/Mn=2.30

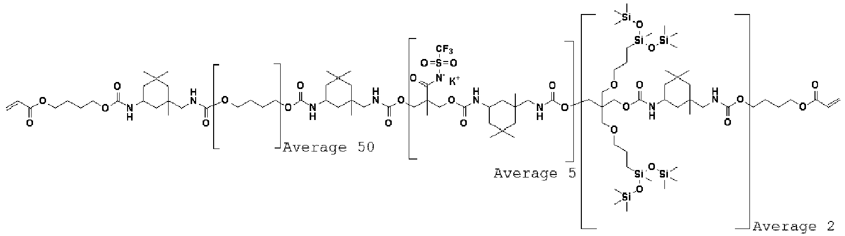

### Ionic urethane resin 8 (derived from dihydroxy group-containing sulfonamide compound 5)

Mw=48,000
Mw/Mn=3.30

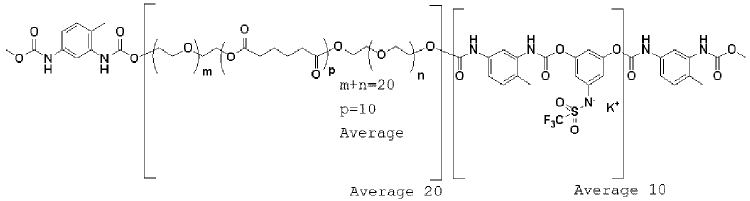

### Ionic urethane resin 9 (derived from dihydroxy group-containing sulfonamide compound 3)

Mw=12,000
Mw/Mn=2.80

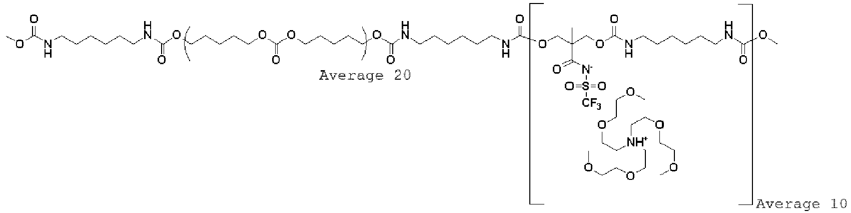

### Ionic urethane resin 10 (derived from dihydroxy group-containing sulfonamide compound 5)

Mw=22,600
Mw/Mn=2.70

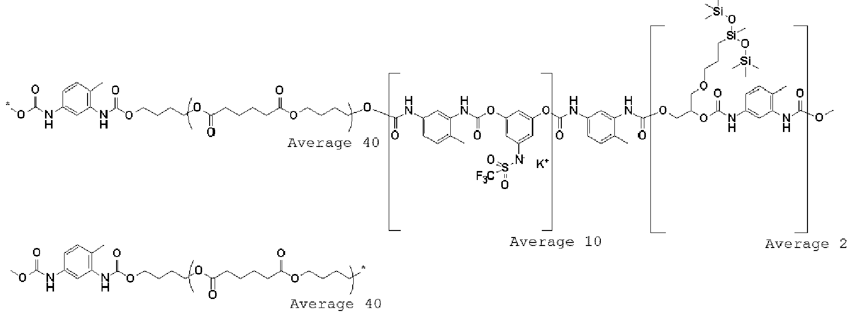

In the formulae, * represents a bonding arm.

### Ionic urethane resin 11 (derived from dihydroxy group-containing sulfonamide compound 5)

Mw=38,000
Mw/Mn=3.10

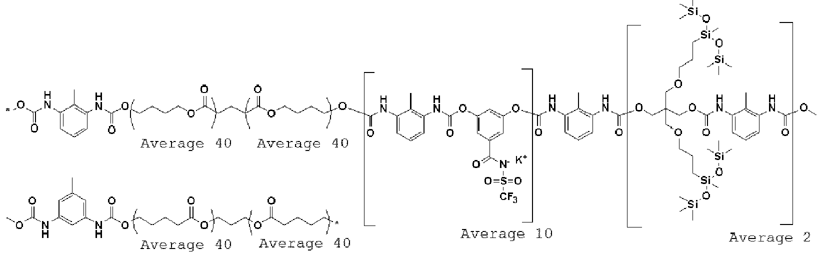

In the formulae, * represents a bonding arm.

### Ionic urethane resin 12 (derived from dihydroxy group-containing sulfonamide compound 6)

Mw=12,000
Mw/Mn=2.90

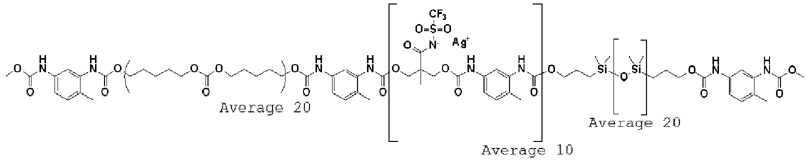

### Ionic urethane resin 13 (derived from dihydroxy group-containing sulfonamide compound 7)

Mw=30,000
Mw/Mn=2.91

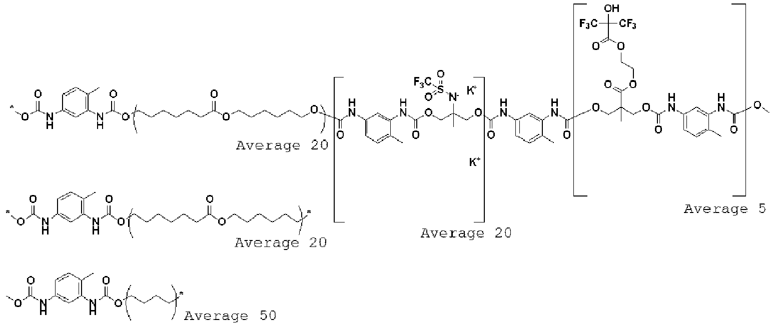

In the formulae, * represents a bonding arm.

### Ionic urethane resin 14 (derived from dihydroxy group-containing sulfonamide compound 7)

Mw=50,500
Mw/Mn=2.97

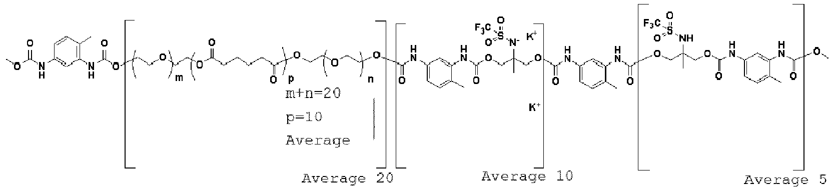

### Ionic urethane resin 15 (derived from dihydroxy group-containing sulfonamide compound 8)

Mw=53,000
Mw/Mn=3.02

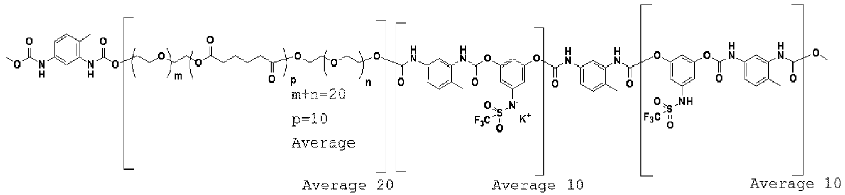

### Ionic urethane resin 16 (derived from dihydroxy group-containing sulfonamide compound 9)

Mw=23,000
Mw/Mn=2.80

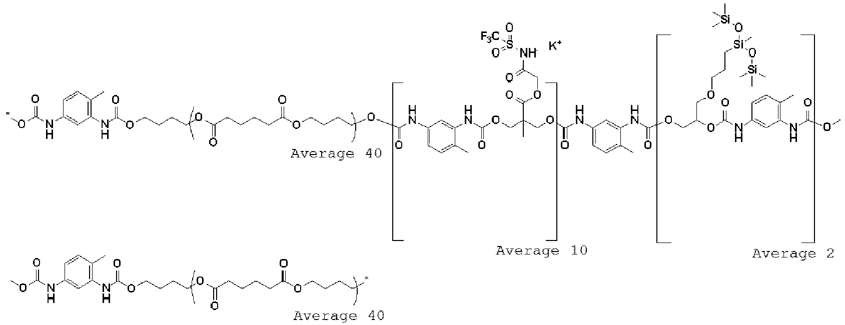

In the formulae, * represents a bonding arm.

### Ionic urethane resin 17 (derived from dihydroxy group-containing sulfonamide compound 10)

Mw=23,600
Mw/Mn=2.88

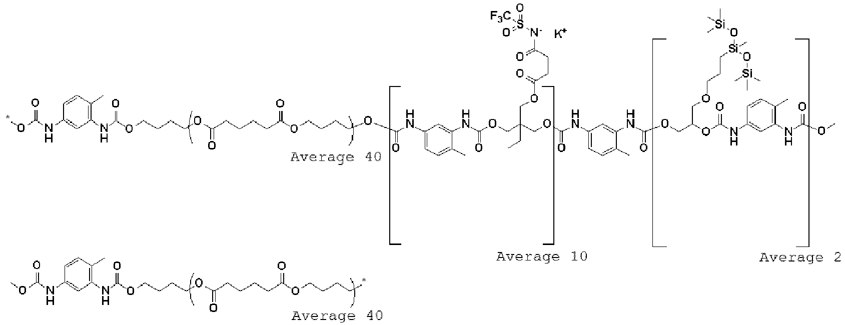

In the formulae, * represents a bonding arm.

### Ionic urethane resin 18 (derived from dihydroxy group-containing sulfonamide compound 11)

Mw=24,000
Mw/Mn=2.82

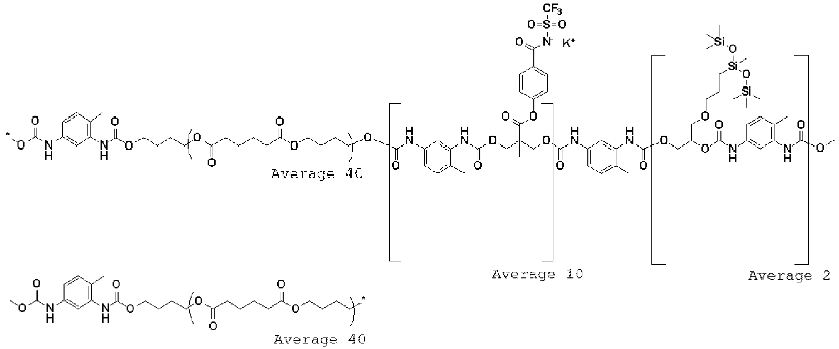

In the formulae, * represents a bonding arm.

### Ionic urethane resin 19 (derived from dihydroxy group-containing sulfonamide compound 12)

Mw=24,400
Mw/Mn=2.89

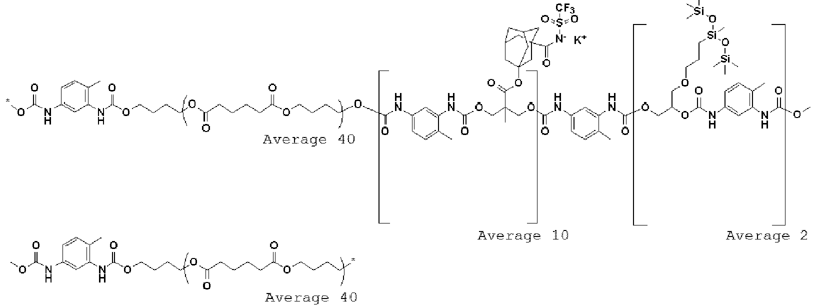

In the formulae, * represents a bonding arm.

### Ionic urethane resin 20 (derived from dihydroxy group-containing sulfonamide compound 9)

Mw=9,900
Mw/Mn=2.08

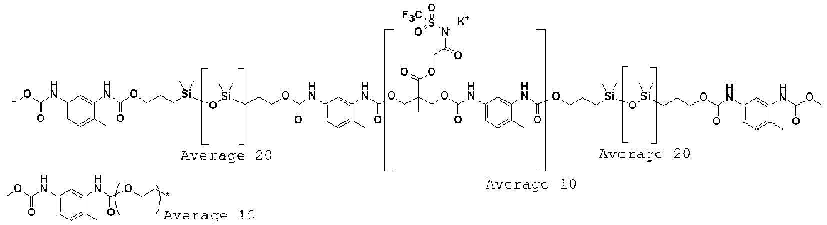

In the formulae, * represents a bonding arm.

### Ionic urethane resin 21 (derived from dihydroxy group-containing sulfonamide compound 13)

Mw=22,900
Mw/Mn=2.78

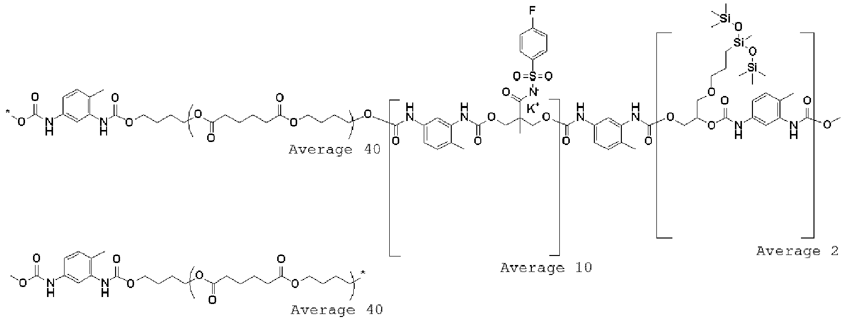

In the formulae, * represents a bonding arm.

### Ionic urethane resin 22 (derived from dihydroxy group-containing sulfonamide compound 14)

Mw=23,000
Mw/Mn=2.81

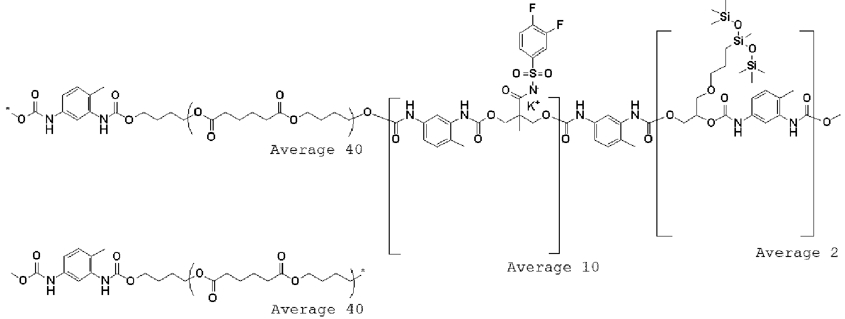

In the formulae, * represents a bonding arm.

### Ionic urethane resin 23 (derived from dihydroxy group-containing sulfonamide compound 15)

Mw=23,200
Mw/Mn=2.83

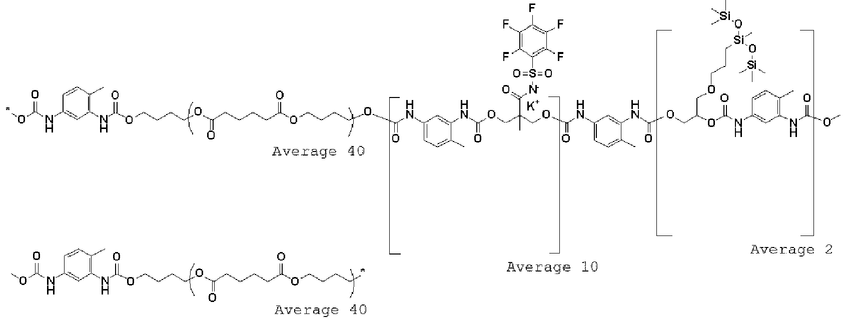

In the formulae, * represents a bonding arm.

### Ionic urethane resin 24 (derived from dihydroxy group-containing sulfonamide compound 16)

Mw=24,000
Mw/Mn=2.87

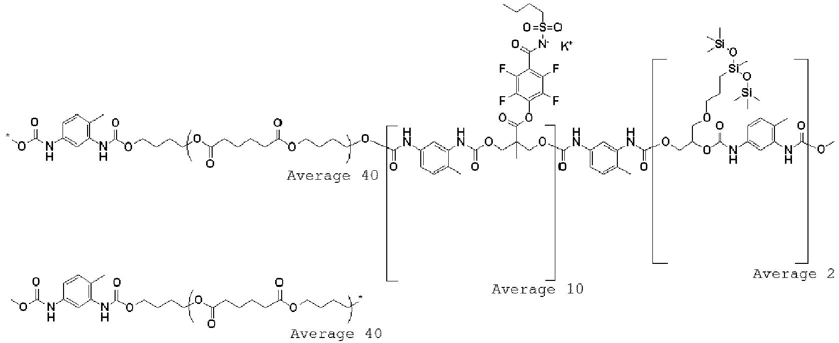

In the formulae, * represents a bonding arm.

### Ionic urethane resin 25 (derived from dihydroxy group-containing sulfonamide compound 17)

Mw=23,200
Mw/Mn=2.80

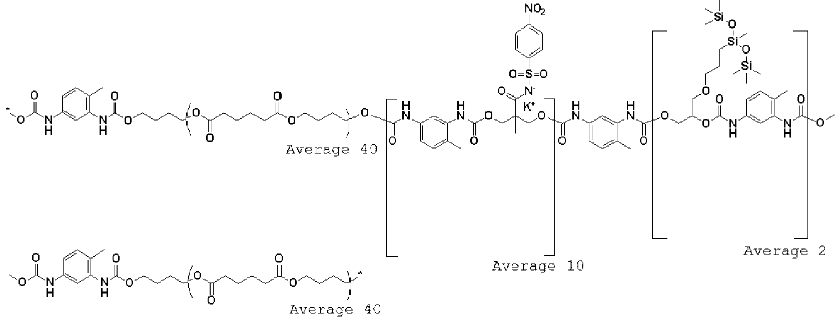

In the formulae, * represents a bonding arm.

### Ionic urethane resin 26 (derived from dihydroxy group-containing sulfonamide compound 18)

Mw=23,000
Mw/Mn=2.79

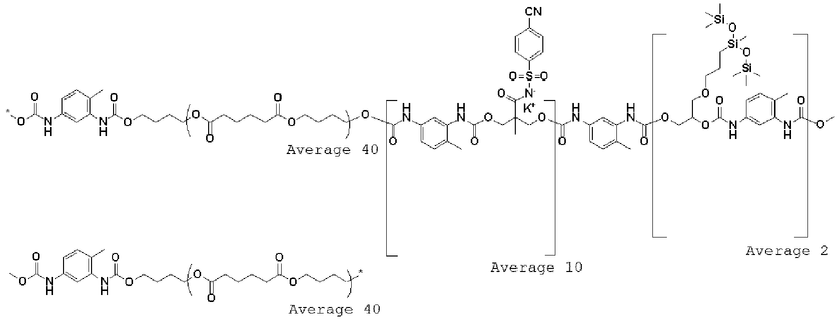

In the formulae, * represents a bonding arm.

### Ionic urethane resin 27 (derived from dihydroxy group-containing sulfonamide compound 19)

Mw=24,000
Mw/Mn=2.81

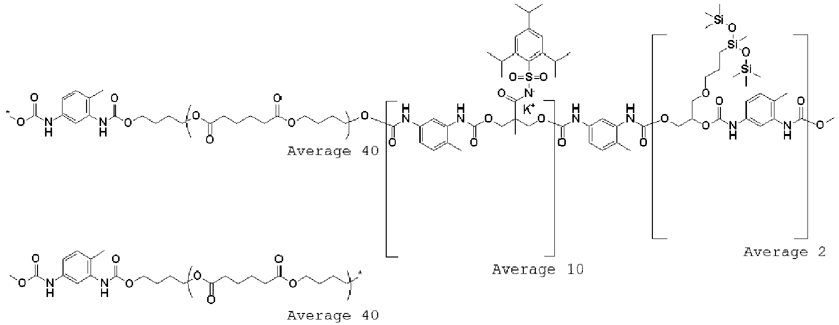

In the formulae, * represents a bonding arm.

### Ionic urethane resin 28 (derived from dihydroxy group-containing sulfonamide compound 20)

Mw=23,300
Mw/Mn=2.63

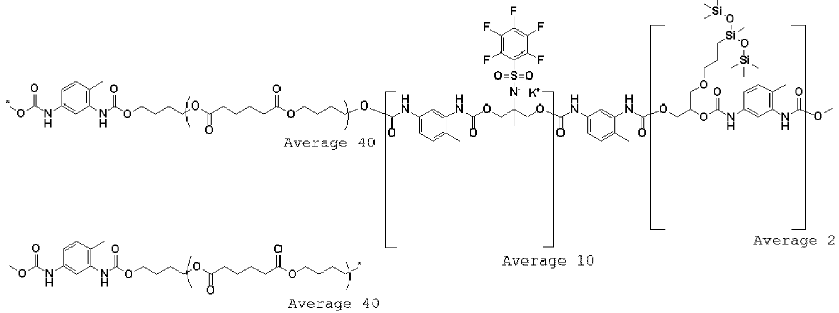

In the formulae, * represents a bonding arm.

Silicone pendant urethane (meth)acrylate 1, which was blended into a bio-electrode solution, is shown below.

### Silicone pendant urethane (meth)acrylate 1

Mw=24,800
Mw/Mn=2.65

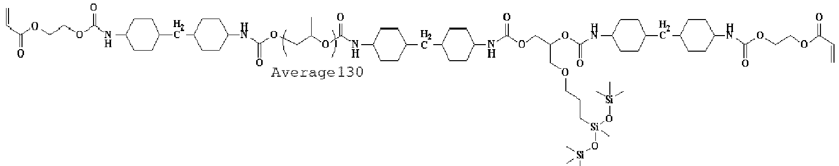

The number of repeating in the formula is the average.

Acrylic resin 1, which was blended into a bio-electrode solution as an acrylic-based resin, and Urethane resins 1 and 2, which was blended into a bio-electrode solution as a urethane-based resin, are shown below.

### Acrylic resin 1

Mw=337,000
Mw/Mn=3.65

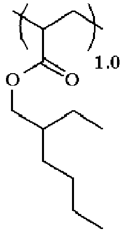

### Urethane resin 1

Mw=83,000
Mw/Mn=4.02

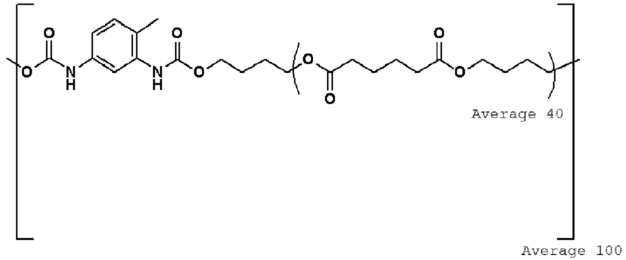

### Urethane resin 2

Mw=84,000
Mw/Mn=4.22

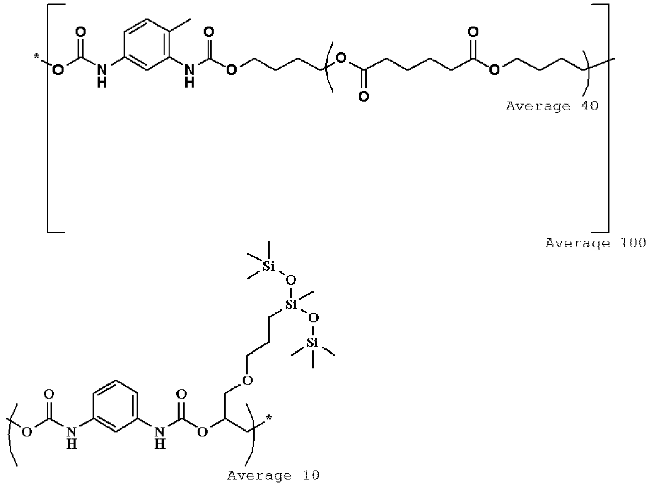

In the formulae, * represents a bonding arm.

Polyglycerin-silicone 1 and 2 are shown below.

### Polyglycerin-silicone 1

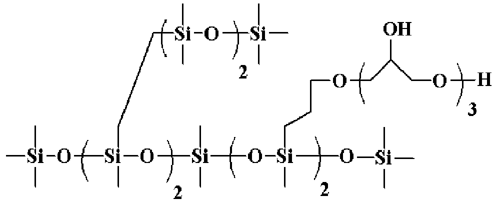

### Polyglycerin-silicone 2

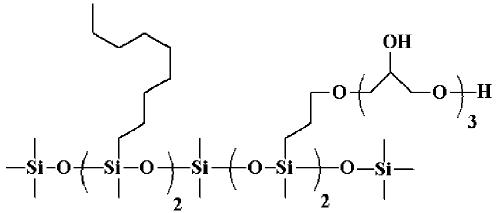

Organic solvents blended into the bio-electrode solutions are shown below.
EDE: diethylene glycol diethyl ether
PGMEA: propylene glycol monomethyl ether acetate

The lithium titanate powder, radical generator, and electric conductivity improver (carbon black, multilayer carbon nanotube,graphite, silver flakes), which was blended into the bio-electrode solutions as additives, are shown below.
Lithium titanate powder: with the size of 200 nm or less, manufactured by Sigma-Aldrich Co., LLC.
Radical generator: IRGACURE TPO manufactured by BASF SE Carbon black: DENKA BLACK Li-400 manufactured by Denka Company Limited
Multilayer carbon nanotube: with the diameter of 110 to 170 nm and length of 5 to 9 pm, manufactured by Sigma-Aldrich Co., LLC.
Graphite: with the diameter of 20 um or less, manufactured by Sigma-Aldrich Co., LLC.
Silver flakes: with the average size of 10 um, manufactured by Sigma-Aldrich Co., LLC.

### [Examples 1 to 27, Comparative Example 1]

According to the compositions shown in Tables 1 to 3, ionic urethane resins, resins, organic solvents, and additives (radical generator, and electric conductivity improver) were blended to prepare bio-electrode solutions (Bio-electrode solutions 1 to 27 and Comparative bio-electrode solution 1).

**[Table 1]**

| Bio-electrode solution | Ionic urethane resin (parts by mass) | Resin (parts by mass) | Organic solvent (parts by mass) | Additive (parts by mass) |
|---|---|---|---|---|
| Bio-electrode solution 1 | Ionic urethane resin 1 (20) | | PGMEA (200) | IRGACURE TPO (1.0) |
| | | | | Lithium titanate powder (12) |
| | Ionic urethane resin 2 (80) | | | |
| | | | | Silver flakes (8) |
| Bio-electrode solution 2 | Ionic urethane resin 3 (60) | Urethane resin 1 (40) | EDE (200) | IRGACURE TPO (1.0) |
| Bio-electrode solution 3 | Ionic urethane resin 4 (60) | Urethane resin 2 (40) | EDE (200) | IRGACURE TPO (1.0) |
| Bio-electrode solution 4 | Ionic urethane resin 5 (50) | Acrylic resin 1 (50) | EDE (200) | IRGACURE TPO (1.0) |
| Bio-electrode solution 5 | Ionic urethane resin 6 (60) | Silicone pendant urethane (meth) acrylate 1 (40) | EDE (200) | IRGACURE TPO (1.0) |
| Bio-electrode solution 6 | Ionic urethane resin 7 (100) | | EDE (200) | IRGACURE TPO (1.0) |
| Bio-electrode solution 7 | Ionic urethane resin 8 (100) | | EDE (200) | Carbon black (20) |
| Bio-electrode solution 8 | Ionic urethane resin 9 (100) | | EDE (200) | Carbon black (20) |
| | | | | Polyglycerin-silicone 1 (10) |
| Bio-electrode solution 9 | Ionic urethane resin 10 (100) | | EDE (200) | Carbon black (20) |
| | | | | Polyglycerin-silicone 1 (10) |
| Bio-electrode solution 10 | Ionic urethane resin 11 (100) | | EDE (200) | Carbon black (20) |
| | | | | Polyglycerin-silicone 1 (10) |
| Bio-electrode solution 11 | Ionic urethane resin 12 (100) | | EDE (200) | Carbon black (20) |
| | | | | Polyglycerin-silicone 1 (10) |
| Bio-electrode solution 12 | Ionic urethane resin 13 (100) | | EDE (200) | Carbon black (20) |
| | | | | Polyglycerin-silicone 1 (10) |
| Bio-electrode solution 13 | Ionic urethane resin 14 (100) | | EDE (200) | Carbon black (20) |
| | | | | Polyglycerin-silicon 1 (10) |
| Bio-electrode solution 14 | Ionic urethane resin 15 (100) | | EDE (200) | Multilayer carbon nanotube (5) |
| | | | | Polyglycerin-silicone 1 (10) |
| Bio-electrode solution 15 | Ionic urethane resin 16 (100) | | EDE (200) | Carbon black (20) |
| Bio-electrode solution 16 | Ionic urethane resin 17 (100) | | EDE (200) | Graphite (7) |
| | | | | Polyglycerin-silicone 1 (10) |
| Bio-electrode solution 17 | Ionic urethane resin 18 (100) | | EDE (200) | Carbon black (20) |
| | | | | Polyglycerin-silicone 1 (10) |
| Bio-electrode solution 18 | Ionic urethane resin 19 (100) | | EDE (200) | Carbon black (12) |
| | | | | Polyglycerin-silicon 1 (8) |
| Bio-electrode solution 19 | Ionic urethane resin 20 (100) | | EDE (200) | Carbon black (12) |
| | | | | Polyglycerin-silicon 2 (8) |

**[Table 2]**

| Bio-electrode solution | Ionic urethane resin (parts by mass) | Resin (parts by mass) | Organic solvent (parts by mass) | Additive (parts by mass) |
|---|---|---|---|---|
| Bio-electrode solution 20 | Ionic urethane resin 21 (100) | | EDE (200) | Graphite (7) |
| | | | | Polyglycerin-silicone 1 (10) |
| Bio-electrode solution 21 | Ionic urethane resin 22 (100) | | EDE (200) | Carbon black (20) |
| | | | | Polyglycerin-silicone 1 (10) |
| Bio-electrode solution 22 | Ionic urethane resin 23 (100) | | EDE (200) | Carbon black (12) |
| | | | | Polyglycerin-silicone 1 (8) |
| Bio-electrode solution 23 | Ionic urethane resin 24 (100) | | EDE (200) | Carbon black (12) |
| | | | | Polyglycerin-silicone 2 (8) |
| Bio-electrode solution 24 | Ionic urethane resin 23 (100) | | EDE (200) | Carbon black (12) |
| | | | | Polyglycerin-silicone 1 (8) |
| Bio-electrode solution 23 | Ionic urethane resin 26 (100) | | EDE (200) | Carbon black (12) |
| | | | | Polyglycerin-silicone 2 (8) |
| Bio-electrode solution 26 | Ionic urethane resin 27 (100) | | EDE (200) | Carbon black (12) |
| | | | | Polyglycerin-silicone 1 (8) |
| Bio-electrode solution 27 | Ionic urethane resin 28 (100) | | EDE (200) | Carbon black (12) |
| | | | | Polyglycerin-silicone 2 (8) |

**[Table 3]**

| Bio-electrode solution | Ionic urethane resin (parts bv mass) | Resin (parts by mass) | Organic solvent (parts by mass) | Additive (parts by mass) |
|---|---|---|---|---|
| Comparative bio-electrode solution 1 | - | Urethane resin 1 (100) | EDE (200) | Carbon black (20) |

### (Manufacture of Bio-electrodes)

As shown in FIG. 3, a thermoplastic urethane (TPU) film ST-604 (designated by number 20, manufactured by Bemis Associates Inc.) was coated with an electro-conductive paste DOTITE FA-333 (manufactured by Fujikura Kasei Co., Ltd.) by screen printing. The coating film was baked in an oven at 120°C for 10 minutes to print electro-conductive pattern of a keyhole-shape having a circular portion with a diameter of 2 cm. Then, one of the bio-electrode solutions shown in Tables 1 to 3 was applied onto the circular portion by stencil printing. After being air-dried at room temperature for 10 minutes, the composition-coated film was baked using an oven at 120°C for 10 minutes for evaporating the solvent and being cured, and thus bio-electrodes 1 was prepared. Each bio-electrode 1 included an electro-conductive base material 2 and a living body contact layer 3 formed on the circular portion of the electro-conductive base material 2. In the case of bio-electrodes coated with Bio-electrode solutions 1 to 6, the composition-coated film was cured by light irradiating of 500 mJ/cm² with a 1,000 W xenon lamp under a nitrogen atmosphere.

### (Measurement of Thickness of Living Body Contact Layer)

In the prepared bio-electrodes, the thickness of the living body contact layer was measured by using a micrometer. Table 4 shows the results.

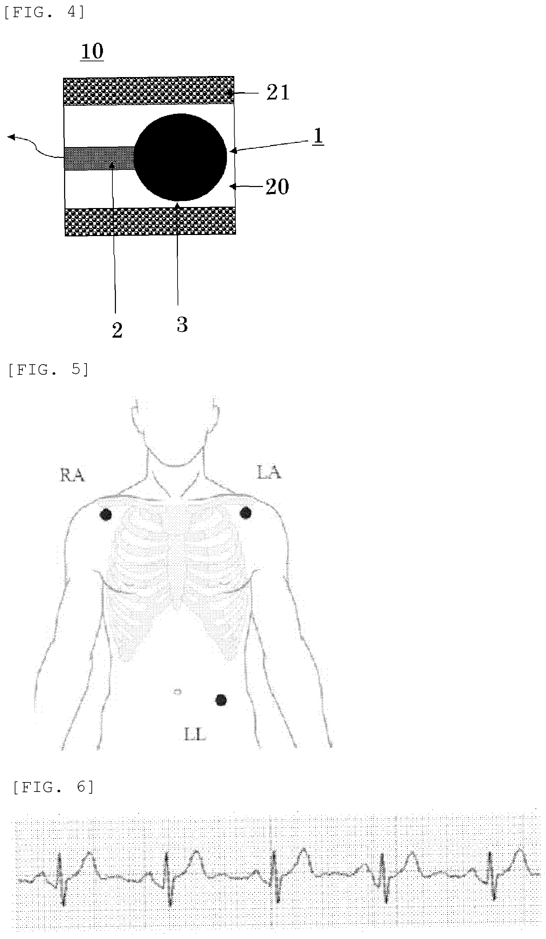

The thermoplastic urethane film 20 having the bio-electrodes printed thereon was cut out and pasted on a double-sided tape 21 as shown in FIG. 4. Thus, three bio-electrode samples 10 were prepared for each of the bio-electrode solutions.

### (Measurement of Biological Signal)

The electro-conductive wiring pattern formed with the electro-conductive paste of the bio-electrodes was connected to a portable electrocardiograph HCG-901 (manufactured by OMRON Healthcare Co., Ltd.) through an electro-conductive wire. A positive electrode of the electrocardiograph was attached to a location LA on a human body as shown in FIG. 5, a negative electrode was attached to a location LL, and an earth was attached to a location RA. Immediately after the attachments, the electrocardiogram measurement was started, and the time until an electrocardiogram waveform including P, Q, R, S, and T waves appeared as shown in FIG. 6 was measured. Table 4 shows the results.

**[Table 4]**

| Example | Bio-electrode solution | Resin thickness (µm) | Time (min.) until ECG signal appeared |
|---|---|---|---|
| Example 1 | Bio-electrode solution 1 | 14 | 5 |
| Example 2 | Bio-electrode solution 2 | 20 | 3 |
| Example 3 | Bio-electrode solution 3 | 28 | 4 |
| Example 4 | Bio-electrode solution 4 | 22 | 4 |
| Example 3 | Bio-electrode solution 5 | 21 | 4 |
| Example 6 | Bio-electrode solution 6 | 26 | 3 |
| Example 7 | Bio-electrode solution 7 | 23 | 5 |
| Example 8 | Bio-electrode solution 8 | 39 | 4 |
| Example 9 | Bio-electrode solution 9 | 39 | 1 |
| Example 10 | Bio-electrode solution 10 | 40 | 0 |
| Example 11 | Bio-electrode solution 11 | 32 | 0 |
| Example 12 | Bio-electrode solution 12 | 38 | 0 |
| Example 13 | Bio-electrode solution 13 | 36 | 0 |
| Example 14 | Bio-electrode solution 14 | 38 | 0 |
| Example 15 | Bio-electrode solution 15 | 32 | 4 |
| Example 16 | Bio-electrode solution 16 | 33 | 0 |
| Example 17 | Bio-electrode solution 17 | 34 | 0 |
| Example 18 | Bio-electrode solution 18 | 40 | 0 |
| Example 19 | Bio-electrode solution 19 | 34 | 0 |
| Example 20 | Bio-electrode solution 20 | 26 | 2 |
| Example 21 | Bio-electrode solution 21 | 24 | 3 |
| Example 22 | Bio-electrode solution 22 | 31 | 4 |
| Example 23 | Bio-electrode solution 23 | 32 | 2 |
| Example 24 | Bio-electrode solution 24 | 36 | 8 |
| Example 23 | Bio-electrode solution 25 | 37 | 9 |
| Example 26 | Bio-electrode solution 26 | 39 | 14 |
| Example 27 | Bio-electrode solution 27 | 32 | 8 |
| Comparative Example 1 | Comparative bio-electrode solution 1 | 23 | ECG signal did not appear |

As shown in Table 4, biological signals could be collected shortly after attaching the bio-electrodes to the body in Examples 1 to 27, where the living body contact layer was formed using Bio-electrode solutions 1 to 27 according to the inventive bio-electrode compositions blended with a resin having a urethane bond in a main chain and at least one salt selected from the group consisting of salts of ammonium, sodium, potassium, and silver formed with sulfonamide in a side chain. On the other hand, in Comparative Example 1 using Comparative bio-electrode solution 1 which did not contain an ionic component having the particular structure, it was not possible to collect biological signals.

The present description includes the following embodiments.
[1]: A bio-electrode composition including a resin as a component (A), wherein
   the component (A) has a urethan bond in a main chain and at least one salt selected from the group consisting of salts of ammonium, sodium, potassium, and silver formed with sulfonamide in a side chain.
[2]: The bio-electrode composition of the above [1], wherein the component (A) is represented by the following general formula (1), 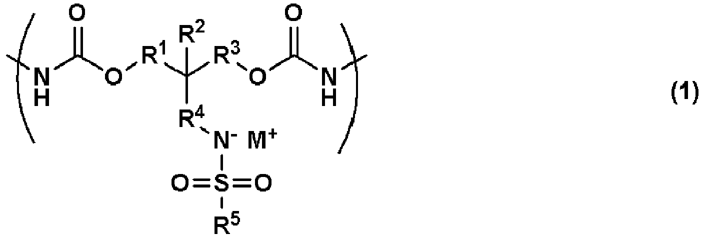 wherein, R¹ and R³ represent a single bond, or a linear or branched alkylene group having 1 to 6 carbon atoms, and optionally have an ether bond or a bromine atom; R² represents a hydrogen atom, a hydroxy group, or a linear or branched alkyl or alkoxy group having 1 to 6 carbon atoms; R⁴ represents a single bond or a linear, branched, or cyclic hydrocarbylene group having 1 to 20 carbon atoms, and optionally have a carbonyl group, an ether group, or an ester group, or optionally form a ring together with R¹; R⁵ represents a linear, branched, or cyclic alkyl group having 1 to 10 carbon atoms and optionally substituted with a fluorine atom, or R⁵ represents an alkenyl group having 2 to 6 carbon atoms, an alkynyl group having 2 to 6 carbon atoms, or an aryl group having 6 to 10 carbon atoms and optionally has a fluorine atom, a trifluoromethyl group, a cyano group, a nitro group, a hydroxy group, an alkoxy group having 1 to 6 carbon atoms, or an alkyl group having 1 to 6 carbon atoms; and M⁺ represents ammonium ion, sodium ion, potassium ion, or silver ion.
[3]: The bio-electrode composition of the above [1] or [2], wherein the component (A) includes an ammonium ion represented by the following general formula (2) as an ammonium ion for forming the ammonium salt, 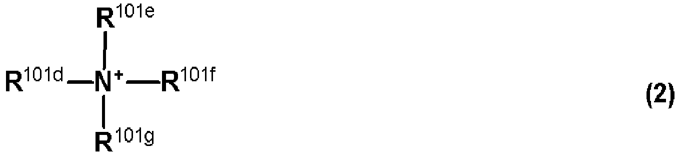 wherein R^{101d}, R^{101e}, R^{101f}, and R^{101g} each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, and optionally have one or more selected from the group consisting of an ether group, a carbonyl group, an ester group, a hydroxy group, an amino group, a nitro group, a sulfonyl group, a sulfinyl group, a halogen atom, and a sulfur atom; and R^{101d} and R^{101e}, or R^{101d}, R^{101e}, and R^{101f}, are optionally bonded to each other together with a nitrogen atom bonded therewith to form a ring in which R^{101d} and R^{101e}, or R^{101d}, R^{101e}, and R^{101f}, represent an alkylene group having 3 to 10 carbon atoms, or to form a heteroaromatic ring having the nitrogen atom in the general formula (2) within the ring.
[4]: The bio-electrode composition of any one of the above [1] to [3], further including a resin other than the component (A) as a component (B).
[5]: The bio-electrode composition of the above [4], wherein the component (B) is at least one resin selected from the group consisting of a silicone resin, a (meth)acrylate resin, and a urethane resin.
[6]: The bio-electrode composition of any one of the above [1] to [5], further including a carbon material and/or a metal powder as a component (C).
[7]: The bio-electrode composition of the above [6], wherein the carbon material is one or both of carbon black and carbon nanotube.
[8]: The bio-electrode composition of the above [6] or [7], wherein the metal powder is a metal powder selected from the group consisting of gold, silver, platinum, copper, tin, titanium, nickel, aluminum, tungsten, molybdenum, ruthenium, chromium, and indium.
[9]: The bio-electrode composition of the above [8], wherein the metal powder is a silver powder.
[10]: The bio-electrode composition of any one of the above [1] to [9], further comprising an organic solvent as a component (D).
[11]: A bio-electrode including an electro-conductive base material and a living body contact layer formed on the electro-conductive base material, wherein
   the living body contact layer is a cured product of the bio-electrode composition of any one of the above [1] to [10].
[12]: The bio-electrode of the above [11], wherein the electro-conductive base material includes one or more selected from the group consisting of gold, silver, silver chloride, platinum, aluminum, magnesium, tin, tungsten, iron, copper, nickel, stainless steel, chromium, titanium, carbon, and an electro-conductive polymer.
[13]: A method for manufacturing a bio-electrode having an electro-conductive base material and a living body contact layer formed on the electro-conductive base material, the method including:
   applying the bio-electrode composition of any one of the above [1] to [10] onto the electro-conductive base material; and
   curing the bio-electrode composition to form the living body contact layer.
[14]: The method for manufacturing a bio-electrode of the above [13], wherein the electro-conductive base material includes one or more selected from the group consisting of gold, silver, silver chloride, platinum, aluminum, magnesium, tin, tungsten, iron, copper, nickel, stainless steel, chromium, titanium, carbon, and an electro-conductive polymer.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A bio-electrode composition comprising a resin as a component (A), wherein
the component (A) has a urethan bond in a main chain and at least one salt selected from the group consisting of salts of ammonium, sodium, potassium, and silver formed with sulfonamide in a side chain.

2. The bio-electrode composition according to claim 1, wherein the component (A) is represented by the following general formula (1), 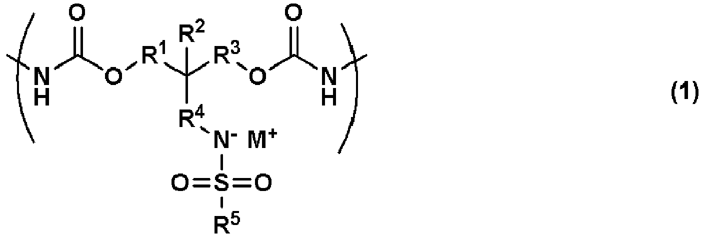 wherein, R¹ and R³ represent a single bond, or a linear or branched alkylene group having 1 to 6 carbon atoms, and optionally have an ether bond or a bromine atom; R² represents a hydrogen atom, a hydroxy group, or a linear or branched alkyl or alkoxy group having 1 to 6 carbon atoms; R⁴ represents a single bond or a linear, branched, or cyclic hydrocarbylene group having 1 to 20 carbon atoms, and optionally have a carbonyl group, an ether group, or an ester group, or optionally form a ring together with R¹; R⁵ represents a linear, branched, or cyclic alkyl group having 1 to 10 carbon atoms and optionally substituted with a fluorine atom, or R⁵ represents an alkenyl group having 2 to 6 carbon atoms, an alkynyl group having 2 to 6 carbon atoms, or an aryl group having 6 to 10 carbon atoms and optionally has a fluorine atom, a trifluoromethyl group, a cyano group, a nitro group, a hydroxy group, an alkoxy group having 1 to 6 carbon atoms, or an alkyl group having 1 to 6 carbon atoms; and M⁺ represents ammonium ion, sodium ion, potassium ion, or silver ion.

3. The bio-electrode composition according to claim 1 or 2, wherein the component (A) comprises an ammonium ion represented by the following general formula (2) as an ammonium ion for forming the ammonium salt, 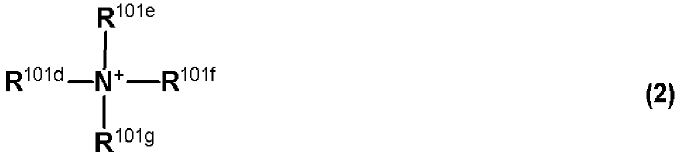 wherein R^{101d}, R^{101e}, R^{101f}, and R^{101g} each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, and optionally have one or more selected from the group consisting of an ether group, a carbonyl group, an ester group, a hydroxy group, an amino group, a nitro group, a sulfonyl group, a sulfinyl group, a halogen atom, and a sulfur atom; and R^{101d} and R^{101e}, or R^{101d}, R^{101e}, and R^{101f}, are optionally bonded to each other together with a nitrogen atom bonded therewith to form a ring in which R^{101d} and R^{101e}, or R^{101d}, R^{101e}, and R^{101f}, represent an alkylene group having 3 to 10 carbon atoms, or to form a heteroaromatic ring having the nitrogen atom in the general formula (2) within the ring.

4. The bio-electrode composition according to any one of claims 1 to 3, further comprising a resin other than the component (A) as a component (B).

5. The bio-electrode composition according to claim 4, wherein the component (B) is at least one resin selected from the group consisting of a silicone resin, a (meth)acrylate resin, and a urethane resin.

6. The bio-electrode composition according to any one of claims 1 to 5, further comprising a carbon material and/or a metal powder as a component (C).

7. The bio-electrode composition according to claim 6, wherein the carbon material is one or both of carbon black and carbon nanotube.

8. The bio-electrode composition according to claim 6 or 7, wherein the metal powder is a metal powder selected from the group consisting of gold, silver, platinum, copper, tin, titanium, nickel, aluminum, tungsten, molybdenum, ruthenium, chromium, and indium.

9. The bio-electrode composition according to claim 8, wherein the metal powder is a silver powder.

10. The bio-electrode composition according to any one of claims 1 to 9, further comprising an organic solvent as a component (D).

11. A bio-electrode comprising an electro-conductive base material and a living body contact layer formed on the electro-conductive base material, wherein
the living body contact layer is a cured product of the bio-electrode composition according to any one of claims 1 to 10.

12. The bio-electrode according to claim 11, wherein the electro-conductive base material comprises one or more selected from the group consisting of gold, silver, silver chloride, platinum, aluminum, magnesium, tin, tungsten, iron, copper, nickel, stainless steel, chromium, titanium, carbon, and an electro-conductive polymer.

13. A method for manufacturing a bio-electrode having an electro-conductive base material and a living body contact layer formed on the electro-conductive base material, the method comprising:
applying the bio-electrode composition according to any one of claims 1 to 10 onto the electro-conductive base material; and
curing the bio-electrode composition to form the living body contact layer.

14. The method for manufacturing a bio-electrode according to claim 13, wherein the electro-conductive base material comprises one or more selected from the group consisting of gold, silver, silver chloride, platinum, aluminum, magnesium, tin, tungsten, iron, copper, nickel, stainless steel, chromium, titanium, carbon, and an electro-conductive polymer.
